# EUROPÄISCHE PATENTSCHRIFT

(11) **EP 2 300 462 B1**
(45) Veröffentlichungstag und Bekanntmachung des Hinweises auf die Patenterteilung: **21.05.2014**
(21) Anmeldenummer: 09757185.5
(22) Anmeldetag: 22.05.2009
(51) Int. Cl.: C07D 413/14, C07D 273/01, A61K 31/553, A61P 7/00

(54) **MAKROCYCLISCHE HARNSTOFF- UND SULFAMIDDERIVATE ALS INHIBITOREN VON TAFIA**
MACROCYCLIC UREA AND SULFAMIDE DERIVATIVES AS INHIBITORS OF TAFIA
DÉRIVÉS MACROCYCLIQUES D'URÉE ET DE SULFAMIDE COMME INHIBITEURS DE TAFIA

(30) Priorität: 06.06.2008 EP 08290520
(43) Veröffentlichungstag der Anmeldung: 30.03.2011
(73) Patentinhaber: SANOFI, 75008 Paris (FR)
(72) Erfinder: KALLUS, Christopher, 65926 Frankfurt am Main (DE); BROENSTRUP, Mark, 65926 Frankfurt am Main (DE); EVERS, Andreas, 65926 Frankfurt am Main (DE); GLOBISCH, Anja, 65926 Frankfurt am Main (DE); SCHREUDER, Herman, 65926 Frankfurt am Main (DE); WAGNER, Michael, 65926 Frankfurt am Main (DE)
(74) Vertreter: Venne-Dunker, Sabine
(86) Internationale Anmeldenummer: PCT/EP2009/003650
(87) Internationale Veröffentlichungsnummer: WO 2009/146802

(56) Entgegenhaltungen:
- WO-A-97/30080
- WO-A-2005/105781
- NANTERMET ET AL.: "Design and Synthesis of Potent and Selective Macrocyclic Thrombin Inhibitors" BIOORGANIC & MEDICINAL CHEMISTRY LETTERS, Bd. 13, 2003, Seiten 2781-2784, XP002499844

## Beschreibung

Die Erfindung betrifft neue Verbindungen der Formel I, die das Enzym TAFIa (aktivierter Thrombin-aktivierbarer Fibrinolyse Inhibitor) inhibieren, Verfahren zu ihrer Herstellung und diese Verbindungen zur medizinischen Verwendung.

Das Enzym TAFIa entsteht beispielsweise durch Thrombinaktivierung aus dem Thrombin-aktivierbaren-Fibrinolyse-Inhibitor-Zymogen (TAFI). Das Enzym TAFI wird auch als Plasma Procarboxypeptidase B, Procarboxypeptidase U oder als Procarboxypeptidase R bezeichnet und ist ein Carboxypeptidase B ähnliches Proenzym (L. Bajzar, Arterioscler. Thromb. Vasc. Biol. 2000, Seiten 2511 - 2518).

Während einer Gerinnselbildung wird Thrombin als das Endprodukt der Koagulationskaskade generiert und induziert die Konversion von löslichem Plasmafibrinogen zu einer unlöslichen Fibrinmatrix. Gleichzeitig aktiviert Thrombin den endogenen Fibrinolyse-Inhibitor TAFI. Aktiviertes TAFI (TAFIa) entsteht also während der Thrombusbildung und der Lyse aus dem Zymogen TAFI unter der Einwirkung von Thrombin; Thrombomodulin im Komplex mit Thrombin verstärkt diesen Effekt etwa 1250 fach. TAFIa spaltet basische Aminosäuren am carboxy-Ende des Fibrins. Der Verlust der carboxy-terminalen Lysine als Bindestellen für Plasminogen führt dann zu einer Inhibition der Fibrinolyse. Effektive Inhibitoren von TAFIa verhindern den Verlust dieser hoch affinen Lysin-Bindungsstellen für Plasminogen und unterstützen auf diese Weise die endogene Fibrinolyse durch Plasmin: TAFIa Inhibitoren wirken profibrinolytisch.

Um die Hämostase im Blut aufrechtzuerhalten, haben sich Mechanismen ausgebildet, die zur Blutgerinnung und zur Auflösung von Gerinnseln führen; diese stehen in einem Gleichgewicht. Wenn ein gestörtes Gleichgewicht die Koagulation begünstigt, entsteht Fibrin in größeren Mengen, so dass pathologische Vorgänge der Thrombusbildung zu schweren Krankheitsbildern im Menschen führen können.
Genauso wie eine überschießende Koagulation zu schwerwiegenden thrombotisch bedingten Krankheitsbildern führen kann, besitzt eine antithrombotische Behandlung das Risiko von nicht erwünschten Blutungen durch eine Störung der Bildung eines notwendigen hämostatischen Pfropfs. Die Hemmung von TAFIa verstärkt - ohne die Koagulation und die Plättchenaggregation zu beeinflussen - die endogene Fibrinolyse d.h. das gestörte Gleichgewicht wird zugunsten der Fibrinolyse verschoben. So kann sowohl dem Aufbau eines klinisch relevanten Thrombus entgegengewirkt, als auch die Lyse eines schon bestehenden Gerinnsels verstärkt werden. Andererseits wird der Aufbau eines hämostatischen Pfropfs nicht beeinträchtigt, sodass eine Blutungsdiathese eher nicht zu erwarten ist (Bouma et al., J. Thrombosis and Haemostasis, 1, 2003, Seiten 1566 - 1574).

Inhibitoren von TAFIa sind bereits in der Internationalen Anmeldung WO2005/105781 beschrieben worden.

Die erfindungsgemäßen TAFIa Inhibitoren eignen sich für eine prophylaktische als auch für eine therapeutische Anwendung am Menschen, die an Erkrankungen leiden, die mit Thrombosen, Embolien, Hyperkoagulabilität oder fibrotischen Veränderungen einhergehen. Sie können zur Sekundär-Prevention eingesetzt werden und eignen sich sowohl für eine akute als auch für eine Langzeittherapie.

Die Erfindung betrifft daher die Verbindung der Formel I 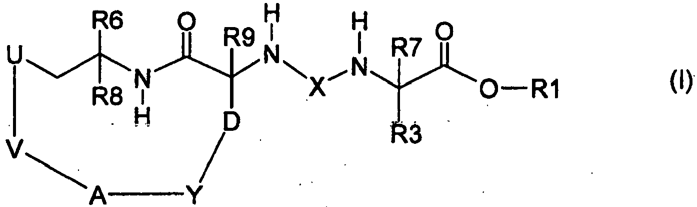 und/oder einer stereoisomeren Form der Verbindung der Formel I und/oder Gemische dieser Formen in jedem Verhältnis, und/oder ein physiologisch verträgliches Salz der Verbindung der Formel I, wobei
X für -C(O)- oder -SO₂- steht,
U für Sauerstoffatom, Schwefelatom, NH, -C(O)-NH- oder -(C₀-C₄)-Alkylen- steht,
A für Sauerstoffatom, Schwefelatom, NH, -C(O)-NH- -NH-C(O)-, -NR2- oder -(C₀-C₄)-Alkylen- steht,
V für
   1) -(C₂-C₉)-Alkylen-, wobei Alkylen unsubstituiert oder unabhängig voneinander ein-, zwei- oder dreifach durch -OH, NH₂ oder Halogen substituiert ist,
   2) -(C₁-C₂)-Alkylen-(C₃-C₆)-Cycloalkyl-(C₁-C₂)-Alkylen-, wobei Cycloalkyl unabhängig voneinander ein-, zwei- oder dreifach durch R15 substituiert ist, oder
   3) -(C₃-C₉)-Alkenylen- steht,
   D für -(C₁-C₂)-Alkylen- steht,
Y für
   1) kovalente Bindung,
   2) -(C₃-C₁₂)-Cycloalkyl, wobei Cycloalkyl unabhängig voneinander ein-, zwei- oder dreifach durch R15 substituiert ist,
   3) -(C₆-C₁₄)-Aryl, wobei Aryl unsubstituiert oder unabhängig voneinander ein-, zwei- oder dreifach durch R15 substituiert ist, oder
   4) Het, wobei unter Het ein 4- bis 15-gliedriges heterocyclisches Ringsystem mit 4 bis 15 Ringatomen verstanden wird, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten und
   worin Het unsubstituiert oder unabhängig voneinander ein-, zwei- oder dreifach durch ein -(C₁-C₃)-Alkyl, Halogen, -NH₂, -CF₃ oder-O-CF₃ substituiert ist, steht,
R1 für
   1) Wasserstoffatom,
   2) -(C₁-C₆)-Alkyl,
   3) -(C₁-C₆)-Alkyl-OH,
   4) -(C₀-C₄)-Alkyl-(C₃-C₆)-Cycloalkyl,
   5) -(C₁-C₁₀)-Alkyl-O-C(O)-O-R2,
   6) -(CH₂)ᵣ-(C₆-C₁₄)-Aryl, worin Aryl unsubstituiert oder unabhängig voneinander ein-, zwei- oder dreifach durch R15 substituiert ist und r die ganze Zahl Null, 1, 2 oder 3 bedeutet, oder
   7) -(CH₂)ₛ-Het, wobei unter Het ein 4- bis 15-gliedriges heterocyclisches Ringsystem mit 4 bis 15 Ringatomen verstanden wird, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten und worin Het unsubstituiert oder unabhängig voneinander ein-zwei- oder dreifach durch R15 substituiert ist und s die ganze Zahl Null, 1, 2 oder 3 bedeutet, steht,
R2 für
   1) -(C₁-C₆)-Alkyl,
   2) -(CH₂)ᵣ-(C₆-C₁₄)-Aryl, worin Aryl unsubstituiert oder unabhängig voneinander ein-, zwei- oder dreifach durch R15 substituiert ist und r die ganze Zahl Null, 1, 2 oder 3 bedeutet, oder
   3) -(C₀-C₄)-Alkyl-(C₃-C₆)-Cycloalkyl, steht,
R3 für
   1) -(C₂-C₆)-Alkylen-NH₂, wobei Alkylen unsubstiuiert ist oder ein-, zwei-, drei- oder vierfach durch Halogen substituiert ist,
   2) -(C₁-C₄)-Alkylen-O-(C₁-C₄)-Alkylen-NH₂,
   3) -(C₁-C₄)-Alkylen-SO₂-(C₁-C₄)-Alkylen-NH₂,
   4) -(C₀-C₄)-Alkylen-Het, wobei Het wie oben definiert ist und durch -NH₂ und ein-, zwei- oder dreifach durch R15 substituiert ist,
   5) -(C₀-C₄)-Alkylen-(C₃-C₈)-Cycloalkyl-NH₂ oder
   6) -(C₀-C₆)-Alkylen-cyclisches Amin, steht
R6 für
   1) Wasserstoffatom,
   2) -(C₁-C₆)-Alkyl, wobei Alkyl unsubstituiert oder unabhängig voneinander ein-, zwei- oder dreifach durch R16 substituiert ist,
   3) -O-(C₁-C₆)-Alkyl, wobei Alkyl unsubstituiert oder unabhängig voneinander ein-, zwei- oder dreifach durch R16 substituiert ist,
   4) -(C₀-C₄)-Alkylen-Het, wobei Het wie oben definiert ist, wobei Alkylen und Het unsubstituiert oder unabhängig voneinander ein-, zwei- oder dreifach durch R16 substituiert sind,
   5) -(C₀-C₄)-Alkylen-Aryl, wobei Alkylen und Aryl unsubstituiert oder unabhängig voneinander ein-, zwei- oder dreifach durch R16 substituiert sind, oder
   6) -(C₀-C₄)-Alkylen-(C₃-C₈)-Cycloalkyl, wobei Alkylen und Cycloalkyl unsubstituiert oder unabhängig voneinander ein-, zwei- oder dreifach durch R16 substituiert sind, steht,
R7 für Wasserstoffatom, Halogen oder -(C₁-C₆)-Alkyl steht,
R8 für Wasserstoffatom, Halogen oder -(C₁-C₆)-Alkyl steht,
R9 für Wasserstoffatom, Halogen oder -(C₁-C₆)-Alkyl steht,
R15 für Wasserstoffatom, -(C₁-C₄)-Alkyl, -O-CF₃, -NH₂, -OH, -CF₃ oder Halogen steht, und
R16 für -O-CF₃, -NH₂, -OH, -CF₃ oder Halogen steht.
   2) Die Erfindung betrifft ferner eine Verbindung der Formel I, wobei
X für -C(O)- oder -SO₂- steht,
U für Sauerstoffatom, Schwefelatom, NH, -C(O)-NH- oder -(C₀-C₄)-Alkylen- steht,
A für Sauerstoffatom, Schwefelatom, NH, -C(O)-NH- oder -(C₀-C₄)-Alkylen- steht,
V für
   1) -(C₂-C₉)-Alkylen- oder
   2) -(C₃-C₉)-Alkenylen- steht,
D für -(C₁-C₂)-Alkylen- steht,
Y für
   1) kovalente Bindung,
   2) -(C₃-C₁₂)-Cycloalkyl, wobei Cycloalkyl unabhängig voneinander ein-, zwei- oder dreifach durch R15 substituiert ist,
   3) -(C₆-C₁₄)-Aryl, wobei Aryl unsubstituiert oder unabhängig voneinander ein-, zwei- oder dreifach durch R15 substituiert ist, oder
   4) Het, wobei unter Het ein 4- bis 15-gliedriges heterocyclisches Ringsystem mit 4 bis 15 Ringatomen verstanden wird, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten und
   worin Het unsubstituiert oder unabhängig voneinander ein-, zwei- oder dreifach durch ein -(C₁-C₃)-Alkyl, Halogen, -NH₂, -CF₃ oder -O-CF₃ substituiert ist, steht,
R1 für
   1) Wasserstoffatom,
   2) -(C₁-C₆)-Alkyl,
   3) -(C₁-C₆)-Alkyl-OH,
   4) -(C₀-C₄)-Alkyl-(C₃-C₆)-Cycloalkyl,
   5) -(C₁-C₁₀)-Alkyl-O-C(O)-O-R2,
   6) -(CH₂)ᵣ-(C₆-C₁₄)-Aryl, worin Aryl unsubstituiert oder unabhängig voneinander ein-, zwei- oder dreifach durch R15 substituiert ist und r die ganze Zahl Null, 1, 2 oder 3 bedeutet, oder
   7) -(CH₂)ₛ-Het, wobei unter Het ein 4- bis 15-gliedriges heterocyclisches Ringsystem mit 4 bis 15 Ringatomen verstanden wird, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten und worin Het unsubstituiert oder unabhängig voneinander ein-, zwei- oder dreifach durch R15 substituiert ist und s die ganze Zahl Null, 1, 2 oder 3 bedeutet, steht,
R2 für
   1) -(C₁-C₆)-Alkyl,
   2) -(CH₂)ᵣ-(C₆-C₁₄)-Aryl, worin Aryl unsubstituiert oder unabhängig voneinander ein-, zwei- oder dreifach durch R15 substituiert ist und r die ganze Zahl Null, 1, 2 oder 3 bedeutet, oder
   3) -(C₀-C₄)-Alkyl-(C₃-C₆)-Cycloalkyl, steht,
R3 für
   1) -(C₂-C₆)-Alkylen-NH₂, wobei Alkylen unsubstiuiert ist oder ein-, zwei-, drei- oder vierfach durch Halogen substituiert ist,
   2) -(C₁-C₄)-Alkylen-O-(C₁-C₄)-Alkylen-NH₂,
   3) -(C₁-C₄)-Alkylen-SO₂-(C₁-C₄)-Alkylen-NH₂,
   4) -(C₀-C₄)-Alkylen-Het, wobei Het wie oben definiert ist und durch -NH₂ und ein-, zwei- oder dreifach durch R15 substituiert ist,
   5) -(C₀-C₄)-Alkylen-(C₃-C₈)-Cycloalkyl-NH₂ oder
   6) -(C₀-C₆)-Alkylen-cyclisches Amin, steht
R6 für
   1) Wasserstoffatom,
   2) -(C₁-C₆)-Alkyl, wobei Alkyl unsubstituiert oder unabhängig voneinander ein-, zwei- oder dreifach durch R16 substituiert ist,
   3) -O-(C₁-C₆)-Alkyl, wobei Alkyl unsubstituiert oder unabhängig voneinander ein-, zwei- oder dreifach durch R16 substituiert ist,
   4) -(C₀-C₄)-Alkylen-Het, wobei Het wie oben definiert ist, wobei Alkylen und Het unsubstituiert oder unabhängig voneinander ein-, zwei- oder dreifach durch R16 substituiert sind,
   5) -(C₀-C₄)-Alkylen-Aryl, wobei Alkylen und Aryl unsubstituiert oder unabhängig voneinander ein-, zwei- oder dreifach durch R16 substituiert sind, oder
   6) (C₀-C₄)-Alkylen-(C₃-C₈)-Cycloalkyl, wobei Alkylen und Cycloalkyl unsubstituiert oder unabhängig voneinander ein-, zwei- oder dreifach durch R16 substituiert sind, steht,
R7 für Wasserstoffatom, Halogen oder -(C₁-C₆)-Alkyl steht,
R8 für Wasserstoffatom, Halogen oder -(C₁-C₆)-Alkyl steht,
R9 für Wasserstoffatom, Halogen oder -(C₁-C₆)-Alkyl steht,
R15 für Wasserstoffatom, -(C₁-C₄)-Alkyl, -O-CF₃, -NH₂, -OH, -CF₃ oder Halogen steht, und
R16 für -O-CF₃, -NH₂, -OH, -CF₃ oder Halogen steht.
   3) Die Erfindung betrifft ferner eine Verbindung der Formel I, wobei
X für -C(O)- oder -SO₂- steht,
U für Sauerstoffatom, Schwefelatom, NH, -C(O)-NH- oder -(C₀-C₄)-Alkylen- steht,
A für Sauerstoffatom oder -(C₀-C₄)-Alkylen- steht,
V für -(C₂-C₈)-Alkylen- oder -(C₃-C₆)-Alkenylen- steht,
D für -(C₁-C₂)-Alkylen- steht,
Y für
   1) kovalente Bindung,
   2) -(C₃-C₆)-Cycloalkyl, wobei Cycloalkyl unabhängig voneinander ein-, zwei- oder dreifach durch R15 substituiert ist,
   3) -(C₆-C₁₄)-Aryl, wobei Aryl ausgewählt ist aus der Gruppe Phenyl, Naphthyl, Anthryl oder Fluorenyl und worin Aryl unsubstituiert oder unabhängig voneinander ein-, zwei- oder dreifach durch R15 substituiert ist, oder 4) Het, wobei Het ausgewählt ist aus der Gruppe Acridinyl, Azepinyl, Azetidinyl, Aziridinyl, Benzimidazalinyl, Benzimidazolyl, Benzo[1,3]dioxolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienoimidazolyl, Thienooxazolyl, Thienopyridin, Thienothiazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl und Xanthenyl, und
   worin Het unsubstituiert oder unabhängig voneinander ein-, zwei- oder dreifach durch ein -(C₁-C₃)-Alkyl, Halogen, -NH₂, -CF₃ oder -O-CF₃ substituiert ist, steht,
R1 für
   1) Wasserstoffatom oder
   2) -(C₁-C₄)-Alkyl, steht,
R3 für
   1) -(C₂-C₆)-Alkylen-NH₂, wobei Alkylen unsubstituiert ist oder ein-, zwei-, drei- oder vierfach durch Halogen substituiert ist,
   2) -(C₁-C₄)-Alkylen-SO₂-(C₁-C₄)-Alkylen-NH₂ oder
   3) -(C₀-C₄)-Alkylen-Het, wobei Het wie oben definiert ist und durch -NH₂ und ein-, zwei- oder dreifach durch R15 substituiert ist, steht
R6 für
   1) Wasserstoffatom,
   2) -(C₁-C₆)-Alkyl, wobei Alkyl unsubstituiert oder unabhängig voneinander ein-, zwei- oder dreifach durch R16 substituiert ist,
   3) -O-(C₁-C₆)-Alkyl, wobei Alkyl unsubstituiert oder unabhängig voneinander ein-, zwei- oder dreifach durch R16 substituiert ist,
   4) -(C₀-C₄)-Alkylen-Het, wobei Het wie oben definiert ist, wobei Alkylen und Het unsubstituiert oder unabhängig voneinander ein-, zwei- oder dreifach durch R16 substituiert sind,
   5) -(C₀-C₄)-Alkylen-Aryl, wobei Alkylen und Aryl unsubstituiert oder unabhängig voneinander ein-, zwei- oder dreifach durch R16 substituiert sind, oder
   6) -(C₀-C₄)-Alkylen-(C₃-C₆)-Cycloalkyl, wobei Alkylen und Cycloalkyl unsubstituiert oder unabhängig voneinander ein-, zwei- oder dreifach durch R16 substituiert sind, steht,
R7 für Wasserstoffatom, F oder -(C₁-C₄)-Alkyl steht,
R8 für Wasserstoffatom, F oder -(C₁-C₄)-Alkyl steht,
R9 für Wasserstoffatom, F oder -(C₁-C₄)-Alkyl steht,
R15 für Wasserstoffatom, -(C₁-C₄)-Alkyl, -O-CF₃, -NH₂, -OH, -CF₃ oder Halogen steht, und
R16 für -O-CF₃, -OH, -CF₃ oder F steht.
   4) Die Erfindung betrifft ferner eine Verbindung der Formel I, wobei
X für -C(O)- steht,
U für Sauerstoffatom steht,
A für Sauerstoffatom oder -(C₀-C₄)-Alkylen- steht,
V für
   1) -(C₂-C₈)-Alkylen-, wobei Alkylen unsubstituiert oder unabhängig voneinander ein- oder zweifach durch -OH, F oder Cl substituiert ist,
   2) -(C₁-C₂)-Alkylen-Cyclopropyl-(C₁-C₂)-Alkylen-, wobei Cyclopropyl ein-oder zweifach durch F substituiert ist, oder
   3) -(C₃-C₆)-Alkenylen- steht,
D für -(C₁-C₂)-Alkylen- steht,
Y für
   1) kovalente Bindung oder
   2) Phenyl, worin Phenyl unsubstituiert oder unabhängig voneinander ein-, zwei- oder dreifach durch R15 substituiert ist, steht,
R1 für
   1) Wasserstoffatom oder
   2) -(C₁-C₄)-Alkyl, steht,
R3 für
   1) -(C₂-C₆)-Alkylen-NH₂,
   2) -(C₁-C₄)-Alkylen-SO₂-(C₁-C₄)-Alkylen-NH₂ oder
   3) -(C₀-C₄)-Alkylen-Pyridyl, wobei Pyridyl durch -NH₂ und ein-, zwei- oder dreifach durch R15 substituiert ist, steht
R6 für
   1) Wasserstoffatom,
   2) -(C₁-C₆)-Alkyl,
   3) -CF₃,
   4) -(C₀-C₄)-Alkylen-Phenyl oder
   5) -(C₀-C₄)-Alkylen-(C₃-C₆)-Cycloalkyl steht,
R7, R8 und R9 jeweils für Wasserstoffatom stehen und
R15 für Wasserstoffatom, -(C₁-C₄)-Alkyl, -CF₃ oder Halogen steht.
   5) Die Erfindung betrifft ferner eine Verbindung der Formel I, wobei
X für -C(O)- steht,
U für Sauerstoffatom steht,
A für Sauerstoffatom oder -(C₀-C₄)-Alkylen- steht,
V für -(C₂-C₈)-Alkylen- oder -(C₃-C₆)-Alkenylen- steht,
D für -(C₁-C₂)-Alkylen- steht,
Y für
   1) kovalente Bindung oder
   2) Phenyl, worin Phenyl unsubstituiert oder unabhängig voneinander ein-, zwei- oder dreifach durch R15 substituiert ist, steht,
R1 für
   1) Wasserstoffatom oder
   2) -(C₁-C₄)-Alkyl, steht,
R3 für
   1) -(C₂-C₆)-Alkylen-NH₂,
   2) -(C₁-C₄)-Alkylen-SO₂-(C₁-C₄)-Alkylen-NH₂ oder
   3) -(C₀-C₄)-Alkylen-Pyridyl, wobei Pyridyl durch -NH₂ und ein-, zwei- oder dreifach durch R15 substituiert ist, steht
R6 für
   1) Wasserstoffatom,
   2) -(C₁-C₆)-Alkyl,
   3) -CF₃,
   4) -(C₀-C₄)-Alkylen-Phenyl oder
   5) -(C₀-C₄)-Alkylen-(C₃-C₆)-Cycloalkyl steht,
R7, R8 und R9 jeweils für Wasserstoffatom stehen und
R15 für Wasserstoffatom, -(C₁-C₄)-Alkyl, -CF₃ oder Halogen steht.

Unter dem Begriff "(C₁-C₆)-Alkyl" werden Kohlenwasserstoffreste verstanden, deren Kohlenstoffkette geradkettig oder verzweigt ist und 1 bis 6 Kohlenstoffatome enthält, beispielsweise Methyl, Ethyl, Propyl, Iso-Propyl, Butyl, Iso-Butyl, tertiär-Butyl, Pentyl, Iso-Pentyl, Neopentyl, Hexyl, 2,3-Dimethylbutan oder Neohexyl.

Unter dem Begriff "-(C₀-C₄)-Alkylen-" werden Kohlenwasserstoffreste verstanden, deren Kohlenstoffkette geradkettig oder verzweigt ist und 1 bis 4 Kohlenstoffatome enthält, beispielsweise Methylen, Ethylen, Propylen, Iso-Propylen, Iso-Butylen, Butylen oder tertiär-Butylen. "-C₀-Alkylen" ist eine kovalente Bindung.

Unter dem Begriff "-(C₃-C₉)-Alkylen-" werden Kohlenwasserstoffreste verstanden, deren Kohlenstoffkette geradkettig oder verzweigt ist und 3 bis 9 Kohlenstoffatome enthält, beispielsweise Propylen, Iso-Propylen, Butylen, Iso-Butylen, Pentylen, Iso-Pentylen, Neopentylen, Hexylen, 2,3-Dimethylbutanylen, Neohexylen, Heptylen, Octanylen oder Nonanylen.

Unter dem Begriff "-(C₃-C₉)-Alkenylen" werden Kohlenwasserstoffreste verstanden, deren Kohlenstoffkette geradkettig oder verzweigt ist und 3 bis 9 Kohlenstoffatome enthält und je nach Kettenlänge 1, 2 oder 3 Doppelbindungen aufweisen, beispielsweise Ethenylen, Propenylen, Iso-Propenylen, Iso-Butenylen oder Butenylen; die Substituenten an der Doppelbindung können, sofern die prinzipielle Möglichkeit besteht, E- oder Z-ständig angeordnet sein.

Unter dem Begriff "(C₃-C₁₂)-Cycloalkyl" werden Reste verstanden wie Verbindungen, die sich von 3- bis 12-gliedrige Mono-, Bi- oder Tricyclen oder überbrückten Cyclen wie den Monocyclen Cyclopropan, Cyclobutan, Cyclopentan, Cyclohexan, Cycloheptan oder Cyclooctan herleiten, die sich von den Bicyclen Bicyclo[4.2.0]octan, Octahydro-inden, Decahydro-naphthalen, Decahydro-azulen, Decahydro-benzocyclohepten oder Dodecahydro-heptalen, oder von Tricyclen wie Adamantan herleiten oder von den überbrücken Cyclen wie Spiro[2.5]octan, Spiro[3.4]octan, Spiro[3.5]nonan, Bicyclo[3.1.1]heptan, Bicyclo[2.2.1]heptan, Bicyclo[2.2.2]octan oder Octahydro-4,7-methan-inden herleiten.

Unter dem Begriff "-(C₆-C₁₄)-Aryl" werden aromatische Kohlenstoffreste verstanden mit 6 bis 14 Kohlenstoffatomen im Ring. -(C₆-C₁₄)-Arylreste sind beispielsweise Phenyl, Naphthyl, zum Beispiel 1-Naphthyl, 2-Naphthyl, 1,2,3,4-Tetrahydronaphthalenyl, Anthryl oder Fluorenyl. Naphthylreste und insbesondere Phenylreste sind bevorzugte Arylreste.

Unter dem Begriff "4- bis 15-gliedriges heterocyclisches Ringsystem" oder "Het" werden Ringsysteme verstanden mit 4 bis 15 Ringatomen, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten. Beispiele für diese Ringsysteme sind die Reste Acridinyl, Azepinyl, Azetidinyl, Aziridinyl, Benzimidazalinyl, Benzimidazolyl, Benzo[1,3]dioxol, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furaxanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienoimidazolyl, Thienooxazolyl, Thienopyridin, Thienothiazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl und Xanthenyl.

Bevorzugte Het-Ringe sind die Reste Isoxazolyl, Benzo[1,3]dioxol, Thiophenyl, Imidazol und Thiazol.

Unter dem Begriff "Halogen" wird Fluor, Chlor, Brom oder Jod verstanden, bevorzugt sind Fluor, Chlor oder Brom, insbesondere Chlor oder Brom.

Unter dem Begriff "-SO₂-" wird ein Sulfonyl-Rest verstanden.

Unter dem Begriff "-C(O)-" wird ein Carbonyl-Rest verstanden.

Unter dem Begriff "cyclisches Amin" werden Ringsysteme verstanden wie cyclische Amine beispielsweise Azetidinyl, Pyrrolidinyl, Piperidinyl, Piperazinyl, Azepinyl, Morpholinyl oder Thiomorpholinyl.

Die Erfindung betrifft ferner ein Verfahren zur Herstellung der Verbindung der Formel I, das dadurch gekennzeichnet, dass man
a) eine Verbindung der Formel (II) 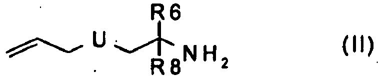 wobei U, R6 und R8 die in der Verbindung der Formel (I) genannten Bedeutungen haben mit einer Aminosäure der Formel (III) umsetzt, 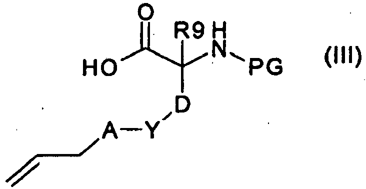 worin R9, A, Y und D die in der Verbindung der Formel (I) genannten Bedeutungen haben, wobei eine Verbindung der Formel (IV) erhalten wird, 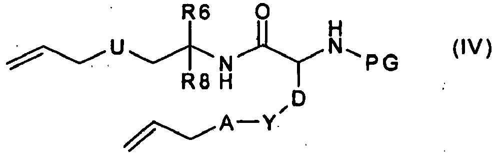 welche unter den Bedingungen der Ringschlußmetathese und anschließender Hydrierung der entstandenen Doppelbindung zu einer Verbindung der Formel (V) umgesetzt wird, 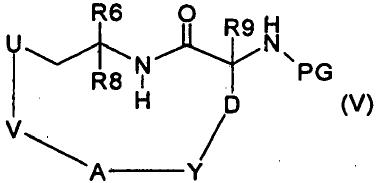 wobei V für -(C₂-C₉)-Alkylen- oder -(C₃-C₉)-Alkenylen- steht, anschließend die Schutzgruppe PG abspaltet und die Verbindung der Formel (VI) erhält, 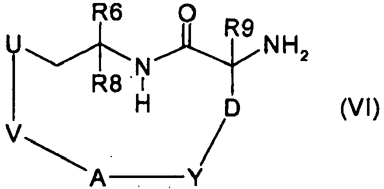 und mit einer Verbindung der Formel (VII) 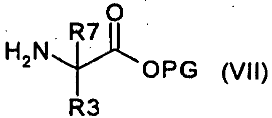 worin R3 und R7 die in Formel (I) genannten Bedeutungen haben, PG einen geeigneten Esterschutzgruppenrest darstellt, sowie der Stickstoff in R3 gegebenfalls durch eine geeignete Aminoschutzgruppe geschützt ist, mit Phosgen oder eines Phosgenäquivalents zu einer Verbindung der Formel (VIII) 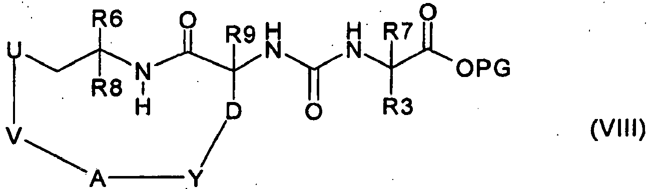 umsetzt, anschließend werden die Schutzgruppe PG und die gegebenenfalls vorhandene Schutzgruppe am Stickstoff in R3 abgespalten, wobei die Verbindung der Formel (I) erhalten wird, oder
b) eine Verbindung der Formel (IX) 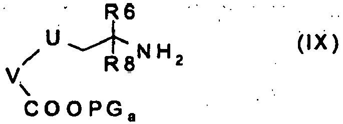 worin U, V, R6 und R8 die in der Verbindung der Formel (I) genannten Bedeutungen haben und PGₐ eine geeignete Carboxylschutzgruppe darstellt, mit einer Aminosäure der Formel (X) umsetzt, 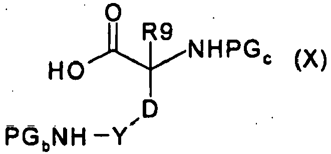 worin R9, Y und D die in der Verbindung der Formel I genannten Bedeutungen haben und PG_{b} und PG_{c} geeignete Aminoschutzgruppen sind, wobei eine Verbindung der Formel (XI) erhalten wird, 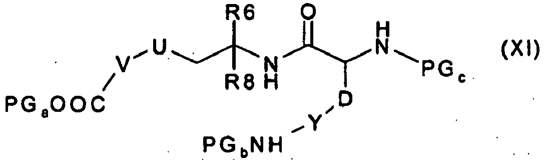 welche nach Abspaltung der Schutzgruppen PGₐ und PG_{b} in die Verbindung der Formel (XII) überführt wird, 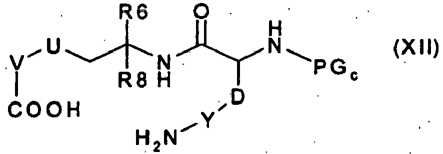 welche mit Hilfe einer Amidkupplung zu einer Verbindung der Formel (V) umgesetzt wird,
   worin A die in der Verbindung der Formel I genannten Bedeutungen hat, anschließend wird die Scutzgruppe abgespalten und die Verbindung der Formel (VI) erhalten,
   und mit einer Verbindung der Formel (VII)
   worin R3 und R7 die in Formen I genannten Bedeutungen haben, PG einen geeigneten Esterschutzgruppenrest darstellt, sowie der Stickstoff in R3 gegebenfalls durch eine geeignete Aminoschutzgruppe geschützt ist, mit Phosgen oder eines Phosgenäquivalents zu einer Verbindung der Formel (VIII) umgesetzt, anschließend werden die Schutzgruppe PG und die gegebenenfalls vorhandene Schutzgruppe am Stickstoff in R3 abgespalten, wobei die Verbindung der Formel (I) erhalten wird, oder
c) eine Verbindung der Formel (XIII) 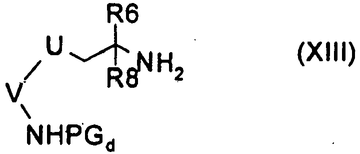 worin U, V, R6 und R8 die in der Verbindung der Formel I genannten Bedeutungen haben und PG_{d} eine geeignete Aminoschutzgruppe darstellt, mit einer Aminosäure der Formel (XIV) umsetzt, 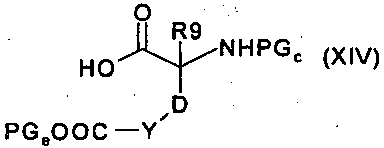 worin R9, Y und D die in der Verbindung der Formel I genannten Bedeutungen haben und PG_{c} eine geeignete Aminoschutzgruppe und PGₑ eine geeignete Carboxylschutzgruppe ist, wobei eine Verbindung der Formel (XV) erhalten wird, 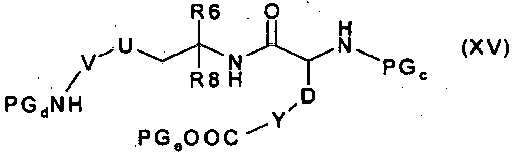 welche nach Abspaltung der Schutzgruppen PG_{d} und PGₑ in die Verbindung der Formel (XVI) überführt wird, 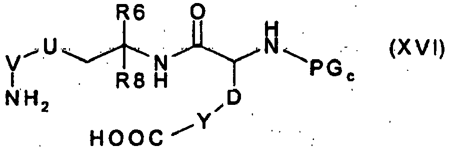 welche zu einer Verbindung der Formel (V) umgesetzt wird,
   worin A die in der Verbindung der Formel I genannten Bedeutungen hat, anschließend wird die Schutzgruppe abgespalten und die Verbindung der Formel (VI) wird erhalten,
   und mit einer Verbindung der Formel (VII),
   worin R3 und R7 die in Formel I genannten Bedeutungen haben, PG einen geeigneten Esterschutzgruppenrest darstellt, sowie der Stickstoff in R3 gegebenfalls durch eine geeignete Aminoschutzgruppe geschützt ist, mit Phosgen oder eines Phosgenäquivalents zu einer Verbindung der Formel (VIII) umgesetzt, anschließend werden die Schutzgruppe PG und die gegebenenfalls vorhandene Schutzgruppe am Stickstoff in R3 abgespalten, wobei die Verbindung der Formel (I) erhalten wird, oder
d) eine Verbindung der Formel XVII, 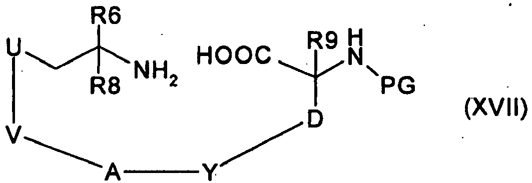 worin U, V, A, Y, D, R₆, R₈ und R₉ die in der Verbindung der Formel I genannten Bedeutungen haben, mit einer Verbindung der Formel (V) umsetzt, anschließend werden die Schutzgruppen abgespalten und eine Verbindung der Formel (VI) wird erhalten,
   und mit einer Verbindung der Formel (VII),
   worin R3 und R7 die in Formel I genannten Bedeutungen haben, PG einen geeigneten Esterschutzgruppenrest darstellt, sowie der Stickstoff in R3 gegebenfalls durch eine geeignete Aminoschutzgruppe geschützt ist, mit Phosgen oder eines Phosgenäquivalents zu einer Verbindung der Formel (VIII) umgesetzt, und anschließend werden die Schutzgruppen PG und gegebenenfalls die Schutzgruppe am Stickstoff in R3 abgespalten, wobei die Verbindung der Formel (I) erhalten wird, oder
e) eine Verbindung der Formel (VIIIa) 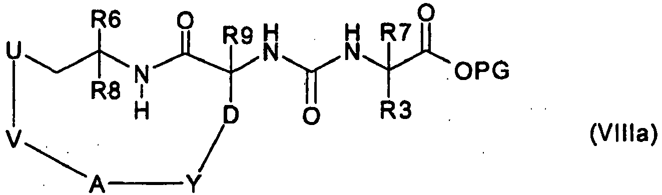 worin V für -(C₃-C₉)-Alkenylen- steht in die Verbindung der Formel (VIIIb) umwandelt, 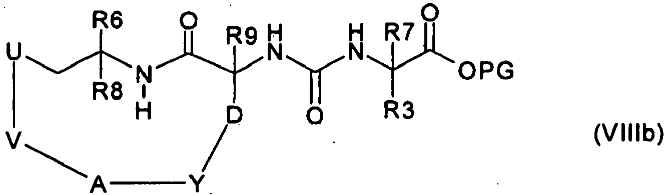 worin V für -(C₃-C₉)-Alkylen- steht, wobei Alkylen unabhängig voneinander ein-, zwei- oder dreifach durch -OH, NH₂ oder Halogen substituiert ist, oder für -(C₁-C₂)-Alkylen-(C₃-C₆)-Cycloalkyl-(C₁-C₂)-Alkylen- steht, wobei Cycloalkyl unabhängig voneinander ein-, zwei- oder dreifach durch R15 substituiert ist, anschließend wird die Verbindung der Formel (VIIIb) analog zu Verfahren a) in die Verbindung der Formel (I) umgesetzt, oder
f) eine Verbindung der Formel (XVIII) 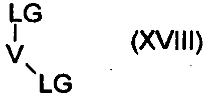 worin V wie in der Verbindung der Formel (I) definiert ist, nacheinander mit den Verbindungen der Formel (XIX) und (XX) 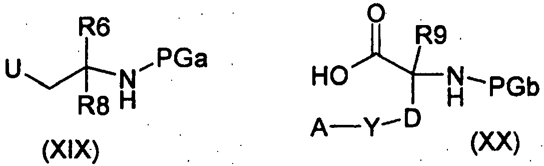 unter Einsatz von Basen in polaren, aprotischen Lösungsmitteln umsetzt und die erhaltenen Verbindungen der Formel (XXI) 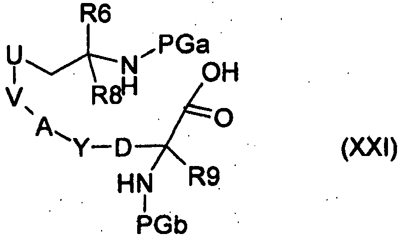 durch Entfernen der Schutzgruppe PGa und anschließender Bildung einer Peptidbindung in eine Verbindung der Formel (V) umwandelt, und diese wie in Verfahren a) zu Verbindungen der Formel (I) umsetzt, worin R6, R8, R9 sowie A, D, U, V und Y die in Formel (I) genannten Bedeutungen haben und PG geeignete Schutzgruppen darstellt sowie LG für eine Abgangsgruppe wie Chlor, Brom, Iod oder Sulfonsäureester steht, oder
g) eine Verbindung der Formel VI mit einer Verbindung der Formel (XXII), 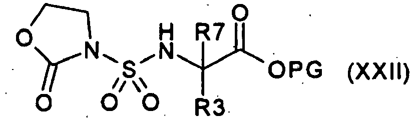 worin R3 und R7 die in der Verbindung der Formel I genannten Bedeutungen haben und PG einen geeigneten Schutzgruppenrest darstellt, zu einer Verbindung der Formel (XXIII) umsetzt, 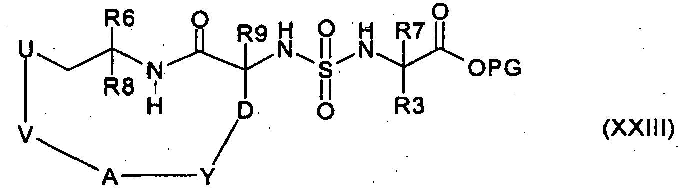 und dann in eine Verbindung der Formel (I) überführt, oder
h) eine nach den Verfahren a), b), c), d), e), f) oder g) hergestellte Verbindung der Formel I, oder eine geeignete Vorstufe der Formel (I), die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
i) die nach den Verfahren a), b), c), d), e), f) oder g) hergestellte Verbindung der Formel (I) entweder in freier Form isoliert oder im Falle des Vorliegens von sauren oder basischen Gruppen in physiologisch verträgliche Salze umwandelt.

Die in den Verfahren a) bis f) beschriebenen Herstellungsmöglichkeiten lassen sich in der Reihenfolge der Verfahrensschritte variieren und sind nicht auf die so beschriebenen Verfahren beschränkt; so kann beispielsweise die Harnstoff- oder Sulfamidbindung zuerst geknüpft werden zu Verbindungen des Typs XII (hier dargestellt für den Harnstofftyp), anschließend entschützt und mit einer Verbindung vom Typ II zu Verbindungen des Typs XIV umgesetzt werden, die dann analog VIII und weiter die erfindungsgemäßen Verbindungen I ergeben.

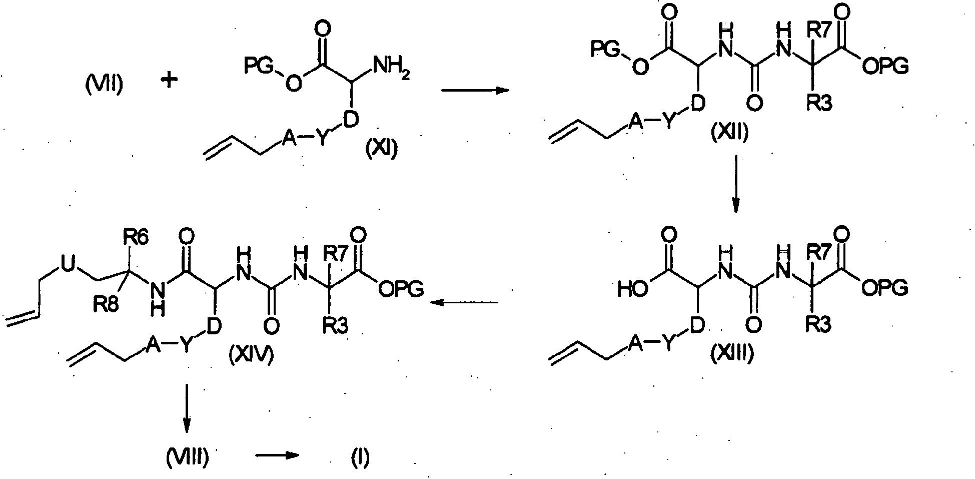

Die Herstellung von Verbindungen des Typs II ist beispielsweise durch direkte Allylierung von kommerziell erhältlichen Aminoalkoholen unter Einsatz von Allylierungsmitteln wie Allylhalogeniden oder Allylsulfonsäureestern in polaren aprotischen Lösunsmitteln wie Tetrahydrofuran (THF) oder Dimethylformamid (DMF) unter Einsatz starker Basen wie Natriumhydrid, Lithiumhexamethyldisilazan oder Alkalicarbonaten beschrieben in Organic Preparations and Procedures International, 34 (5) 511-514. Alternativ kann die Aminogruppe zuvor geschützt werden z.B. durch Bildung der Schiffschen Basen, wie in Journal of the Chemical Society, Perkin Transactions 1: Organic and Bio-Organic Chemistry, (14), 2139-2145; 1997, beschrieben. Die Entfernung der Schutzgruppe kann in sauren Medien, bevorzugt verdünnte Salzsäure, in wasserlöslischen organischen Lösungsmitteln wie THF oder Methanol oder Gemischen mehrerer derartiger Lösungsmittel erfolgen. Verbindungen des Typs III sind kommerziell erhältlich oder können aus kommerziell erhältlichen Aminosäurederivaten wie oben beschrieben durch Allylierung an einem Atom, vorzugsweise an einem Heteroatom der Seitenkette gewonnen werden. Verbindungen des Typs VII sind kommerziell erhältlich oder können durch Standardverfahren der Schutzgruppenchemie erhalten werden (siehe unten).

Verbindungen des Typs IX können durch Standardverfahren der organischen Chemie hergestellte werden, wie durch Michael-Addtition von substituierten β-Aminoalkoholen an α,β-ungesättigte Esterverbindungen in Gegenwart von katalytischen Mengen Natrium (J. Org. Chem. Vol. 69, No. 5, 2004, 1716-1719) 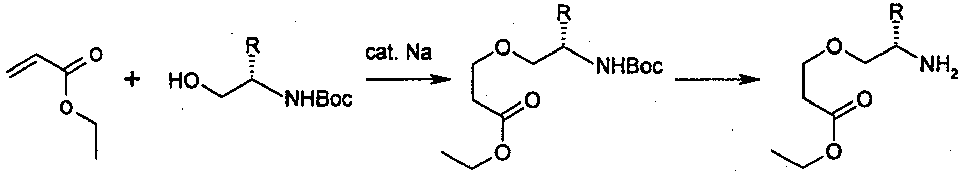 oder aber durch Lewis-Säure katalysierte Addition von entsprechend substituierten Alkoholen an geschützte Aziridine.

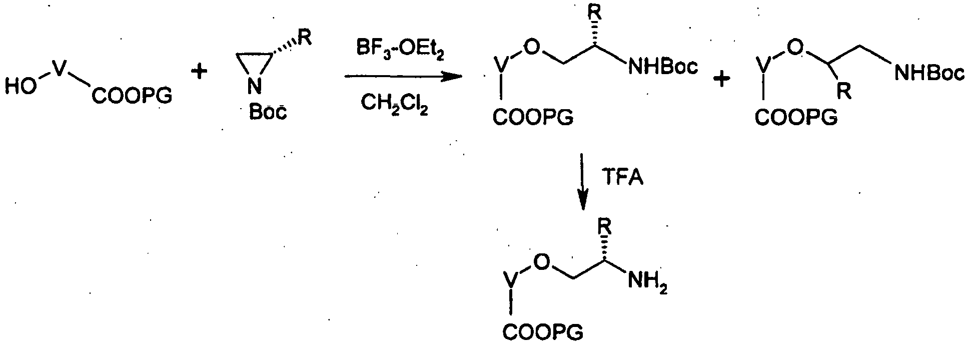

Verbindungen des Typs X sind in der Regel kommerziell erhältlich, wie 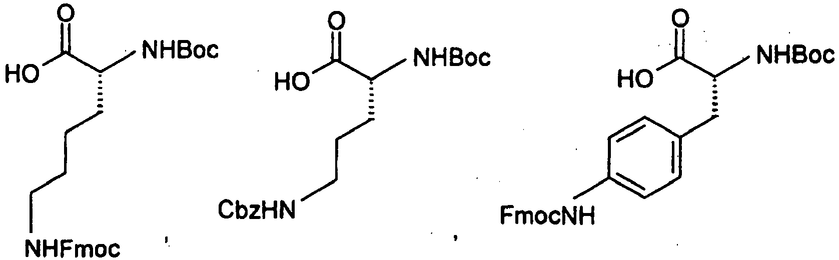 oder lassen sich aus diesen durch Standardtransformationen herstellen. Ebenso sind Verbindungen des Typs XIV in der Regel kommerziell erhältlich, wie 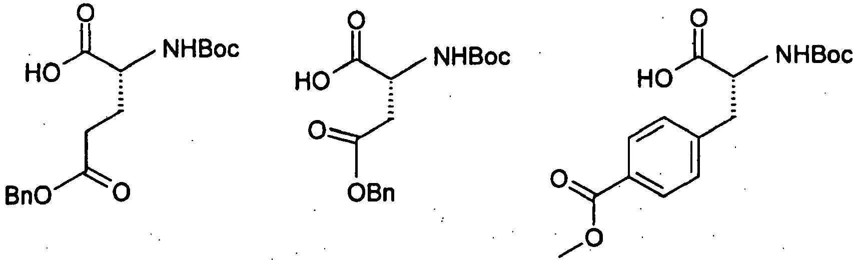 oder lassen sich aus diesen durch Standardtransformationen herstellen. Verbindungen des Typs XIII können, wie oben beschrieben, durch Lewis-Säure katalysierte Addition von entsprechenden Aminoalkoholen an geschützte substituierte Aziridine erhalten werden, 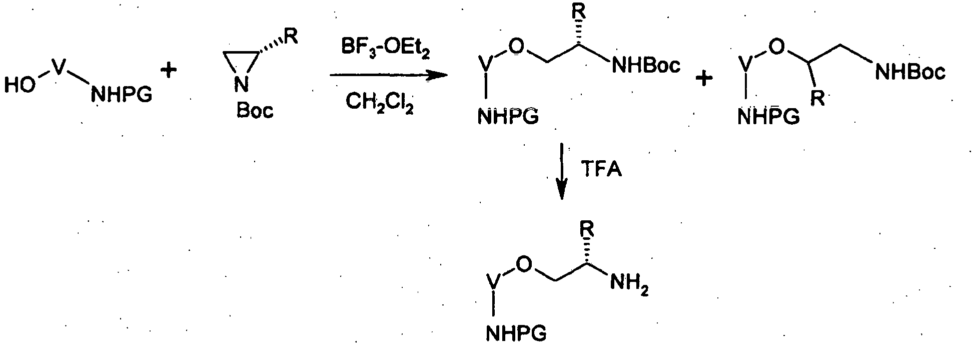 wie es in Tetrahedron Letters, Vol. 34, No. 41, 1993, 6513-6516 beschrieben ist.

Die Herstellung von 2-Oxo-oxazolidin-3-sulfonsäureamiden des Typs XVIII und die nachfolgende Umwandlung in Sulfamide ist beispielsweise in Organic Process Research & Development (2006), 10(4), 770-775 beschrieben und kann hier aus bekannten Aminosäurederivaten vorgenommen werden.

Methoden zur Peptidkupplung sind etwa bei Bodanszky (M. Bodanszky, Principles of Peptide Synthesis, 2nd ed, Springer, Berlin, 1993) beschrieben. Beispielsweise, seien aus der Vielfalt bekannter Methoden die Carbonsäureaktivierungen durch das Carbodiimidverfahren (J. Am. Chem. Soc., 1955, 77, 1067) und durch Aktivesterverfahren wie beispielsweise durch Phosphoniumsalze (Tetrahedron Lett., 1975, 14; Int, J. Peptide Protein Res. 1988, 31, 231) oder Uroniumsalze (Tetrahedron Lett., 1978, 1269, Tetrahedron Lett. 1989, 30, 1927, J. Chem. Soc., Chem. Commun. 1994, 201). Schutzgruppen, deren Einführung, Abspaltung und Stabilität sind beispielsweise bei Greene (T. W. Greene, P. G. M. Wuts, Protective Groups in Organic Synthesis, 3rd ed, Wiley, New York, 1999) beschrieben. Bevorzugte Schutzgruppen sind beispielweise Aminoschutzgruppen wie tert-Butyloxycarbonyl-, Fluorenyloxycarbonyl-, Benzyloxycarbonyl-, Benzyliden- oder Diphenylmethylidenschutzgruppen, bevorzugte Carboxylschutzgruppen sind Alkylester wie Methyl-, Ethyl-, Benzyl- oder Silylester wie Trimethylsilylester. Die Herstellung von Harnstoffen ist beispielsweise bei G. Sartori; R. Maggi, Acyclic and cyclic ureas Science of Synthesis (2005), 18, 665-758, im Detail beschrieben. Die beiden Aminokomponenten werden hierbei in polaren oder unpolaren aprotischen Lösungsmitteln wie DMF, Dichlormethan, Acetonitril gegebenenfalls unter Zusatz einer Base wie Alkalicarbonat oder organischen Stickstoffbasen, bevorzugt Triethylamin oder Diisopropylethylamin und gegebenenfalls einem Acylierungskatalysator wie Dimethylaminopyridin mit Phosgen, Triphosgen, Diphosgen oder Phosgenäquivalenten wie 1,1'-Carbonyldiimidazol oder Disuccidinylcarbonat oder Chlorameisensäureestern unter Bildung aktivierter Harnstoffvorläufer umgesetzt. Die Bildung der Harnstoffverknüpfung kann auch über die Verwendung der entsprechenden Isocyanate einer der beiden Kupplungspartner erfolgen. Unter Ringschlußmetathese (Olefinmetathese) wird die ringschließende Kohlenstoff-Kohlenstoffverknüpfung zwischen zwei Alkenylgruppen durch Einwirkung von Metallocarben-Katalysatoren nach Grubbs, Hoyveda-Grubbs oder Schrock in Lösungsmitteln wie beispielsweise Dichlormethan, Benzol, Pentan oder THF unter Abspaltung von Ethylen verstanden, vorzugsweise bei Temperaturen zwischen 25 und 50° C, und wird beispielsweise in Angew. Chem., Int. Ed. Engl. 1995, 34, 2039-2041; Acc. Chem. Res. 2001, 34, 18-29; Handbook of Metathesis, Grubbs, R. H. Ed., Wiley-VCH, Weinheim, Germany, 2003; Angew. Chem. Int. Ed. 2003, 42, 1900-1923; Angew. Chem., Int. Ed. 2005, 44, 4490-4527 beschrieben. Unter Hydrierung wird die wohlbekannte Wasserstoffübertragung auf ungesättigte Kohlenstoff-Kohlenstoff-Bindung in geeingten Lösungsmittels wie niederen Alkoholen, Ethern und Estern durch Einwirkung einer Wasserstoffatmosphäre und Einsatz von übergangsmetallen als Katalysatoren verstanden. Diese und weitere Modifikationen der Kohlenstoffmehrfachbinung wie Hydroborierung, Oxymercurierung und Epoxidierung sind im Detail beschrieben z.B. in Organikum, Wiley-VCH, 22. Auflage, S. 288ff. Die Dihydroxylierung einer Doppelbindung kann beispielsweise durch eine Sharpless-Dihydroxylierung mit Umsetzung einer Kohlenstoffmehrfachbindung zu 1,2-Diolen unter Verwendung eines Osmium-Katalysators, eines stöchiometrischen Oxidationsmittels wie K₃Fe(CN)₆ in einem Puffer, gegebenenfalls unter Zusatz chiraler Liganden oder vorgefertigter Mischungen wie AD-Mix in geeigneten Lösungsmitteln durchgeführt werden. Cyclopropanierungen werden an Kohlenstoffmehrfachbindungen durch Einsatz von Carbenquellen in inerten Lösungsmitteln wie Ethern durchgeführt, beispielsweise nach Simmons-Smith (Dihalogenmethan und Zink/Kupferpaar) oder Furukawa (Dihalogenmethan und Diethylzink) wie beispielsweise in J. Am. Chem. Soc., 1958, 80, 5323-5324 beschrieben; Difluorcyclopropanierungen können wie in J. Fluorine Chem. 2004, 125, 459 beschrieben, beispielsweise durch Erhitzen mit Trimethylsilylfluorosulfonyldifluoracetat und katalytischen Mengen an Fluorid in Substanz oder in inerten hochsiedenden Lösungsmittel durchgeführt werden. Aminosäurederivate, die eine Sulfongruppe in der Seitenkette tragen, können beispielsweise durch Oxidation der entsprechenden Cysteinderivate oder anderer schwefelhaltiger Seitenketten in inerten Lösungsmitteln mit Oxidationsmitteln wie beispielsweise Oxon oder m-Chlorperbenzoesäure erhalten werden. Aminosäurederivate, die einen Het-Alkylidenrest in der Seitenkette tragen, sind entweder kommerziell erhältlich oder können durch Alkylierung nach folgendem Schema unter Bedingungen wie oben bei der Allylierung beschrieben, erhalten werden, 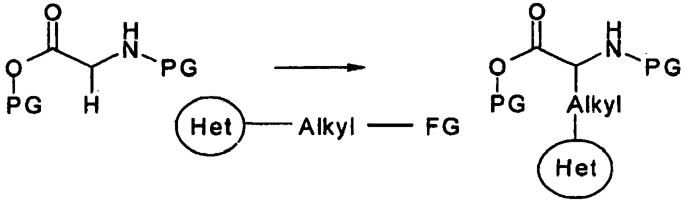 wobei FG eine geeignete Fluchtgruppe wie Halogen oder Sulfonsäureester und PG eine geeignete Schutzgruppe darstellt. Die Enantiomeren dieser Aminosäuren können beispielsweise durch chirale Chromatographie erhalten werden. Alternativ können die Derivate durch Kondensation der heterocyclischen Aldehyde mit Aminosäuren oder Aminomalonsäurederivaten gegebenenfalls unter Zusatz von Mineralsäuren oder organischen Säurekatalysatoren oder wasserziehenden Mitteln wie Orthoameisensäureestern oder anorganischen Sulfaten und anschließende Hydrierung gegebenenfalls mit chiralen Hydrierkatalysatoren gewonnen werden 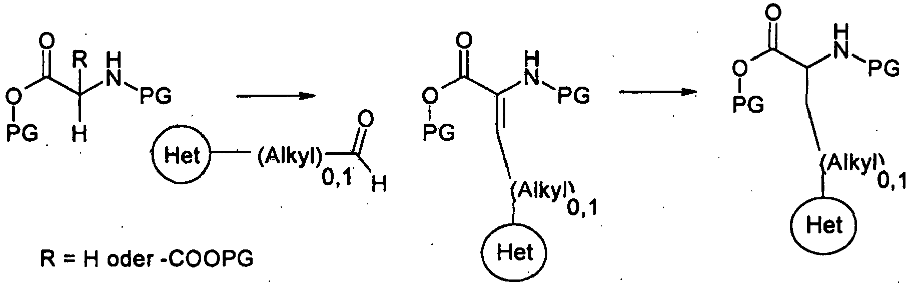 wobei PG eine geeignete Schutzgruppe darstellt.

Eine nach diesen Verfahren hergestellte Verbindung der Formel I, oder eine geeignete Vorstufe der Formel I, die aufgrund ihrer chemischen Struktur in enantiomeren Formen auftritt, kann durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren aufgetrennt werden (Verfahren b), oder
die nach Schema 1 oder 3 hergestellte Verbindung der Formel I kann entweder in freier Form isoliert oder im Falle des Vorliegens von sauren oder basischen Gruppen in physiologisch verträgliche Salze umwandelt werden (Verfahren d).

Im Verfahrensschritt h) wird die Verbindung der Formel I, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formel I zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D-und L-Milchsäure sowie (+) und (-)-Mandelsäure in die reinen Enantiomeren überführen. Auch kann man chirale Verbindungen, die Alkohol- oder Amin-funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Weiterhin ergibt sich bei einigen der erfindungsgemäßen Verbindungen die Möglichkeit, zur Herstellung der Gerüststrukturen diastereo- oder enantiomerenreine Ausgangsprodukte einzusetzen. Dadurch können auch andere oder vereinfachte Verfahren zur Aufreinigung der Endprodukte eingesetzt werden. Diese Ausgangsprodukte wurden zuvor nach literatur-bekannten Verfahren enantiomeren- oder diastereomerenrein hergestellt. Das kann insbesondere bedeuten, dass in der Synthese der Grundgerüste entweder enantioselektive Verfahren zum Einsatz kommen, oder aber eine Enantiomeren- (oder Diastereomeren-) Trennung auf früher Synthesestufe und nicht erst auf der Stufe der Endprodukte durchgeführt wird. Ebenso kann eine Vereinfachung der Trennungen dadurch erreicht werden, dass zwei- oder mehrstufig vorgegangen wird.

Saure oder basische Produkte der Verbindung der Formel I können in Form ihrer Salze oder in freier Form vorliegen. Bevorzugt sind pharmakologisch verträgliche Salze, beispielsweise Alkali- oder Erdalkalimetallsalze, Hydrochloride, Hydrobromide, Sulfate, Hemisulfate, alle möglichen Phosphate sowie Salze der Aminosäuren, natürlicher Basen oder Carbonsäuren.
Die Herstellung physiologisch verträglicher Salze aus zur Salzbildung befähigten Verbindungen der Formel I, einschließlich deren stereoisomeren Formen, gemäß Verfahrensschritt h) erfolgt in an sich bekannter Weise. Die Verbindungen der Formel I bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron- Palmitin-, oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur Prophylaxe, Sekundär-Prevention und Therapie all solcher Erkrankungen, die durch eine Hemmung von TAFIa behandelbar sind. So eignen sich TAFIa Inhibitoren sowohl für eine prophylaktische als auch für eine therapeutische Anwendung am Menschen. Sie eignen sich sowohl für eine akute Behandlung als auch für eine Langzeittherapie. TAFIa Inhibitoren können eingesetzt werden in Patienten, die an Störungen des Wohlbefindens oder Krankheiten leiden, die mit Thrombosen, Embolien, Hyperkoagulabilität oder fibrotischen Veränderungen einhergehen.

Dazu gehören der Myokardinfarkt, die Angina pectoris und alle anderen Formen des akuten Koronarsyndroms, der Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung, Angioplastie und ähnlichen Eingriffen wie Stentimplantationen und Bypass-Operationen. Weiterhin können TAFIa Inhibitoren eingesetzt werden bei allen Eingriffen, die zu einem Kontakt des Blutes mit Fremdoberflächen führen wie z. B. bei Dialysepatienten und Patienten mit Verweilkathetern. TAFIa Inhibitoren können eingesetzt werden, um die Thrombosegefahr nach chirurgischen Eingriffen wie bei Knie- und Hüftgelenksoperationen zu reduzieren.

TAFIa Inhibitoren eignen für die Behandlung von Patienten mit disseminierter intravaskulärer Koagulation, Sepsis und anderen intravaskulären Ereignissen, die mit einer Entzündung einhergehen. Weiterhin eignen sich TAFIa Inhibitoren für die Prophylaxe und Behandlung von Patienten mit Atherosklerose, Diabetes und dem metabolischen Syndrom und deren Folgen. Störungen des hämostatischen Systems (beispielsweise Fibrinablagerungen) wurden impliziert in Mechanismen, die zu Tumorwachstum und Tumormetastasierung führen, sowie bei entzündlichen und degenerativen Gelenkserkrankungen wie der rheumatoiden Arthritis und der Arthrose. TAFIa Inhibitoren eignen sich zur Verlangsamung oder Verhinderung solcher Prozesse.

Weitere Indikationen für den Einsatz von TAFIa Inhibitoren sind fibrotische Veränderungen der Lunge wie die chronische obstruktive Lungenerkrankung, das adult respiratory distress syndrome (ARDS) und des Auges wie Fibrinablagerungen nach Augenoperationen. TAFIa Inhibitoren eignen sich auch zur Verhinderung und/oder Behandlung von Narbenbildung.

Die Applikation der erfindungsgemäßen Arzneimittel kann durch orale, inhatative, rektale oder transdermale Applikation oder durch subkutane, intfaartikuläre, intraperitoneale oder intravenöse Injektion erfolgen. Bevorzugt ist die orale Applikation. Eine Beschichtung mit TAFIa Inhibitoren von Stents und anderen Oberflächen, die im Körper mit Blut in Kontakt kommen, ist möglich.

Die Erfindung betrifft auch ein Verfahren zur Herstellung eines Arzneimittels, das dadurch gekennzeichnet, dass man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylen glykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind je nach Wirksamkeit der Verbindung gemäß Formel I, Tagesdosen von etwa 2 mg bis 1000 mg Wirkstoff, bevorzugt etwa 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

TAFIa Inhibitoren können sowohl als Monotherapie als auch in Kombination oder gemeinsam mit allen Antithrombotika (Antikoagulanzien und Plättchenaggregationshemmer), Thrombolytika (Plasminogenaktivatoren jeglicher Art), anderen profibrinolytisch wirksamen Substanzen, Blutdrucksenkern, Regulatoren des Blutzuckers, Lipidsenkern und Antiarrhythmika verabreicht werden.

### Beispiele

Endprodukte werden in der Regel durch massenspektroskopische Methoden (FAB-, ESI-MS) und ¹H-NMR bestimmt, angegeben sind jeweils der Hauptpeak oder die beiden Hauptpeaks. Temperaturangaben in Grad Celsius, Ausb. bedeutet Ausbeute, RT bedeutet Raumtemperatur (21 °C bis 24 °C), TFA bedeutet Trifluoressigsäure, THF bedeutet Tetrahydrofuran, DMF bedeutet Dimethylformamid, HATU bedeutet 2-(7-Aza-1H-benzotriazol-1-yl)-1,1,3,3-tetramethyluroniumhexafluorophosphat, HOAt bedeutet 1-Hydroxy-7-Azabenzotriazol. Verwendete Abkürzungen sind entweder erläutert oder entsprechen den üblichen Konventionen.

Wenn nicht anders aufgeführt, wurden die LC/MS-Analysen unter folgenden Bedingungen durchgeführt:
Methode A: = Methode Säule: YMC Jsphere ODS H80 20 x 2 mm, Packungsmaterial 4 µm, Laufmittel: CH₃CN: H₂O + 0,05% Trifluoressigsäure (TFA), Gradient: 4:96 (0 min.) nach 95:5 (2,0 min.) nach 95:5 (2,4 min) nach 4:96 (2,45 min) Fluß: 1,0 mL/min. Temperatur: 30 °C.
Methode B: Säule: Phenomenex LunaC₁₈ 10 x 2,0 mm, Packungsmaterial 3 µm, Laufmittel: CH₃CN: H₂O + 0,05% TFA, Gradient: 7:93 (0 min) nach 95:5 (1,2 min) nach 95:5 (1,4 min) nach 7:93 (1,45 min), Fluß: 1,1 mL/min, Temperatur: 30 C. Methode C: Säule: WatersXBridgeC₁₈, 4,6*50 mm, 2,5 µm, Gradient: H₂O + 0,05% TFA: CH₃CN + 0,05% TFA 95:5 (0 min) nach 95:5 (0,3 min) nach 5:95 (3,5 min) nach 5:95 (4 min), Fluß: 1,3 mL/min, Temperatur: 40 C.
Methode D: Säule: Waters XBridge C₁₈ 4,6*50 mm; 2,5 µm Gradient: H₂O + 0,1% Ameisensäure: CH₃CN + 0,08% Ameisensäure 97:3 (0 min) nach 40:60 (3,5 min) nach 2: 98(4 min) nach 2:98(5 min) nach 97:3 (5,2 min) nach 97:3 (6,5 min), Fluß: 1,4 mL/min, Temperatur: RT.
Methode E: Säule: YMC Jsphere 33*2 mm, 4 µm, H80, Gradient: H₂O + 0,05% TFA: CH₃CN + 0,05% TFA 98:2 (1 min) nach 5:95 (5,0 min) nach 5:95 (6,25 min), Fluß: 1 mL/min, Temperatur: RT.
Methode F: Säule: WatersXBridgeC₁₈, 4,6*50 mm, 2,5 µm, Gradient: H₂O + 0,1% Ameisensäure : CH₃CN + 0.1% Ameisensäure 97:3 (0 min) nach 40:60 (3,5 min) nach 2:98 (4 min) nach 2:98 (5 min) nach 97:3 (5,2 min) nach 97:3 (6,5 min), Temperatur: RT.
Methode G: Säule: WatersXBridgeC₁₈, 4,6*50 mm, 2,5 µm; Gradient: H₂O + 0,05% TFA : CH₃CN + 0,05% TFA 95:5 (0 min) nach 95:5 (0,2 min) nach 5:95 (2,4 min) nach 5:95 (3,5 min) nach 95:5 (3,6 min) nach 95:5 (4,5 min), Fluß: 1,7 ml/min, Temperatur: 50 °C.
Methode H: Säule WatersXBridgeC₁₈, 4,6*50 mm, 2,5 µm; Gradient: H₂O + 0,05% TFA : CH₃CN + 0,05% TFA 95:5 (0 min) nach 95:5 (0,2 min) nach 5:95 (2,4 min) nach 5:95 (3,2 min) nach 95:5 (3,3 min) nach 95:5 (4,0 min), Fluß: 1,7 ml/min, Temperatur: 40 °C.
Methode I: Säule Merck Chromolith FastGrad RP-18e, 50x2 mm; Gradient: H₂O + 0,05% TFA : CH₃CN + 0,05% TFA 98:2 (0,2 min) nach 2:98 (2,4 min) nach 2:98 (3,2 min) nach 98:2 (3,3 min) nach 98:2 (4 min), Fluß: 2,0 ml/min, Temperatur: RT. Methode J: Säule: YMC Jsphere 33*2 mm, 4µm, Gradient: H₂O + 0,05% TFA : CH₃CN + 0,05% TFA 98:2 (1 min) nach 5:95 (5,0 min) nach 5:95 (6,25 min), Fluß: 1ml/min, Temperatur: RT.
Methode K: Säule YMC Jsphere 33*2 mm, 4 µm, H80, Gradient: H₂O + 0,05% TFA : CH₃CN + 0,05% TFA 96:4 (0 min) nach 5:95 (2,0 min) nach 5:95 (2,4 min) nach 96:4 (2,45 min).
Methode L: Säule YMC Jsphere 33*2 mm, 4 µm, Gradient: CH₃OH + 0.05% TFA : H₂O + 0.05% TFA 2:98 (1 min) nach 95:5 (5 min) nach 95:5 (6.25 min), Fluß: 1 ml/min, Temperatur: RT.

Soweit nicht anders angegeben, wurden chromatographische Trennungen an Kieselgel mit Ethylacetat/Heptan-Gemischen als Laufmittel durchgeführt. Präparative Trennungen an Reversed Phase-(RP)-Kieselgel (HPLC) wurden, soweit nicht anders angegeben, an C18-RP-Phasen als stationärer Phase und H₂O-TFA-Acetonitrilgemischen als mobiler Phase durchgeführt.

Das Abdampfen von Lösungsmitteln geschah in der Regel unter vermindertem Druck bei 35 °C bis 45 °C am Rotationsverdampfer.

### Beispiel 1-1

### (S)-6-Amino-2-[3-((9S,12R)-9-isopropyl-11-oxo-2,7-dioxa-10-aza-bicyclo[12.2.2]octadeca-1(17),14(18),15-trien-12-yl)-ureido]-hexansäure

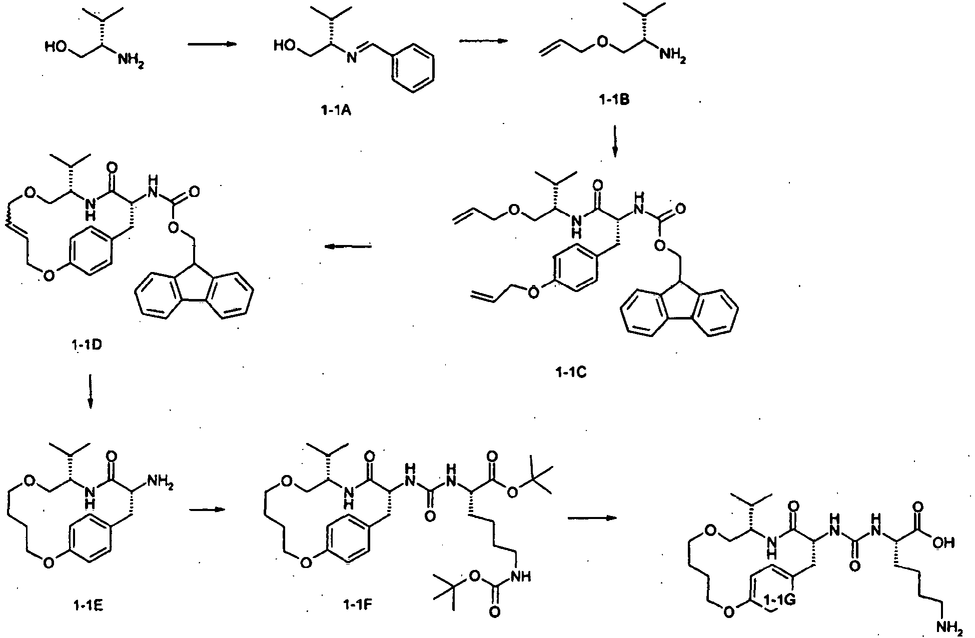

### A. (S)-3-Methyl-2-{[1-phenyl-methylidene]-amino}butan-1-ol

Zu einer Lösung von 2,57g (24,91 mmol) L-Valinol. in 28 ml Toluol wurden unter Rühren 2,64 ml (2,78 g, 26,16 mmol) Benzaldehyd gegeben und am Wasserabscheider für eine Stunde unter Rückfluß erhitzt. Nach dem Abkühlen wurde eingeengt und aus Heptan umkristallisiert. Der farblose Feststoff wurde abgesaugt und unter verminderten Druck getrocknet (3,74g).
¹H-NMR (DMSO-d6, 400 MHz) *δ* [ppm] = 8.23 (s, 1 H), 7.77 (d, 2H), 7.45-7.40 (m, 3H), 4.49 (t, 1H), 3.68-3.31 (m, 1H), 3.48-3.40 (m, 1 H), 2.96 (ddd, 1H), 1.94-1.81 (m, 1H), 0.88 (s, 5H).

### B. (S)-1-Allyloxymethyl-2-methyl-propylamin

Eine Lösung von 3,00g (15,68 mmol) (S)-3-Methyl-2-{[1-phenyl-methylidene]-amino}-butan-1-ol in 28 ml trockenem THF wurde mit 1,25g (60%ig, 31,36 mmol) Natrumhydrid versetzt und 45min bei RT gerührt. Anschließend wurden 1,43 ml (16,46 mmol) Allylbromid zugegeben und über Nacht bei RT weiter gerührt. Es wurde durch Zugabe von 20 ml Methanol gequencht und mit 1 N Salzsäure angesäuert (pH 1) und weitergerührt. Nach 3 h wurde das Reaktionsgemisch zweimal mit Dichlormethan gewaschen und die vereinigten Dichlormethanphasen mit 1 N Salzsäure extrahiert. Die vereinigten wässrigen Phasen wurden mit 1 N Natronlauge basisch gestellt (pH 14), mit Kochsalz gesättigt und dreimal mit Ethylacetat extrahiert, wobei nach jedem Extraktionsschritt der pH-Wert nachreguliert wurde. Die vereinigten organischen Phasen wurden über Natriumsulfat getrocknet, filtriert und eingeengt, sowie einmal in Dichlormethan aufgenommen und erneut eingeengt. Man erhielt 1,56g der Titelverbindung als hellgelbe Flüssigkeit.
LC/MS (Methode A): Rₜ = 0,68 min, m/z: 144,2 [MH⁺].

### C. [(R)-1-((S)-1-Allyloxymethyl-2-methyl-propylcarbamoyl)-2-(4-allyloxy-phenyl)-ethyl]-carbaminsäure-9H-fluoren-9-ylmethylester

25,74g (58,04 mmol) (R)-3-(4-Allyloxy-phenyl)-2-(9H-fluoren-9-ylmethoxycarbonyl-amino)-propionsäure wurden in 400 ml THF vorgelegt, sukzessive mit 8,88 g (58,04 mmol) N-Hydroxybenzotriazol sowie 11,98g (58,04 mmol) N,N'-Dicyclohexyl-carbodiimid versetzt und bei RT gerührt. Nach Stehenlassen über Nacht wurde abfiltriert und eingeengt. Der Rückstand wurde in Ethylacetat aufgenommen und sukzessive mit gesättigter Natriumhydrogencarbonat-Lösung und verdünnter Salzsäure gewaschen. Die organische Phase wurde über Natriumsulfat getrocket, eingeengt und der Rückstand durch Chromatographie an Kieselgel getrennt. Man erhielt 13,55g der gewünschten Verbindung.
LC/MS (Methode A): Rₜ = 1,95 min, m/z: 569,3 [MH⁺].

### D. ((9S,12R)-9-Isopropyl-11-oxo-2,7-dioxa-10-aza-bicyclo[12.2.2]octadeca-1(17),4,14(18),15-tetraen-12-yl)-carbaminsäure-9H-fluoren-9-ylmethylester

Eine Lösung von 1,25g (2,20 mmol) [(R)-1-((S)-1-Allyloxymethyl-2-methyl-propylcarbamoyl)-2-(4-allyloxy-phenyl)-ethyl]-carbaminsäure-9H-fluoren-9-ylmethylester und 0,40g (0,659 mmol) Dichloro(o-isopropoxyphenylmethylen)(tri-cyclohexylphosphin)-ruthenium (Hoyveda-Grubbs-Katalysator) in 610 ml Dichlormethan wurde für 24 h bei 40° C gerührt. Anschließend wurde das Reaktionsgemisch eingeengt und der Rückstand durch Chromatographie an Kieselgel gereinigt. Man erhielt 1,03g der gewünschten Verbindung als farblosen Feststoff. LC/MS (Methode A): Rₜ = 1,75 min, m/z: 541,3 [MH⁺].

### E. (9S,12R)-12-Amino-9-isopropyl-2,7-dioxa-10-aza-bicyclo[12.2.2]octadeca-1(17),14(18),15-trien-11-on

Eine Mischung aus 6,82g (12,62 mmol) ((9S,12R)-9-isopropyl-11-oxo-2,7-dioxa-10-aza-bicyclo[12.2.2]octadeca-1(17),4,14(18),15-tetraen-12-yl)-carbaminsäure-9H-fluoren-9-ylmethylester und 4,4g Pd/C 10 % in 880 ml Methanol wurde unter einer Wasserstoffatmosphäre bei RT gerührt. Nach 6 h wurde abfiltriert und vom Lösungsmittel befreit. Der Rückstand wurde durch präparative HPLC getrennt. Die Wertfraktionen wurden vereinigt, Acetonitril abgedampft und die erhaltene wässrige Lösung mit Natriumhydrogencarbonat leicht alkalisch gestellt. Die wässrige Phase wurde mehrfach mit Ethylacetat extrahiert, die vereinigten organischen Phasen über Natriumsulfat getrocknet, filtriert und eingeengt. Chromatographie an Kieselgel mit Ethylacetat/Methanol-Gemischen lieferte die Titelverbindung (2,90g).
LC/MS (Methode A): Rₜ = 1,02 min, m/z: 321,2 [MH⁺].

### F. (S)-6-tert-Butoxycarbonylamino-2-[3-((9S,12R)-9-isopropyl-11-oxo-2,7-dioxa-10-aza-bicyclo[12.2.2]octadeca-1(17),14(18),15-trien-12-yl)-ureido]-hexansäure-tert-butylester

Eine Lösung aus 1,59 g (4,68 mmol) (S)-2-Amino-6-tert-butoxycarbonylamino-hexansäure-tert-butylester Hydrochlorid, 1,43 ml (1,04g , 10,30 mmol) Triethylamin und 0,76g (4,68 mmol) Carbonyldiimidazol in 25 ml Dimethylformamid (DMF) wurde 1 h bei RT gerührt und dann mit einer Lösung von 1,5 g in 20 ml DMF versetzt. Es wurde bei RT nachgerührt und über Nacht stehen gelassen. Nach Abdampfen des Lösungsmittels wurde zwischen Ethylacetat und Wasser verteilt, die organische Phase über Natriumsulfat getrocknet, filtriert und eingeengt. Der Rückstand wurde über präparative HPLC getrennt, die Wertfraktionen vereinigt und vom Acetonitril befreit. Es wurde mit Natriumhydrogencarbonat leicht alkalisch gestellt und mit Ethylacetat extrahiert. Die vereinigten organischen Phasen wurden über Natriumsulfat getrocknet, filtriert und eingeengt. Es wurden 0,73g der Titelverbindung erhalten.
LC/MS (Methode A): Rₜ = 1,68 min, m/z: 649,4 [MH⁺].

### G. (S)-6-Amino-2-[3-((9S,12R)-9-isopropyl-11-oxo-2,7-dioxa-10-aza-bicyclo[12.2.2]octadeca-1(17),14(18),15-trien-12-yl)-ureido]-hexansäure

0,71g (1,10 mmol) (S)-6-tert-Butoxycarbonylamino-2-[3-((9S,12R)-9-isopropyl-11-oxo-2,7-dioxa-10-aza-bicyclo[12.2.2]octadeca-1(17),14(18),15-trien-12-yl)-ureido]-hexansäure-tert-butylester wurden in 11 ml Dichlormethan gelöst und mit dem gleichen Volumen Trifluoressigsäure versetzt. Nach 4 h Rühren bei RT wurde eingeengt und der Rückstand durch präparative HPLC fraktioniert. Die Wertfraktionen wurden vereinigt und nach Abdampfen von Acetonitril mit verdünnter Salzsäure versetzt, weiter eingeengt und schließlich gefriergetrocknet. Es wurden 0,42g der Titelverbindung als Hydrochlorid erhalten.
¹H-NMR (DMSO-d6, 400 MHz) *δ* [ppm] = 7,79 (3H, s, br), 7,22 (1H, d), 6,99-6,90 (m, 3H), 6,87 (d, 1H), 6,29 (d, 1 H), 5,79 (d, 1 H), 4,32-4,17 (m, 3H), 4,10 (dd, 1 H), 3,29-3,18 (m, 3H), 3,12 (dd, 1H), 2,96-2,87 (m, 2H), 2,83-2,72 (m, 2H), 2,65 (dd, 1 H), 1,78-1,2 (m, 11 H), 0,72 (d, 3H), 0,67 (d, 2H);
LC/MS (Methode A): Rₜ = 0,94 min, m/z: 493,4 [MH⁺].

Die folgenden Beispiele wurden in analoger Weise erhalten, indem die entsprechenden Aminoalkohole anstelle von Valinol eingesetzt wurden:

| Beispiel | Name | Rₜ (min) | m/z [MH⁺] | Methode |
|---|---|---|---|---|
| 1-2 | (S)-6-Amino-2-[3-((R)-11-oxo-2,7-dioxa-10-azabicyclo[12.2.2]octadeca-1(17),14(18),15-trien12-yl)-ureido]-hexansäure | 0,77 | 451,4 | A |
| 1-3 | (S)-6-Amino-2-[3-((R)-9,9-dimethyl-11-oxo-2,7-dioxa-10-aza-bicyclo[12.2.2]octadeca-1(17),14(18),15-trien-12-yl)-ureido]-hexansäure | 0,93 | 479,2 | A |
| 1-4 | (S)-6-Amino-2-[3-((9S,12R)-11-oxo-9-phenyl-2,7-dioxa-10-aza-bicyclo[12.2.2]octadeca-1(17),14(18),15-trien-12-yl)-ureido]-hexansäure | 0,96 | 527,5 | A |
| 1-5 | (S)-6-Amino-2-[3-((9S,12R)-9-benzyl-11-oxo-2,7-dioxa-10-aza-bicyclo[12.2.2]octadeca-1(17),14(18),15-trien-12-yl)-ureido]-hexansäure | 1,03 | 541,3 | A |
| 1-6 | (S)-6-Amino-2-[3-((9S,12R)-9-cyclohexyl-11-oxo-2,7-dioxa-10-aza-bicyclo[12.2.2]octadeca-1(17),14(18),15-trien-12-yl)-ureido]-hexansäure | 1,02 | 533,3 | A |
| 1-7 | (S)-6-Amino-2-[3-((9S,12R)-9-ethyl-11-oxo-2,7-dioxa-10-aza-bicyclo[12.2.2]octadeca-1(17),14(18),15-trien-12-yl)-ureido]-hexansäure | 2,26 | 479,26 | C |
| 1-8 | (S)-6-Amino-2-[3-((9S,12R)-9-methyl-11-oxo-2,7-dioxa-10-aza-bicyclo[12.2.2]octadeca-1(17),14(18),15-trien-12-yl)-ureido]-hexansäure | 2,21 | 465,25 | C |
| 1-9 | (S)-6-Amino-2-[3-((9S,12R)-9-tert-butyl-11-oxo-2,7-dioxa-10-aza-bicyclo[12.2.2]octadeca-1(17),14(18),15-trien-12-yl)-ureido]-hexansäure | 2,39 | 507,33 | E |
| 1-10 | (S)-6-Amino-2-{3-[(9S,12R)-9-((S)-sec-butyl)-11-oxo-2,7-dioxa-10-aza-bicyclo[12.2.2]octadeca-1(17),14(18),15-trien-12-yl]-ureido}-hexansäure | 2,41 | 507,24 | C |

### Beispiel 2-1

### (S)-6-Amino-2-[3-((13S,16R)-13-isopropyl-15-oxo-2,11-dioxa-14-aza-bicyclo[16.2.2]docosa-1(21),18(22),19-trien-16-yl)-ureido]-hexansäure

### A. (R)-1-(2-Hept-6-enyloxy-ethyl)-2-methyl-propylamin

Eine Lösung von 3,00g (15,68 mmol) (S)-3-Methyl-2-{[1-phenyl-methyliden]-amino}-butan-1-ol (1-1A) in 28 ml trockenem THF wurde unter Argon vorgelegt, mit 1,50g (60%ig, 37,51 mmol) Natriumhydrid versetzt und 45 min rühren gelassen. Nach der Zugabe von 2,83g (15,68 mmol) 7-Brom-hept-1-en wurde über Nacht weitergerührt, mit 20 ml Methanol vorsichtig gequencht, anschließend mit 300 ml 1N Salzsäure versetzt (pH =1) und bei 40°C 2 h gerührt. Es wurde mit Dichlormethan gewaschen, die wässrige Phase mit 1 N Natronlauge auf pH 14 gestellt und dreimal mit Ethylacetat extrahiert. Die vereinigten organischen Phasen wurden über Natriumsulfat getrocknet, filtriert und eingeengt. Man erhielt 0,91g des Rohprodukts. Das Rohprodukt wurde ohne weitere Reinigung weiter umgesetzt.
LC/MS (Methode B): Rₜ = 0,72min, m/z: 200,2 [MH⁺].

### B. [(R)-2-(4-Allyloxy-phenyl)-1-((S)-1-hept-6-enyloxymethyl-2-methyl-propylcarbamoyl)-ethyl]-carbaminsäure-9H-fluoren-9-yl-methylester

Eine Lösung von 2,02g (4,57 mmol) N-α-(9-Fluorenylmethyloxycarbonyl)-O-allyl-D-Tyrosin in 46 ml DMF wurde mit 0,77g (5,02 mmol) 1-Hydroxybenzotriazol und 1,04g (5,02 mmol) N,N'-Dicyclohexylcarbodiimid versetzt und 2 h bei RT gerührt. Anschließend wurden 0,91g (4,57 mmol) (R)-1-(2-Hept-6-enyloxy-ethyl)-2-methyl-propylamin zugegeben, bei RT weitergerührt und über Nacht stehen gelassen. Danach wurde vom Niederschlag abfiltriert, eingeengt, der Rückstand in Ethylacetat aufgenommen, sukzessive mit gesättigte Natriumhydrogencarbonat-Lösung sowie verdünnter Salzsäure gewaschen, die organische Phase über Natriumsulfat getrocknet, filtriert und eingeengt. Der Rückstand wurde über präparative HPLC getrennt, die Wertfraktionen vereinigt, vom Acetonitril befreit und mit Ethylacetat extrahiert. Die organische Phase wurde über Natriumsulfat getrocknet, filtriert und eingeengt. Es wurden 1,02g der Titelverbindung erhalten (Methode D): Rₜ = 5,49 min, m/z: 669 [M-H+HCOOH⁻].

### C. ((13S,16R)-13-Isopropyl-15-oxo-2,11-dioxa-14-aza-bicyclo[16.2.2]docosa-1(21),4,18(22),19-tetraen-16-yl)-carbaminsäure-9H-fluoren-9-yl-methylester

Eine Lösung von 1,01g (1,61 mmol) [(R)-2-(4-Allyloxy-phenyl)-1-((S)-1-hept-6-enyloxymethyl-2-methyl-propylcarbamoyl)-ethyl]-carbaminsäure-9H-fluoren-9-yl-methylester und 0,15g (0,24 mmol) Dichloro(o-isopropoxyphenylmethylen)(tri-cyclohexylphosphin)-ruthenium (Hoyveda-Grubbs-Katalysator) in 460 ml Dichlormethan wurde für 24 h bei 40° C gerührt. Anschließend wurde das Reaktionsgemisch eingeengt und der Rückstand durch Chromatographie an Kieselgel gereinigt. Man erhielt 0,87g der gewünschten Verbindung als farblosen Feststoff. LC/MS (Methode C): Rₜ = 4,30 min, m/z: 597,39 [MH⁺].

### D. (13S,16R)-16-Amino-13-isopropyl-2,11-dioxa-14-aza-bicyclo[16.2.2]docosa-1(21),18(22),19-trien-15-on

Eine Mischung aus 0,87 (1,46 mmol) ((13S,16R)-13-Isopropyl-15-oxo-2,11-dioxa-14-aza-bicyclo[16.2.2]docosa-1(21),4,18(22),19-tetraen-16-yl)-carbaminsäure-9H-fluoren-9-yl-methylester und 0,16g Pd/C 10% in 100 ml Methanol wurde unter einer Wasserstoffatmosphäre bei RT gerührt. Nach Rühren über Nacht wurde nach Zugabe von 0,5 ml Piperidin noch 2 h weitergerührt, abfiltriert und vom Lösungsmittel befreit. Der Rückstand wurde durch präparative HPLC getrennt. Die Wertfraktionen wurden vereinigt und gefriergetrocknet. Man erhielt 0,18 g der Titelverbindung als Trifluoracetat.
LC/MS (Methode C): Rₜ = 3,07 min, m/z: 377,22 [MH⁺].

### E. (S)-6-Amino-2-[3-((13S,16R)-13-isopropyl-15-oxo-2,11-dioxa-14-aza-bicyclo[16.2.2]docosa-1(21),18(22),19-trien-16-yl)-ureido]-hexansäure

Eine Lösung von 0,18g (0,37 mmol) (13S,16R)-16-Amino-13-isopropyl-2,11-dioxa-14-aza-bicyclo[16.2.2]docosa-1(21),18(22),19-trien-15-on in 3,8 ml DMF wurde zu 60 mg (0,37 mmol) vorgelegtem 1,1'-Carbonyldiimidazol gegeben, mit 0,2 ml (0,15 g, 1,47 mmol) Triethylamin versetzt und unter Argon gerührt. Nach 10 min wurden 124 mg (0,37 mmol) (S)-2-Amino-6-tert-butoxycarbonylamino-hexansäure-tert-butylester Hydrochlorid zugegeben und die Mischung 3 h gerührt. Es wurde eingeengt, zwischen Wasser und Ethylacetat verteilt, die organische Phase über Natriumsulfat getrocknet, filtriert und eingeengt. Der Rückstand wurde über präparative HPLC gereinigt. Die Wertfraktionen wurden vereinigt und eingeengt. Der Rückstand wurde in 20 ml Dichlormethan/TFA (1:1, v/v) aufgenommen und 2 h stehen gelassen. Es wurde eingeengt, in 1 N Salzsäure mit etwas Acetonitril gelöst und gefriergetrocknet. Man erhielt einen amorphen Feststoff (0,12 g) als Hydrochlorid. LC/MS (Methode C): Rₜ = 2,88 min, m/z: 549,21 [MH⁺].

### Beispiel 3-1 (S)-6-Amino-2-[3-((E)-(9S,12R)-9-isopropyl-11-oxo-2,7-dioxa-10-aza-bicyclo[12.2.2]octadeca-1(17),4,14(18),15-tetraen-12-yl)-ureido]-hexansäure

Die Titelverbindung wurde analog zu Beispiel 1-1 unter Auslassung des Hydrierschrittes und ohne abschließende Gefriertrocknung mit Salzsäure direkt als Trifluoracetat erhalten. LC/MS (Methode A): Rₜ = 0,89 min, m/z: 491,2 [MH⁺].

### Beispiel 4-1 (S)-6-Amino-2-[3-((3S,6R)-3-isopropyl-5-oxo-1-oxa-4-aza-cyclotetradec-6-yl)-ureido]-hexansäure

Die Titelverbindung wurde analog zu Beispiel 2-1 unter Verwendung von Fmoc-D-Allylgylcin anstelle von Fmoc-D-O-Allyltyrosin erhalten.
LC/MS (Methode C): Rₜ = 2,35 min, m/z: 443,34 [MH⁺].

In gleicher Weise unter Verwendung anderer Aminoalkohole und gegebenenfalls der Verwendung oder Einführung einer Esterschutzgruppe wurden erhalten:

| Beispiel | Name | Rₜ (min) | m/z [MH⁺] | Methode |
|---|---|---|---|---|
| 4-2 | (S)-3-(6-Amino-pyridin-3-yl)-2-[3-((3S,16R)-3-isopropyl-5-oxo-1-oxa-4-aza-cyclotetradec-6-yl)-ureido]-propionsäure | 2,52 | 478,27 | H |
| 4-3 | (S)-3-(6-Amino-pyridin-3-yl)-2-[3-((3S,6R)-3-methyl-5-oxo-1-oxa-4-aza-cyclotetradec-6-yl)-ureido]-propionsäure | 1,66 | 450,26 | G |
| 4-4 | (S)-3-(6-Amino-pyridin-3-yl)-2-[3-((3S,8R)-3-isopropyl-5-oxo-1-oxa-4-aza-cyclotetradec-6-yl)-ureido]-propionsäureethylester | 0,66 | 506,4 | B |
| 4-5 | (S)-3-(6-Amino-pyridin-3-yl)-2-[3-((3S,6R)-3-methyl-5-oxo-1-oxa-4-aza-cyclotetradec-6-yl)-ureido]-propionsäureethylester | 1,81 | 478,37 | G |

### Beispiel 5-1

### (R)-3-(2-Amino-ethansulfonyl)-2-[3-((9S,12R)-9-isopropyl-11-oxo-2,7-dioxa-10-aza-bicyclo[12.2.2]octadeca-1(17),14(18),15-trien-12-yl)-ureido]-propionsäure

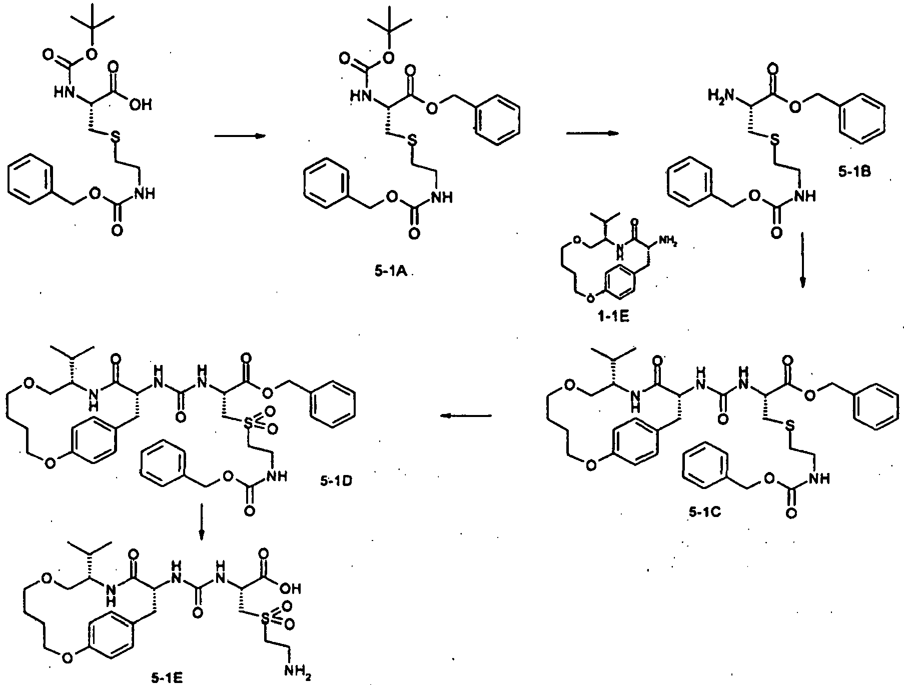

### A. (R)-3-(2-Benzyloxycarbonylamino-ethylsulfanyl)-2-tert-butoxycarbonylamino-propionsäure-benzylester

Eine Lösung von 2,30g (5,77 mmol) (R)-3-(2-Benzyloxycarbonylamino-ethyl-sulfanyl)-2-tert-butoxycarbonylamino-propionsäure, 0,60 ml (0,62g, 5,77 mmol) Benzylalkohol, 0,07g (0,58 mmol) 4-Dimethylaminopyridin und 1,33g (6,93 mmol) N-(3-Dimethylaminopropyl)-N'-ethylcarbodiimid-Hydrochlorid in 30 ml Dichlormethan wurde bei RT über Nacht gerührt. Das Reaktionsgemisch wurde sukzessive mit 1 N Salzsäure, 1 N Natriumhydrogencarbonat-Lösung sowie mit Wasser gewaschen. Nach Trocknen über Natriumsulfat, Filtrieren und Einengen wurde der Rückstand an Kieselgel chromatographiert. Man erhielt 2,09 g der Titelverbindung als farbloses Öl.
LC/MS (Methode C): Rₜ = 3,63 min, m/z: 389,17 [M-Boc+H⁺].

### B. (R)-2-Amino-3-(2-benzyloxycarbonylamino-ethylsulfanyl)-propionsäure-benzylester

Eine Lösung von 0,50 g (1,023 mmol) (R)-3-(2-Benzyloxycarbonylamino-ethyl-sulfanyl)-2-tert-butoxycarbonylamino-propionsäure-benzylester in 5 ml Dichlormethan wurde mit dem gleichen Volumen Trifluoressigsäure versetzt und 1 h bei RT stehengelassen. Anschließend wurde eingeengt und direkt weiter umgesetzt. LC/MS (Methode A): Rₜ = 1,06 min, m/z: 389,1 [MH⁺].

### C. (R)-3-(2-Benzyloxycarbonylamino-ethylsulfanyl)-2-[3-((9S,12R)-9-isopropyl-11-oxo-2,7-dioxa-10-aza-bicyclo[12.2.2]octadeca-1(17),14(18),15-trien-12-yl)-ureido]-propionsäure-benzylester

Eine Lösung von 166 mg (1,02 mmol) 1,1'-Carbonyldiimidazol in 8 ml DMF wurde mit 515 mg (1,02 mmol) des Trifluoracetats aus Schritt B sowie 0,57 ml (415 mg, 4,10 mmol) Triethylamin versetzt. Zu diesem Gemisch wurde unter Argon eine Lösung von 328 mg (1,02 mmol) (9S,12R)-12-Amino-9-isopropyl-2,7-dioxa-10-aza-bicyclo[12.2.2]octadeca-1(17),14(18),15-trien-11-on (1-1E) in 8 ml DMF gegeben und über Nacht bei RT gerührt. Anschließend wurde das Reaktionsgemisch eingeengt und durch präparative HPLC getrennt. Die Wertfraktionen wurden vereinigt und vom Acetonitril befreit. Der erhaltene wäßrige Lösung wurde mit Ethylacetat extrahiert, die organische Phase über Natriumsulfat getrocknet, filtriert und eingeengt. Es wurden 222 mg der Titelverbindung erhalten.
LC/MS (Methode A): Rₜ = 1,72 min, m/z: 735,3 [MH⁺].

### D. (R)-3-(2-Benzyloxycarbonylamino-ethanesulfonyl)-2-[3-(9S,1 2R)-9-isopropyl-11-oxo-2,7-dioxa-10-aza-bicyclo[12.2.2]octadeca-1(17),14(18),15-trien-12-yl)-ureido]-propionsäurebenzylester

Eine Mischung von 222 mg (0,30 mmol) (R)-3-(2-Benzyloxycarbonylamino-ethyl-sulfanyl)-2-[3-((9S,12R)-9-isopropyl-11-oxo-2,7-dioxa-10-aza-bicyclo[12.2.2] octadeca-1(17),14(18),15-trien-12-yl)-ureido]-propionsäure-benzylester in 5 ml Methanol und 5 ml Wasser wurde auf 0 C gekühlt und portionsweise mit 742 mg (1,21 mmol) Oxon versetzt. Nach 2 h wird mit Ethylacetat verdünnt, die organische Phase abgetrennt und noch zweimal mit Ethylacetat extrahiert. Die vereinigten organischen Phasen wurden über Natriumsulfat getrocknet, filtriert und eingeengt. Man erhielt 198 mg, die ohne weitere Reinigung weiter umgesetzt wurden.
LC/MS (Methode A): Rₜ = 1,54 min, m/z: 767,3 [MH⁺].

### E. (R)-3-(2-Amino-ethansulfonyl)-2-[3-((9S,12R)-9-isopropyl-11-oxo-2,7-dioxa-10-aza-bicyclo[12.2.2]octadeca-1(17),14(18),15-trien-12-yl)-ureido]-propionsäure

Zu einer Lösung von 198 mg (0,26 mmol) (R)-3-(2-Benzyloxycarbonylamino-ethanesulfonyl)-2-[3-((9S,12R)-9-isopropyl-11-oxo-2,7-dioxa-10-aza-bicyclo[12.2.2] octadeca-1(17),14(18),15-trien-12-yl)-ureido]-propionsäurebenzylester in 10ml Methanol wurden 83 mg Palladium/C 5% gegeben und unter einer Wasserstoffatmosphäre (Ballondruck) hydriert. Nach Stehenlassen über 2 Tage wurde abfiltriert, eingeengt und durch präparative HPLC gereinigt. Die Wertfraktionen wurden vereinigt und nach Zugabe von 1 N Salzsäure gefriergetrocknet. Man erhielt 23 mg der Titelverbindung als Hydrochlorid.
LC/MS (Methode C): Rₜ = 2,35 min, m/z: 543,26 [MH⁺].

### Beispiel 5-2 (S)-3-(6-Amino-pyridin-3-yl)-2-[3-((9S,12R)-9-isopropyl-11-oxo-2,7-dioxa-10-aza-bicyclo[12.2.2]octadeca-1(17),14(18),15-trien-12-yl)-ureido]-propionsäure

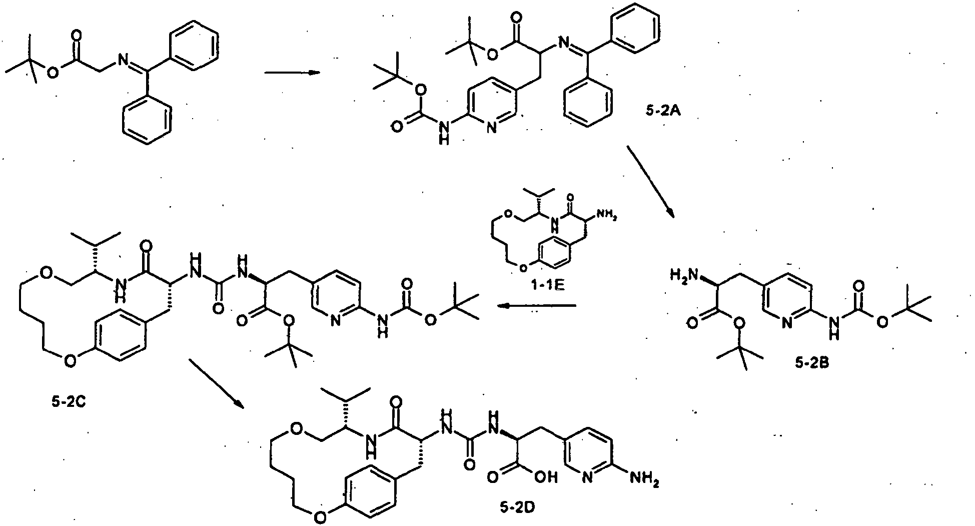

### A. 2-(Benzhydryliden-amino)-3-(6-tert-butoxycarbonylamino-pyridin-3-yl)-propionsäure-tert-butylester

Zu einer Lösung von 25,00 g (84,64 mmol) N-(Diphenylmethylen)-glycin-tert-butylester in 185 ml THF wurden unter Argon bei 0 C 84,64 ml (84,64 mmol) einer 1 M Lösung von Lithium-bis(trimethylsilyl)amid in THF zugetropft. Nach 15 min Rühren bei dieser Temperatur wurden 24,31g (84,64 mmol) (5-Brommethyl-pyridin-2-yl)-carbaminsäure-tert-butylester als Feststoff zugegeben und für 1 h weitergerührt. Das Reaktionsgemisch wurde vorsichtig mit Wasser verdünnt und mit Ethylacetat extrahiert. Die organische Phase wurde über Natriumsulfat getrocknet, filtriert und eingeengt. Es wurden 44,08 g des Rohprodukts erhalten, welches ohne weitere Reinigung weiter umgesetzt wurde.
LC/MS (Methode A): Rₜ = 1,68 min, m/z: 502,2 [MH⁺].

### B. (S)-2-Amino-3-(6-tert-butoxycarbonylamino-pyridin-3-yl)-propionsäure-tert-butylester

Eine Lösung von 19,50 g (38,87 mmol) des Rohprodukts aus Schritt A in 1,8 l Methanol wurde nach Zugabe von 4,14 g Palladium/Kohle 10% unter Eigendruck hydriert. Nach vollständiger Umsetzung wurde abfiltriert und eingeengt. Das Rohgemisch wurde in Heptan aufgenommen, mit 45 ml 1 N Salzsäure und 90 ml Wasser gemischt, die wässrige Phase in 90 ml 1 N Natronlauge gegeben und viermal mit Ethylacetat extrahiert. Die vereinigten organischen Phasen wurden über Natriumsulfat getrocknet, filtriert und eingeengt. Das Racemat wurde durch chirale Chromatographie getrennt.

LC/MS (Methode A): Rₜ = 0,89 min, m/z: 338,1 [MH⁺]. Chirale Chromatographie (Chiralpak AD-H/44, 250x4,6mm, Ethanol:Methanol 1:1 + 0,1 % Diethylamin, 40min): Rₜ = 17,48 min.

### C. (S)-3-(6-tert-Butoxycarbonylamino-pyridin-3-yl)-2-[3-((9S,12R)-9-isopropyl-11-oxo-2,7-dioxa-10-aza-bicyclo[12.2.2]octadeca-1(17),14(18),15-trien-12-yl)-ureido]-propionsäure-tert-butylester

Zu einer Mischung von 234 mg (0,69 mmol) (S)-2-Amino-3-(6-tert-butoxycarbonyl-amino-pyridin-3-yl)-propionsäure-tert-butylester und 106 µl (77 mg, 0,76 mmol) Triethylamin in 2,5 ml DMF wurden 112 mg (0,69 mmol) 1,1'-Carbonyldiimidazol gegeben. Die Lösung wurde 1 h bei RT gerührt, bevor eine Lösung von 222 mg (0,69 mmol) (9S,12R)-12-Amino-9-isopropyl-2,7-dioxa-10-aza-bicyclo[12.2.2] octadeca-1(17),14(18),15-trien-11-on (1-1E) in 2,5 ml DMF hinzugegeben wurde. Nach Stehenlassen über Nacht wurde das Gemisch eingeengt und zwischen Wasser und Ethylacetat verteilt. Die organische Phase wurde abgetrennt, über Natriumsulfat getrocknet, filtriert und eingeengt. Der Rückstand wurde durch präparative HPLC gereinigt. Die Wertfraktionen wurden vereinigt, vom Acetonitril befreit, mit Natriumhydrogencarbonat leicht alkalisch gestellt und mit Ethylacetat extrahiert. Die organische Phase wurde über Natriumsulfat getrocknet, filtriert und eingeengt. LC/MS (Methode A): Rₜ = 1,47 min, m/z: 684,3 [MH⁺].

### D. (S)-3-(6-Amino-pyridin-3-yl)-2-[3-((9S,12R)-9-isopropyl-11-oxo-2,7-dioxa-10-aza-bicyclo[12.2.2]octadeca-1(17),14(18),15-trien-12-yl)-ureido]-propionsäure

160 mg (0,23 mmol) (S)-3-(6-tert-Butoxycarbonylamino-pyridin-3-yl)-2-[3-((9S,12R)-9-isopropyl-11-oxo-2,7-dioxa-10-aza-bicyclo[12.2.2]octadeca-1(17),14(18),15-trien-12-yl)-ureido]-propionsäure-tert-butylester wurden in 6 ml Dichlormethan gelöst und mit dem gleichen Volumen TFA versetzt und bei RT gerührt. Nach 5 h wurde eingeengt und über präparative HPLC gereingt. Die Wertfraktionen wurden vereinigt, vom Acetonitril befreit, mit 1 N Salzsäure versetzt, weiter eingeengt und schließlich gefriergetrocknet. Man erhielt 108 mg der Titelverbindung als Hydrochlorid.
LC/MS (Methode A): Rₜ = 0,92 min, m/z: 528,3 [MH⁺].

### Beispiel 5-3

### (S)-3-(6-Amino-pyridin-3-yl)-2-[3-((9S,12R)-9-cyclopropyl-11-oxo-2,7-dioxa-10-aza-bicyclo[12.2.2]octadeca-1(17),14(18),15-trien-12-yl)-ureido]-propionsäure

Die Titelverbindung wurde analog zu Beispiel 5-2 unter Verwendung von (9S,12R)-12-Amino-9-cyclopropyl-2,7-dioxa-10-aza-bicyclo[12.2.2]octadeca-1(17),14(18),15-trien-11-on aus (S)-2-Amino-2-cyclopropyl-ethanol (US 6,191,306) anstelle von 1-1E hergestellt.
LC/MS (Methode J): Rₜ = 2,39 min, m/z: 526,41 [MH⁺].

### Beispiel 5-4

### (S)-6-Amino-2-[3-((9S,12R)-9-isopropyl-11-oxo-2,7-dioxa-10-aza-bicyclo[12.2.2]octadeca-1(17),14(18),15-trien-12-yl)-ureido]-6-methyl-heptansäure

Die Titelverbindung wird analog zu Beispiel 5-2 unter Verwendung von (S)-2,6-Diamino-6-methyl-heptansäureethylester-Hydrochlorid unter Zusatz der Hilfbase Triethylamin erhalten.
LC/MS (Methode C): Rₜ = 2,34 min, m/z: 521,26 [MH⁺].

### Beispiel 5-5

### (S)-3-(6-Amino-pyridin-3-yl)-2-[3-((9S,12R)-9-isopropyl-11-oxo-2,7-dioxa-10-aza-bicyclo[12.2.2]octadeca-1-(17),14(18),15-trien-12-yl)-ureido]-propionsäureethylester

Die Titelverbindung wird analog zu Beispiel 5-2 unter Verwendung von N-(Diphenylmethylen)-glycin-ethylester anstelle von N-(Diphenylmethylen)-glycin-tert-butylester erhalten.
LC/MS (Methode C): Rₜ = 2,53 min, m/z: 556,2 [MH⁺].

In analoger Weise wurde die folgende Verbindung hergestellt:

### Beispiel 5-6

### (S)-2-[3-((9S,12R)-9-Isopropyl-11-oxo-2,7-dioxa-10-aza-bicyclo[12.2.2]octadeca-1(17),14(18),15-trien-12-yl)-ureido]-3-piperidin-3-yl-propionsäure

LC/MS (Methode I): Rₜ = 1,20 min, m/z: 519,38 [MH⁺].

### Beispiel 5-7

### (S)-3-(6-Amino-pyridin-3-yl)-2-[3-((9S,12R)-9-methyl-11-oxo-2,7-dioxa-10-aza-bicyclo[12.2.2]octadeca-1(17),14(18),15-trien-12-yl)-ureido]-propionsäure

LC/MS (Methode B): Rₜ = 0,54 min, m/z: 500,3 [MH⁺].

### Beispiel 6-1

### (S)-3-(6-Amino-pyridin-3-yl)-2-[3-((8S,11R)-8-isopropyl-10-oxo-6-oxa-1,9,14-triaza-bicyclo[11.2.1]hexadeca-13(16),14-dien-11-yl)-ureido]-propionsäure

### A. (R)-3-(1-Allyl-1H-imidazol-4-yl)-2-benzyloxycarbonylamino-propionsäure

Eine Lösung von 4,85g (24,84 mmol) (R)-3-(1-Allyl-1 H-imidazol-4-yl)-2-amino-propionsäure (wie für (S)-Enantiomer in Bioorg. Med. Chem. 2006, 14, 5981-5988 beschrieben) in 13ml 2 N Natronlauge wurde unter Rühren bei 0 C mit 3,53 ml (4,24 g, 24,84 mmol) Benzylchloroformiat versetzt und unter Zusatz von weiteren 13 ml 2 N Natronlauge bei dieser Temperatur für 2 h weitergerührt und dann auf RT erwärmt. Das Reaktionsgemisch wurde mit Ethylacetat überschichtet und der pH mit 6 N Salzsäure auf 3 bis 4 eingestellt. Die organische Phase wurde abgetrennt und die wässrige Phase nochmals mit Ethylacetat gewaschen und anschließend gefriergetrocknet. Der Rückstand wurde bis zur Rührbarkeit mit THF und DMF versetzt, ausgerührt und die Suspension filtriert. Die erhaltene Lösung wurde eingeengt und ohne weitere Reinigung im nächsten Schritt umgesetzt.
LC/MS (Methode B): Rₜ = 0,52 min, m/z: 330,2 [MH⁺].

### B. [(R)-2-(1-Allyl-1 H-imidazol-4-yl)-1-((S)-1-allyloxymethyl-2-methyl-propylcarbamoyl)-ethyl]-carbaminsäurebenzylester

Eine Lösung des Materials aus Schritt A in 180 ml DMF wurde mit 4,18g (27,32 mmol) N-Hydroxybenzotriazol sowie 5,64 g (27,32 mmol) N,N'-Dicyclohexylcarbodiimid versetzt und für 2 h bei RT gerührt. Nach Zugabe von 3,56 g (24,84 mmol) (S)-1-Allyloxymethyl-2-methyl-propylamin (1-1 B) wurde für weitere 24 h bei RT gerührt, anschließend eingeengt und der Rückstand zwischen Ethylacetat und gesättigter NaHCO₃-Lösung verteilt. Die organische Phase wurde über Na₂SO₄ getrocknet, filtriert und eingeengt. Der Rückstand wurde durch präparative HPLC gereinigt. Nach Waschen mit gesättigter NaHCO₃-Lösung erhält man 5,02g der Titelverbindung.
LC/MS (Methode B): Rₜ = 0,75 min, m/z: 454,9 [MH⁺].

### C. ((8S,11R)-8-Isopropyl-10-oxo-6-oxa-1,9,14-triaza-bicyclo[11.2.1]hexadeca-3,13(16),14-trien-11-yl)-carbaminsäurebenzylester

Eine Lösung von 0,735g (1,62 mmol) [(R)-2-(1-Allyl-1H-imidazol-4-yl)-1-((S)-1-allyloxymethyl-2-methyl-propylcarbamoyl)-ethyl]-carbaminsäurebenzylester in 370 ml Dichlormethan wurde mit 0,210 g (0,24 mmol) Benzyliden[1,3-bis(2,4,6-trimethylphenyl)-2-imidazolidinyliden]dichloro(tricyclohexylphosphin)ruthenium (Grubbs II-Katalysator) versetzt und bei RT gerührt. Die Mischung wurde für mehrere Tage unter wiederholte Zugabe von Grubbs II-Katalysator bei RT gerührt, bis kein Ausgangsmaterial mehr vorhanden war (LC/MS). Die Mischung wurde eingeengt und der Rückstand an Kieselgel chromatographiert (Dichlormethan/ Methanol 99:1 → 9:1). Es wurden 0,506 g der Titelverbindung erhalten.
LC/MS (Methode B): Rₜ = 0,64 min, m/z: 427,3 [MH⁺].

### D. (8S,11R)-11-Amino-8-isopropyl-6-oxa-1,9,14-triaza-bicyclo[11.2.1]hexadeca-13(16),14-dien-10-on.

Eine Lösung von 0,49 g (1,14 mmol) ((8S,11R)-8-Isopropyl-10-oxo-6-oxa-1,9,14-triaza-bicyclo[11.2.1]hexadeca-3,13(16),14-trien-11-yl)-carbaminsäurebenzylester aus Schritt C in 80 ml Methanol wurde unter einer Wasserstoffatmosphäre an 0,12g Palladium auf Kohle (10%) bei RT unter Eigendruck hydriert. Nach vollständiger Umsetzung des Ausgangsmaterials wurde abfiltriert und eingeengt. Der Rückstand wurde über präparative HPLC gereinigt. Es wurden 0,06g der Titelverbindung erhalten.
LC/MS (Methode B): Rₜ = 0,22 min, m/z: 295 [MH⁺].

### E. (S)-3-(6-tert-Butoxycarbonylamino-pyridin-3-yl)-2-[3-((8S,11R)-8-isopropyl-10-oxo-6-oxa-1,9,14-triaza-bicyclo[11.2.1]hexadeca-13(16),14-dien-11-yl)-ureido]-propionsäure-tert-butyl ester

Eine Lösung von 56 mg (190µmol) (8S,11R)-11-Amino-8-isopropyl-6-oxa-1,9,14-triaza-bicyclo[11.2.1]hexadeca-13(16),14-dien-10-on aus Schritt D in 5ml DMF wurde auf 0 C gekühlt und unter Rühren mit 32 mg (194µmol) 1,1'-Carbonyldi-imidazol versetzt. Es wurde für 30 min gerührt, anschließend 64 mg (190µmol) (S)-2-Amino-3-(6-tert-Butoxycarbonylamino-pyridin-3-yl)-propionsäure-tert-butylester zugegeben und auf RT erwärmt. Nach Stehen über Nacht wurde nochmals die gleiche Menge 1,1'-Carbonyldiimidazol zugegeben und für weitere 24 h gerührt. Danach wurde eingeengt und über präparative HPLC gereinigt. Es wurden 27 mg der gewünschten Verbindung erhalten.

### LC/MS (Methode B): Rₜ = 0,58 min, m/z: 658,9 [MH⁺].

### F. (S)-3-(6-Amino-pyridin-3-yl)-2-[3-((8S,11R)-8-isopropyl-10-oxo-6-oxa-1,9,14-triaza-bicyclo[11.2.1]hexadeca-13(16),14-dien-11-yl)-ureido]-propionsäure

Eine Lösung von 27 mg (41µmol) in 6ml TFA/Dichlormethan (1:1, v/v) wurde für 4 h bei RT gerührt. Anschließend wurde eingeengt und nach Aufnehmen in 1 N Salzsäure gefriergetrocknet. Es wurden 20 mg der Titelverbindung als Hydrochlorid erhalten.

LC/MS (Methode B): Rₜ = 0,36 min, m/z: 502,9 [MH⁺].

### Beispiel 6-2

### (S)-3-(6-Amino-pyridin-3-yl)-2-[3-((3R,6S)-6-isopropyl-4-oxo-8-oxa-5-azabicyclo[11.2.2]heptadeca-1(16),13(17),14-trien-3-yl)ureido]-propionsäure

### A. (R)-3-(4-Allyl-phenyl)-2-tert-butoxycarbonylamino-propionsäure

Eine Lösung von 2,03g (6,36 mmol) (R)-3-(4-Allyl-phenyl)-2-tert-butoxycarbonyl-amino-propionsäuremethylester (hergestellt nach Synlett, 2005, 12, 1877-1880) in 20 ml Dioxan/Wasser (1:1, v/v) und 0,30g (12,71 mmol) Lithiumhydroxyd wurde 2 h bei RT gerührt und anschließend mit 1 N Salzsäure neutralisiert. Das Reaktionsgemisch wurde mit Ethylacetat extrahiert, die organische Phase über Na₂SO₄ getrocknet, filtriert und eingeengt. Es wurden 1,90 g der Titelverbindung erhalten. LC/MS (Methode B): Rₜ = 0,97 min, m/z: 206,0 [MH⁺].

Die Verbindung wurde anstelle von (R)-3-(4-Allyloxy-phenyl)-2-(9H-fluoren-9-ylmethoxycarbonylamino)-propionsäure eingesetzt, um analog zu Beispiel 5-2 das Beispiel 6-2 herzustellen. Die anstelle der Fmoc-Schutzgruppe verwendetet Boc-Schutzgruppe wurde in bekannter Weise mit Dichlormethan/TFA-Gemischen abgespalten. LC/MS (Methode I): Rₜ = 1,29 min, m/z: 512,3 [MH⁺].

### Beispiel 6-3

### (S)-3-(6-Amino-pyridin-3-yl)-2-[3-((9S,12R)-16-fluoro-9-isopropyl-11-oxo-2,7-dioxa-10-aza-bicyclo[12.2.2]octadeca-1(17),14(18),15-trien-12-yl)-ureido]-propionsäure

### A. (R)-3-(4-Allyloxy-3-fluoro-phenyl)-2-tert-butoxycarbonylamino-propionsäure

Zu einer Lösung von 4,0 g (13,36 mmol) (R)-2-tert-Butoxycarbonylamino-3-(3-fluoro-4-hydroxy-phenyl)-propionsäure in 23 ml DMF wurden bei 0 °C unter Rühren 1,18 g (60%ig, 29,39 mmol) Natriumhydrid gegeben und bei dieser Temperatur gerührt. Nach 15 min wurden 1,27ml (1,78g, 14,7 mmol) Allylbromid zugegeben und für 1 h weiter gerührt, während der Ansatz auf RT kam. Es wurde mit Methanol gequencht und nach dem Abkühlen überschüssiges Methanol abrotiert. Nach Verdünnen mit Diethylether wurde mit 1 N Salzsäure angesäuert, die wässrige Phase noch zweimal mit Diethylether extrahiert, die vereinigten organischen Phasen mit gesättigter Natriumchlorid-Lösung gewaschen, über NaSO₄ getrocknet, filtriert und eingeengt. Es wurden 4,50 g eines gelblichen Öls erhalten.
LC/MS (Methode B): Rₜ = 0,94 min, m/z: 239,9 [MH⁺].

Die Verbindung wurde anstelle von (R)-3-(4-Allyloxy-phenyl)-2-(9H-fluoren-9-ylmethoxycarbonylamino)-propionsäure eingesetzt, um analog zu Beispiel 5-2 das Beispiel 6-3 herzustellen. Die anstelle der Fmoc-Schutzgruppe verwendetet Boc-Schutzgruppe wurde in bekannter Weise mit Dichlormethan/TFA-Gemischen abgespalten.
LC/MS (Methode E): Rₜ = 2,40 min, m/z: 546,43 [MH⁺].

Unter Verwendung von (R)-3-Allyloxy-2-tert-butoxycarbonylamino-propionsäure (hergestellt wie analog in JACS, 129 (22), 6986-6987, 2007, beschrieben) anstelle von (R)-3-(4-Allyloxy-phenyl)-2-(9H-fluoren-9-ylmethoxycarbonylamino)-propionsäure wurden in gleicher Weise wie oben folgende Beispiele hergestellt:

| Beispiel | Name | Rₜ (min) | m/z [MH⁺] | Methode |
|---|---|---|---|---|
| 6-4 | (S)-3-(6-Amino-pyridin-3-yl)-2-[3-((3S,6R)-3-isopropyl-5-oxo-1,8-dioxa-4-aza-cyclododec-6-yl)-ureido]-propionsäure | 1,42 | 452,29 | G |
| 6-5 | (S)-3-(6-Amino-pyridin-3-yl)-2-(3-((3S,6R)-3-isopropyl-5-oxo-1,8-dioxa-4-aza-cyclododec-6-yl)-ureido]-propionsäuremethylester | 2,40 | 466,33 | D |
| 6-6 | (S)-6-Amino-2-[3-((3S,6R)-3-isopropyl-5-oxo-1,8-dioxa-4-aza-cyclododec-6-yl)-ureido]-hexansäure | 1,94 | 417,23 | C |

### Beispiel 6-7

### (S)-6-Amino-2-[3-((9S,12S)-9-isopropyl-11-oxo-2,7-dioxa-10-aza-bicyclo-[12.2.2]octadec-12-yl)-ureido]-hexansäure

### A. (S)-2-tert-Butoxycarbonylamino-3-(4-hydroxy-cyclohexyl)-propionsäure

Zu einer Lösung von 7,00g (24,88 mmol) (S)-2-tert-Butoxycarbonylamino-3-(4-hydroxy-phenyl)-propionsäure in 50 ml Methanol wurden 0,74 g (7,17 mmol) Rhodium gegeben und bei 50 C/6bar Wasserstoffdruck hydriert. Nach vollständigem Umsatz wurde vom Katalysator abfiltriert und das Filtrat eingeengt. Das Produkt war rein genug für weitere Umsetzungen.
LC/MS (Methode K): Rₜ = 1,00 min, m/z: 188,2 [MH⁺].

### B. (S)-3-(4-Allyloxy-cyclohexyl)-2-tert-butoxycarbonylamino-propionsäure

Zu einer Suspension aus 1,89 g (47,14 mmol) 60%igem Natriumhydrid in 20 ml DMF wurde innerhalb von 30 min bei 0 C eine Lösung von 5,42 g (18,86 mmol) (S)-2-tert-Butoxycarbonylamino-3-(4-hydroxy-cyclohexyl)-propionsäure in 20 ml DMF getropft. Anschließend wurden 2,28g (18,86 mmol) Allylbromid zugegeben und es wurde auf RT erwärmt und 3 h nachgerührt. Es wurde vorsichtig mit Wasser gequencht und eingeengt. Der Rückstand wurde in Wasser gelöst und mit Ethylacetat gewaschen. Die wässrige Phase wurde mit 6 M HCl auf pH 2 gestellt und mit Ethylacetat extrahiert. Die organische Phase wurde über Na₂SO₄ getrocknet, filtriert und eingeengt. Die so erhaltene Titelverbindung wurde ohne weitere Reinigung weiter umgesetzt.
LC/MS (Methode K): Rₜ = 1,46 min, m/z: 228,1 [MH⁺-Boc].

Die weiteren Umsetzungen zu Beispiel 6-7 wurden wie oben beschrieben durchgeführt, indem B. anstelle von D-Boc-O-Allyl-Tyrosin eingesetzt wurde.
LC/MS (Methode F): Rₜ = 2,8 min, m/z: 499,55 [MH⁺].

### Beispiel 7-1

### (S)-6-Amino-2-[3-((8S,11R)-8-isopropyl-3,10-dioxo-6-oxa-2,9-diaza-bicyclo[11.2.2]heptadeca-1(16),13(17),14-trien-11-yl)-ureido]-hexansäure

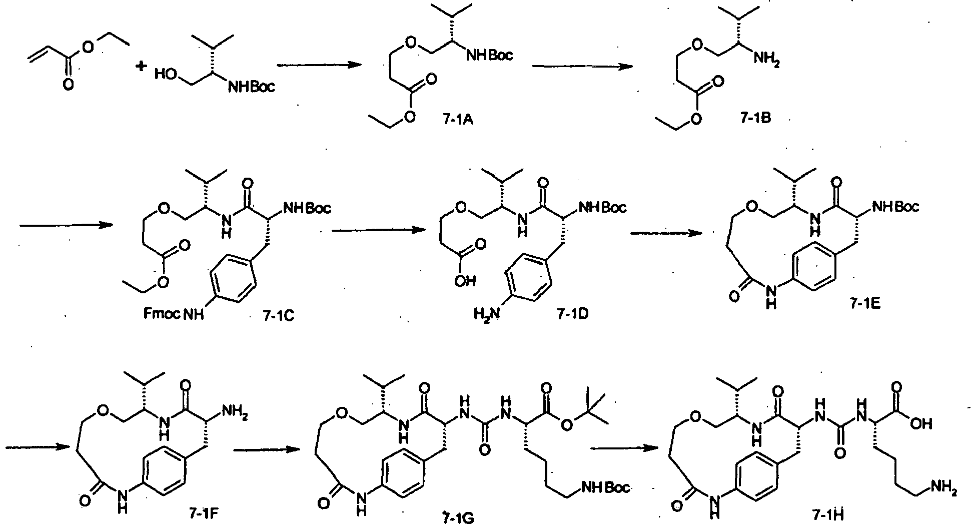

### A. 3-((S)-2-tert-Butoxycarbonylamino-3-methyl-butoxy)-propionsäureethylester

Zu einer Lösung von 3,00 g (14,76 mmol) Boc-L-Valinol in 20 ml absol. THF wurden unter Argon ca. 200 mg Natrium gegeben. Nach 2 h wurde die Lösung in einen mit Argon gespülten Kolben überkanüliert - nicht gelöstes Natrium blieb zurück. Zu der Lösung wurden 2.25 ml (2,22 g, 22,14 mmol) Ethylacrylat gegeben und 2 h bei Raumtemp. gerührt. Die Reaktionsmischung wurde mit 6 Tropfen Eisessig versetzt und unterverminderten Druck eingeengt. Der so erhaltene Rückstand (4,45 g farbloses Öl) wurde roh in der nächsten Stufe eingesetzt.

### B. 3-((S)-2-Amino-3-methyl-butoxy)-propionsäureethylester

Das in 7-1 A erhaltene Rohprodukt (∼ 4,45 g) wurde in einer Mischung aus 10 ml CH₂Cl₂ und 10 ml TFA gelöst und 2 h bei Raumtemp. gerührt. Nach beendeter Reaktion wurde im Vakuum eingeengt und mehrfach mit Toluol kodestilliert. Das so erhaltene Rohprodukt wurde durch präparative HPLC (Acetonitril / Wasser+ Zusatz von 0.5% TFA) gereinigt. Auf diese Weise wurden 370 mg 3-((S)-2-Amino-3-methyl-butoxy)-propionsäureethylester als Trifluoracetat in Form eines farblosen Öls erhalten (Ausbeute - 8%).
LC/MS (Methode B): Rₜ = 0,52 min, m/z: 204,3 [MH⁺].

### C. 3-((S)-2-{(R)-2-tert-Butoxycarbonylamino-3-[4-(9H-fluoren-9-ylmethoxy-carbonylamino)-phenyl]-propionylamino}-3-methyl-butoxy)-propionsäureethylester

Zu einer Mischung aus 586,0 mg (1,17 mmol) R)-2-tert-Butoxycarbonylamino-3-[4 -(9H-fluoren-9-ylmethoxycarbonylamino)-phenyl]-propionsäure und 370,0 mg (1,17 mmol) 3-((S)-2-Amino-3-methyl-butoxy)-propionsäureethylester in 10 ml DMF wurden nacheinander 158,7 mg (1,17 mmol) HOAt, 0,6 ml (3,5 mmol) N,N-Diisopropylethylamin sowie 443,3 mg (1,17 mmol) HATU gegeben und 2h bei Raumtemp. gerührt. Die Reaktionsmischung wurde im Vakuum eingeengt. Der verbliebene Rückstand wurde in Dichlormethan aufgenommen, mit ges. NaHCO₃-Lösung gewaschen, getrocknet und unter vermindertem Druck vom Lösungsmittel befreit. Das so erhaltene Rohprodukt wurde durch Flash-Chromatographie an Kieselgel (Heptan / Ethylacetat 1:1) gereinigt. Auf diese Weise wurden 355,0 mg reiner 3-((S)-2-{(R)-2-tert-Butoxycarbonylamino-3-[4-(9H-fluoren-9-ylmethoxy-carbonylamino)-phenyl]-propionylamino}-3-methyl-butoxy)-propionsäureethylester erhalten (Ausbeute ∼ 44%).
LC/MS (Methode B): Rₜ = 1,23 min, m/z: 588,3 [M-Boc+H⁺].

### D. 3-{(S)-2-[(R)-3-(4-Amino-phenyl)-2-tert-butoxycarbonylamino-propionylamino]-3-methyl-butoxy}-propionsäure

355,0 mg (0,52 mmol) 3-((S)-2-{(R)-2-tert-Butoxycarbonylamino-3-[4-(9H-fluoren-9-ylmethoxy-carbonylamino)-phenyl]-propionylamino}-3-methyl-butoxy)-propionsäure-ethylester wurden in einer Mischung aus 9 ml THF und 3 ml MeOH gelöst. Es wurden 1,29 ml (1,29 mmol) einer wässrigen 1 M LiOH-Lösung hinzugegeben und die resultierende Reaktionsmischung 1 h bei Raumtemp. gerührt. Nach beendeter Reaktion wurde durch Zugabe von wenig wässriger 1 N HCl-Lösung neutral gestellt, unter vermindertem Druck eingeengt und mit Toluol kodestilliert. Das so erhaltene Produkt (225,0 mg) wurde roh in der nächsten Reaktion eingesetzt.
LC/MS (Methode B): Rₜ = 0,63 min, m/z: 438,3 [MH⁺].

### E. ((8S,11R)-8-Isopropyl-3,10-dioxo-6-oxa-2,9-diaza-bicyclo[11.2.2]heptadeca-1(16),13(17),14-trien-11-yl)-carbaminsäure-tert-butylester

Die als Rohprodukt in 7-1 D erhaltene 3-{(S)-2-[(R)-3-(4-Amino-phenyl)-2-tert-butoxycarbonylamino-propionylamino]-3-methyl-butoxy}-propionsäure (∼ 225 mg) wurde in 225 ml DMF gelöst. Zu dieser Lösung wurden nacheinander 70,0 mg (0,51 mmol) HOAt, 0,26 ml (1,5 mmol) N,N-Diisopropylethylamin sowie 195,4 mg (0,51 mmol) HATU gegeben und 1 h bei Raumtemp. gerührt. Die Reaktionsmischung wurde im Vakuum eingeengt. Der so erhaltene Rückstand wurde in CH₂Cl₂ aufgenommen, mit ges. NaHCO₃-Lösung gewaschen, über MgSO₄ getrocknet und unter Vakuum vom Lösungsmittel befreit. Die so erhaltene ring-geschlossene Verbindung wurde roh in der nächsten Stufe eingesetzt. Ausbeute: 215,0 mg.
LC/MS (Methode B): Rₜ = 0,76 min, m/z: 364,3 [M-tBu+H⁺].

### F. (8S,11R)-11-Amino-8-isopropyl-6-oxa-2,9-diaza-bicyclo[11.2.2]heptadeca-1(16),13(17),14-triene-3,10-dion

Das in 7-1 E erhaltene Rohprodukt (∼215 mg) wurde in einer Mischung aus 4,75 ml TFA, 0,13 ml Wasser und 0,13 ml Triisopropylsilan 2 h bei Raumtemp. gerührt. Nach beendeter Reaktion wurde im Vakuum eingeengt und mehrfach mit Toluol kodestilliert. Das so erhaltene Rohprodukt wurde durch präparative HPLC (Acetonitril / Wasser+ Zusatz von 0.1% TFA) gereinigt. Auf diese Weise wurden 80 mg (8S,11R)-11-Amino-8-isopropyl-6-oxa-2,9-diaza-bicyclo[11.2.2]heptadeca-1(16),13(17),14-triene-3,10-dion als Trifluoracetat in Form eines farblosen amorphen Materials erhalten.
LC/MS (Methode B): Rₜ = 0,44 min, m/z: 321,3 [MH⁺].

### G. (S)-6-tert-Butoxycarbonylamino-2-[3-((8S,11R)-8-isopropyl-3,10-dioxo-6-oxa-2,9-diaza-bicyclo[11.2.2]heptadeca-1(16),13(17),14-trien-11-yl)-ureido]-hexansäure-tert-butyl ester

81,9 mg (0,41 mmol) 4-Nitrophenylchlorformiat wurden in 3 ml CH₂Cl₂ gelöst. Unter Eisbadkühlung wurden eine Lösung aus 80 mg (0.19 mmol) des in 7-1 F erhaltenen (8S,11 R)-11-Amino-8-isopropyl-6-oxa-2,9-diaza-bicyclo[11.2.2]heptadeca-1(16),13(17),14-triene-3,10-dions und 69 µl (0,41 mmol) N,N-Diisopropylethylamin in 3 ml CH₂Cl₂ zugegeben. Anschließend wurde 3 h bei Raumtemp. gerührt. Die Reaktionsmischung wurde mit ges. NaHCO₃-Lösung, Wasser und gesättigter NaCl-Lösung gewaschen, über MgSO₄ getrocknet und im Vakuum eingeengt. Der so erhaltene Rückstand wurde in 3 ml DMF gelöst und mit einer Lösung aus 68,8 mg (0,2 mmol) H-Lys(Boc)-OtBu Hydrochlorid und 65,9 µl (0,39 mmol) N,N-Diisopropylethylamin in 3 ml DMF versetzt. Nach Rühren bei Raumtemp. über Nacht wurde die Reaktionsmischung im Vakuum eingeengt. Das so erhaltene Rohprodukt wurde durch Flash-Chromatographie an Kieselgel (Heptan / Ethylacetat 1:1) gereinigt. Ausbeute : 20 mg (S)-6-tert-Butoxycarbonylamino-2-[3-((8S,11R)-8-isopropyl-3,10-dioxo-6-oxa-2,9-diaza-bicyclo[11.2.2]heptadeca-1(16),13(17),14-trien-11-yl)-ureido]-hexansäure-tert-butylester.
LC/MS (Methode B): Rₜ = 0,91 min, m/z: 648,5 [MH⁺].

### H. (S)-6-Amino-2-[3-((8S,11R)-8-isopropyl-3,10-dioxo-6-oxa-2,9-diaza-bicyclo[11.2.2]heptadeca-1(16),13(17),14-trien-11-yl)-ureido]-hexansäure

20,0 mg (0,03 mmol) (S)-6-tert-Butoxycarbonylamino-2-[3-((8S,11R)-8-isopropyl-3,10-dioxo-6-oxa-2,9-diaza-bicyclo[11.2.2]heptadeca-1(16),13(17),14-trien-11-yl)-ureido]-hexansäure-tert-butyl ester wurden in einer Mischung aus 0,95 ml TFA, 25 µl Wasser und 25 µl Triisopropylsilan 2 h bei Raumtemp. gerührt. Nach beendeter Reaktion wurde im Vakuum eingeengt und mehrfach mit Toluol kodestilliert. Das so erhaltene Rohprodukt wurde durch präparative HPLC (Acetonitril / Wasser+ Zusatz von 0. 1 % TFA) gereinigt. Auf diese Weise wurden 5 mg der Titelverbindung als Trifluoracetat in Form eines farblosen amorphen Materials erhalten.
LC/MS (Methode B): Rₜ = 0,47 min, m/z: 492,3 [MH⁺].

Die folgenden Beispiele wurden in analoger Weise hergestellt:

### Beispiel 7-2

### (S)-3-(6-Amino-pyridin-3-yl)-2-[3-((8S,11R)-8-methyl-3,10-dioxo-6-oxa-2,9-diaza-bicyclo[11.2.2]heptadeca-1(16),13(17),14-trien-11-yl)-ureido]-propionsäure

LC/MS (Methode B): Rₜ = 0,28 min, m/z: 499,25 [MH⁺].

### Beispiel 7-3

### (S)-3-(6-Amino-pyridin-3-yl)-2-[3-((3S,6R)-3-isopropyl-5,9-dioxo-1-oxa-4,10-diaza-cyclotridec-6-yl)-ureido]-propionsäure

LC/MS (Methode B): Rₜ = 0,42 min, m/z: 479,33 [MH⁺].

### Beispiel 7-4

### (S)-3-(6-Amino-pyridin-3-yl)-2-[3-((8S,11R)-8-isopropyl-2,10-dioxo-6-oxa-3,9-diaza-bicyclo[11.2.2]heptadeca-1(16),13(17),14-trien-11-yl)-ureido]-propionsäure

LC/MS (Methode A): Rₜ = 0,82 min, m/z: 527,20 [MH⁺].

### Beispiel 8-1

### (S)-6-Amino-2-[3-((9S,12R)-4,5-dihydroxy-9-isopropyl-11-oxo-2,7-dioxa-10-aza-bicyclo[12.2.2]octadeca-1 (17),14(18),15-trien-12-yl)-ureido]-hexansäure

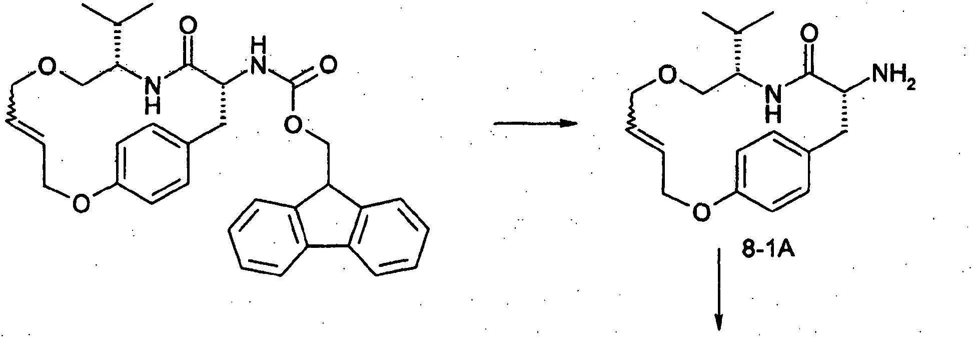

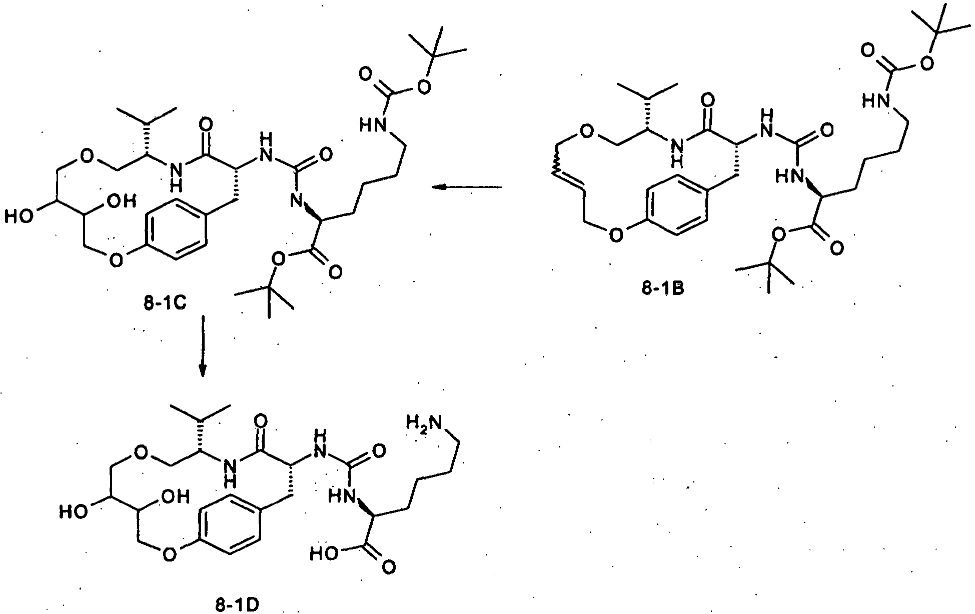

### A. (9S,12R)-12-Amino-9-isopropyl-2,7-dioxa-10-aza-bicyclo[12.2.2]octadeca-1(17),4,14(18),15-tetraen-11-one

Eine Lösung von 3,66g (6,78 mmol) ((9S,12R)-9-Isopropyl-11-oxo-2,7-dioxa-10-aza-bicyclo[12.2.2]octadeca-1(17),4,14(18),15-tetraen-12-yl)-carbaminsäure 9H-fluoren-9-ylmethylester (1-1 D) in 300 ml Dichlormethan wurde mit 75 ml Diethylamin versetzt und für 5h bei RT gerührt. Der Ansatz wurde eingeengt und druch präparative HPLC gereinigt. Von den vereinigten Produktfraktionen wurde Acetonitril abrotiert, mit gesättigter NaHCO₃-Lösung vesetzt und zweimal mit Ethylacetat extrahiert, mit Natriumchlorid gesättigt und nochmals mit Ethylacetat extrahiert. Die vereinigten Ethylacetatphasen wurden über Na₂SO₄ getrocknet, filtriert und eingeengt. Die erhaltene Substanz war rein genug für weitere Umsetzungen.
LC/MS (Methode B): Rₜ = 0,58 min, m/z: 319,2 [MH⁺].

### B. (S)-6-tert-Butoxycarbonylamino-2-[3-((9S,12R)-9-isopropyl-11-oxo-2,7-dioxa-10-aza-bicyclo[12.2.2]octadeca-1(17),4,14(18),15-tetraen-12-yl)-ureido]-hexansäure tert-butylester

Die Umsetzung erfolgte wie unter 1-1F beschrieben. Man erhielt 1,13 g der Titelverbindung.
LC/MS (Methode B): Rₜ = 1,00 min, m/z: 647,3 [MH⁺].

### C. (S)-6-tert-Butoxycarbonylamino-2-[3-((9S,12R)-4,5-dihydroxy-9-isopropyl-11-oxo-2,7-dioxa-10-aza-bicyclo[12.2.2]octadeca-1(17),14(18),15-trien-12-yl)-ureido]-hexansäure tert-butyl ester

Eine Mischung aus 541 mg (387 mmol) AD-Mix-alpha und 3 mg (7,7 µmol) Kaliumosmat wurde in tert-Butanol/Wasser (1:1, v/v) vorgelegt und mit 8 mg (9,7 µmol) (DHQ)2PHAL (Hydroquinine 1,4-phthalazindiyl diether) versetzt. Nach Entstehen einer klaren Lösung wurde das Reaktionsgemisch auf 0 °C gekühlt, 41 mg (425 µmol) Methansulfonamid zugegeben und für 15 min bei dieser Temperatur gerührt. Danach wurden 250 mg (387 µmol) (S)-6-tert-Butoxycarbonylamino-2-[3-((9S,12R)-9-isopropyl-11-oxo-2,7-dioxa-10-aza-bicyclo[12.2.2]octadeca-1(17),4,14(18),15-tetraen-12-yl)-ureido]-hexansäure tert-butylester (Schritt B) zugegeben, man erwärmte auf RT und ließ über Wochenende stehen. Danach wurde mit 195 mg (1,55 mmol) Natriumsulfit versetzt und 1 h bei RT gerührt. Das Reaktionsgemisch wurde mit Ethylacetat extrahiert, die organische Phase über Na₂SO₄ getrocknet, filtriert und einrotiert. Der Rückstand wurde über präparative HPLC gereinigt. Man erhielt 164 mg der Titelverbindung.
LC/MS (Methode B): Rₜ = 0,84 min, m/z: 681,3 [MH⁺].

### D. (S)-6-Amino-2-[3-((9S,12R)-4,5-dihydroxy-9-isopropyl-11-oxo-2,7-dioxa-10-aza-bicyclo[12.2.2]octadeca-1 (17),14(18),15-trien-12-yl)-ureido]-hexansäure

Die Abspaltung der Schutzgruppen erfolgte wie oben beschrieben in TFA/Dichlormethangemisch. Es wurde ein Gemisch der Diastereomeren erhalten.
LC/MS (Methode B): Rₜ = 0,42 min (Doppelpeak), m/z: 525,3 [MH⁺].

### Beispiel 8-2

### (S)-6-Amino-2-[3-((9S,12R)-5-hydroxy-9-isopropyl-11-oxo-2,7-dioxa-10-aza-bicyclo[12.2.2]octadeca-1(17),14(18),15-trien-12-yl)-ureido]-hexansäure und (S)-6-Amino-2-[3-((9S,12R)-4-hydroxy-9-isopropyl-11-oxo-2,7-dioxa-10-aza-bicyclo[12.2.2]octadeca-1(17),14(18),15-trien-12-yl)-ureido]-hexansäure

### A. (S)-6-tert-Butyloxycarbonylamino-2-[3-((9S,12R)-5-hydroxy-9-isopropyl-11-oxo-2,7-dioxa-10-aza-bicyclo[12.2.2]octadeca-1(17),14(18),15-trien-12-yl)-ureido]-hexansäure-tert-butylester

Zu einer Lösung von 50 mg (77 µmol) (S)-6-tert-Butoxycarbonylamino-2-[3-((9S,12R)-9-isopropyl-11-oxo-2,7-dioxa-10-aza-bicyclo[12.2.2]octadeca-1(17),4,14(18),15-tetraen-12-yl)-ureido]-hexansäure tert-butylester (8-1 B) in 1,5ml THF wurden 1,55ml (773 µmol) (0,5M in THF) 9-Borabicyclo[3.3.1]nonan gegeben und über Nacht bei RT gerührt. Nach Zugabe von 250µl (1,49 mmol) 6N Natronlauge und 207 µl (1,83 mmol) Wasserstoffperoxid wurde mit Dichlormethan extrahiert. Die organische Phase wurde mit gesättigter Natriumchlorid-Lösung gewaschen, über Na₂SO₄ getrocknet, filtriert und eingeengt. Der Rückstand wurde über präparative HPLC gereinigt.
LC/MS (Methode B): Rₜ = 0,86 min, m/z: 665,5 [MH⁺].

### B. (S)-6-Amino-2-[3-((9S,12R)-5-hydroxy-9-isopropyl-11-oxo-2,7-dioxa-10-aza-bicyclo[12.2.2]octadeca-1 (17),14(18),15-trien-12-yl)-ureido]-hexansäure und (S)-6-Amino-2-[3-((9S,12R)-4-hydroxy-9-isopropyl-11-oxo-2,7-dioxa-10-azabicyclo[12.2.2]octadeca-1(17),14(18),15-trien-12-yl)ureido]hexansäure. Die Abspaltung der Schutzgruppen erfolgte wie oben beschrieben in TFA/Dichlormethangemisch und anschließenden Rühren in 1 N HCl.

LC/MS (Methode B): Rₜ = 0,48 min, m/z: 509,2 [MH⁺].

### Beispiel 8-3

3-(6-Amino-pyridin-3-yl)-2-[3-((9S,12R)-4,5-dihydroxy-9-isopropyl-11-oxo-2,7-dioxa-10-aza-bicyclo[12.2.2]octadeca-1(17),14(18),15-trien-12-yl)-ureido]-propionsäure Die Titelverbindung wurde in analoger Weise wie in den vorherigen Beispielen erhalten. LC/MS (Methode L): Rₜ = 2,15 min, m/z: 560,26 [MH⁺].

### Beispiel 9-1

### (S)-6-Amino-2-[((95,12R)-9-isopropyl-11-oxo-2,7-dioxa-10-aza-bicyclo[12.2.2]octadeca-1(17),14(18),15-trien-12-ylsulfamoyl)-amino]-hexansäure

### A. (S)-6-tert-Butyloxycarbonylamino-2-[((9S,12R)-9-isopropyl-11-oxo-2,7-dioxa-10-azabicyclo[12.2.2]octadeca-1 (17),14(18),15-trien-12-ylsulfamoyl)-amino]-hexansäure-tert-butylester

Eine Lösung von 387 mg (0,858 mmol) (S)-6-tert-Butoxycarbonylamino-2-(2-oxo-oxazolidine-3-sulfonylamino)-hexansäure-tert-butylester und 250mg (0,780 mmol) (9S,12R)-12-Amino-9-isopropyl-2,7-dioxa-10-aza-bicyclo[12.2.2]octadeca-1(17),14(18),15-trien-11-on (Verbindung 1-1E) in 15 ml Acetonitril wurde bei 80° C gerührt. Nach 1 Tag (d) wurde nochmals die gleiche Menge des Oxazolidins zugegeben und für 1d weitergerührt. Das Reaktionsgemisch wurde anschließend eingeengt und der Rückstand durch präp. HPLC gereinigt. Produkthaltige Fraktionen wurden vereinigt, das Acetonitril abgedampft, mit ges. NaHCO₃-Lsg. leicht alkalisch gestellt und mehrfach mit Ethylacetat extrahiert. Die vereinigten organischen Phasen wurden über Na₂SO₄ getrocknet, filtriert und eingeengt. Es wurden 78mg der Titelverbindung erhalten.
LC/MS (Methode B): Rₜ = 1,16 min, m/z: 585,9 [MH-Boc⁺].

### B. (S)-6-Amino-2-[((9S,12R)-9-isopropyl-11-oxo-2,7-dioxa-10-aza-bicyclo[12.2.2]octadeca-1(17),14(18),15-trien-12-ylsulfamoyl)-amino]-hexansäure

Eine Lösung von 78mg (0,114 mmol) (S)-6-tert-Butyloxycarbonylamino-2-[((9S,12R)-9-isopropyl-11-oxo-2,7-dioxa-10-aza-bicyclo[12.2.2]octadeca-1(17),14(18),15-trien-12-ylsulfamoyl)-amino]-hexansäure-tert-butylester in 3 ml Dichlormethan/TFA (1:1, v/v) wurde für 2 h bei RT stehen gelassen und dann eingeengt. Das Rohprodukt wurde duch präp. HPLC gereinigt. Die Wertfraktionen wurden vereinigt und nach Zugabe von 1 N Salzsäure gefriergetrocknet. Es wurden 34 mg der Titelverbindung als Hydrochlorid erhalten. LC/MS (Methode F): Rₜ = 2,97 min, m/z: 529,23 [MH⁺].

### Beispiel 9-2

### (S)-6-Amino-2-[((9S,12S)-9-isopropyl-11-oxo-2,7-dioxa-10-azabicyclo[12.2.2]octadeca-1(17),14(18),15-trien-12-ylsulfamoyl)amino]hexansäure

Die Titelverbindung wurde in analoger Weise wie in den vorherigen Beispielen erhalten. LC/MS (Methode F): Rₜ = 2,81 min, m/z: 529,23 [MH⁺].

### Pharmakologische Beispiele

Die hergestellten Substanzen wurden mit dem Actichrome Plasma TAFI Activity Kit der Firma American Diagnostica (Pr.Nr. 874) auf Inhibition von TAFIa geprüft. Dabei wurden zu 2 µl 2,5 mM DMSO-Lösung der Substanz 28 µL Testpuffer (20mM Hepes, 150mM NaCl, pH 7,4) und 10 µL TAFIa (American Diagnostica Pr.Nr. 8.74TAFIA; 2,5 µg /ml) gegeben und 15 Minuten bei RT in einer 96 half-well Mikrotiterplatte inkubiert. Die Enzymreaktion wurde durch Zugabe von 10 µL TAFIa "Developer" (1:2 mit Testpuffer vorverdünnt) gestartet. Der Zeitverlauf der Reaktion wurde bei 420 nm in einem Mikrotiterplattenreader (SpectraMax plus 384; Fa. Molecular Devices) über 15 Minuten verfolgt.

Die IC₅₀-Werte wurden aus den gemittelten Werten (Doppelbestimmung) einer Verdünnungsreihe der Substanz mit Hilfe der Software Softmax Pro (Version 4.8; Fa. Molecular Devices) berechnet.

Tabelle 1 zeigt die Ergebnisse.

**Tabelle 1:**

| Beispiel Nr. | IC₅₀-Wert [µM] | Beispiel Nr. | IC₅₀-Wert [µM] | Beispiel Nr. | IC₅₀-Wert [µM] | Beispiel Nr. | IC₅₀-Wert [µM] |
|---|---|---|---|---|---|---|---|
| 1-1 | 0,006 | 1-8 | 0,029 | 5-3 | 0,032 | 7-4 | 0,040 |
| 1-2 | 9,044 | 1-9 | 0,019 | 6-1 | 0,052 | 8-1 | 0,023 |
| 1-3 | 0,269 | 2-1 | 0,0088 | 6-2 | 0,021 | 8-2 | 0,033 |
| 1-4 | 0,071 | 3-1 | 0,0077 | 6-3 | 0,012 | 8-3 | 0,059 |
| 1-5 | 0,105 | 4-1 | 0,017 | 6-4 | 0,055 | | |
| 1-6 | 0,811 | 5-1 | 0,649 | 6-6 | 0,053 | | |
| 1-7 | 0,049 | 5-2 | 0,009 | 7-1 | 0,022 | | |

## Patentansprüche

1. Verbindung der Formel I 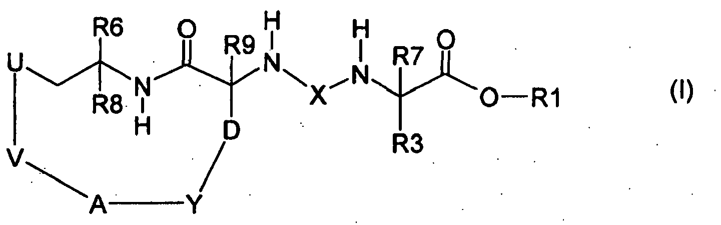 und/oder einer stereoisomeren Form der Verbindung der Formel I und/oder Gemische dieser Formen in jedem Verhältnis, und/oder ein physiologisch verträgliches Salz der Verbindung der Formel (I), wobei
X für -C(O)- oder -SO₂- steht,
U für Sauerstoffatom, Schwefelatom, NH, -C(O)-NH- oder -(C₀-C₄)-Alkylen-steht,
A für Sauerstoffatom, Schwefelatom, NH, -C(O)-NH- -NH-C(O)-, -NR2- oder -(C₀-C₄)-Alkylen- steht,
V für
1) -(C₂-C₉)-Alkylen-, wobei Alkylen unsubstituiert oder unabhängig voneinander ein-, zwei- oder dreifach durch -OH, NH₂ oder Halogen substituiert ist,
2) -(C₁-C₂)-Alkylen-(C₃-C₆)-Cycloalkyl-(C₁-C₂)-Alkylen-, wobei Cycloalkyl unabhängig voneinander ein-, zwei- oder dreifach durch R15 substituiert ist, oder
3) -(C₃-C₉)-Alkenylen- steht,
D für -(C₁-C₂)-Alkylen- steht,
Y für
1) kovalente Bindung,
2) -(C₃-C₁₂)-Cycloalkyl, wobei Cycloalkyl unabhängig voneinander ein-, zwei- oder dreifach durch R15 substituiert ist,
3) -(C₆-C₁₄)-Aryl, wobei Aryl unsubstituiert oder unabhängig voneinander ein-, zwei- oder dreifach durch R15 substituiert ist, oder
4) Het, wobei unter Het ein 4- bis 15-gliedriges heterocyclisches Ringsystem mit 4 bis 15 Ringatomen verstanden wird, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten und
worin Het unsubstituiert oder unabhängig voneinander ein-, zwei- oder dreifach durch ein -(C₁-C₃)-Alkyl, Halogen, -NH₂, -CF₃ oder -O-CF₃ substituiert ist, steht,
R1 für
1) Wasserstoffatom,
2) -(C₁-C₆)-Alkyl,
3) -(C₁-C₆)-Alkyl-OH,
4) -(C₀-C₄)-Alkyl-(C₃-C₆)-Cycloalkyl,
5) -(C₁-C₁₀)-Alkyl-O-C(O)-O-R2,
6) -(CH₂)ᵣ-(C₆-C₁₄)-Aryl, worin Aryl unsubstituiert oder unabhängig voneinander ein-, zwei- oder dreifach durch R15 substituiert ist und r die ganze Zahl Null, 1, 2 oder 3 bedeutet, oder
7) -(CH₂)ₛ-Het, wobei unter Het ein 4- bis 15-gliedriges heterocyclisches Ringsystem mit 4 bis 15 Ringatomen verstanden wird, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten und worin Het unsubstituiert oder unabhängig voneinander ein-, zwei- oder dreifach durch R15 substituiert ist und s die ganze Zahl Null, 1, 2 oder 3 bedeutet, steht,
R2 für
1) -(C₁-C₆)-Alkyl,
2) -(CH₂)ᵣ-(C₆-C₁₄)-Aryl, worin Aryl unsubstituiert oder unabhängig voneinander ein-, zwei- oder dreifach durch R15 substituiert ist und r die ganze Zahl Null, 1, 2 oder 3 bedeutet, oder
3) -(C₀-C₄)-Alkyl-(C₃-C₆)-Cycloalkyl, steht,
R3 für
1) -(C₂-C₆)-Alkylen-NH₂, wobei Alkylen unsubstiuiert ist oder ein-, zwei-, drei- oder vierfach durch Halogen substituiert ist,
2) -(C₁-C₄)-Alkylen-O-(C₁-C₄)-Alkylen-NH₂,
3) -(C₁-C₄)-Alkylen-SO₂-(C₁-C₄)-Alkylen-NH₂,
4) -(C₀-C₄)-Alkylen-Het, wobei Het wie oben definiert ist und durch -NH₂ und ein-, zwei- oder dreifach durch R15 substituiert ist,
5) -(C₀-C₄)-Alkylen-(C₃-C₈)-Cycloalkyl-NH₂ oder
6) -(C₀-C₆)-Alkylen-cyclisches Amin, steht
R6 für
1) Wasserstoffatom,
2) -(C₁-C₆)-Alkyl, wobei Alkyl unsubstituiert oder unabhängig voneinander ein-, zwei- oder dreifach durch R16 substituiert ist,
3) -O-(C₁-C₆)-Alkyl, wobei Alkyl unsubstituiert oder unabhängig voneinander ein-, zwei- oder dreifach durch R16 substituiert ist,
4) -(C₀-C₄)-Alkylen-Het, wobei Het wie oben definiert ist, wobei Alkylen und Het unsubstituiert oder unabhängig voneinander ein-, zwei- oder dreifach durch R16 substituiert sind,
5) -(C₀-C₄)-Alkylen-Aryl, wobei Alkylen und Aryl unsubstituiert oder unabhängig voneinander ein-, zwei- oder dreifach durch R16 substituiert sind, oder
6) -(C₀-C₄)-Alkylen-(C₃-C₈)-Cycloalkyl, wobei Alkylen und Cycloalkyl unsubstituiert oder unabhängig voneinander ein-, zwei- oder dreifach durch R16 substituiert sind, steht,
R7 für Wasserstoffatom, Halogen oder -(C₁-C₆)-Alkyl steht,
R8 für Wasserstoffatom, Halogen oder -(C₁-C₆)-Alkyl steht,
R9 für Wasserstoffatom, Halogen oder -(C₁-C₆)-Alkyl steht,
R15 für Wasserstoffatom, -(C₁-C₄)-Alkyl, -O-CF₃, -NH₂, -OH, -CF₃ oder Halogen steht, und
R16 für -O-CF₃, -NH₂, -OH, -CF₃ oder Halogen steht.

2. Verbindung der Formel I gemäß Anspruch 1, wobei
X für -C(O)- oder -SO₂- steht,
U für Sauerstoffatom, Schwefelatom, NH, -C(O)-NH- oder -(C₀-C₄)-Alkylensteht,
A für Sauerstoffatom, Schwefelatom, NH, -C(O)-NH- oder -(C₀-C₄)-Alkylensteht,
V für -(C₂-C₉)-Alkylen- oder -(C₃-C₉)-Alkenylen- steht,
D für -(C₁-C₂)-Alkylen- steht,
Y für
1) kovalente Bindung,
2) -(C₃-C₁₂)-Cycloalkyl, wobei Cycloalkyl unabhängig voneinander ein-, zwei- oder dreifach durch R15 substituiert ist,
3) -(C₆-C₁₄)-Aryl, wobei Aryl unsubstituiert oder unabhängig voneinander ein-, zwei- oder dreifach durch R15 substituiert ist, oder
4) Het, wobei unter Het ein 4- bis 15-gliedriges heterocyclisches Ringsystem mit 4 bis 15 Ringatomen verstanden wird, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten und
worin Het unsubstituiert oder unabhängig voneinander ein-, zwei- oder dreifach durch ein -(C₁-C₃)-Alkyl, Halogen, -NH₂, -CF₃ oder -O-CF₃ substituiert ist, steht,
R1 für
1) Wasserstoffatom,
2) -(C₁-C₆)-Alkyl,
3) -(C₁-C₆)-Alkyl-OH,
4) -(C₀-C₄)-Alkyl-(C₃-C₆)-Cycloalkyl,
5) -(C₁-C₁₀)-Alkyl-O-C(O)-O-R2,
6) -(CH₂)ᵣ-(C₆-C₁₄)-Aryl, worin Aryl unsubstituiert oder unabhängig voneinander ein-, zwei- oder dreifach durch R15 substituiert ist und r die ganze Zahl Null, 1, 2 oder 3 bedeutet, oder
7) -(CH₂)ₛ-Het, wobei unter Het ein 4- bis 15-gliedriges heterocyclisches Ringsystem mit 4 bis 15 Ringatomen verstanden wird, die in ein, zwei oder drei miteinander verbundenen Ringsystemen vorliegen und die je nach Ringgröße ein, zwei, drei oder vier gleiche oder verschiedene Heteroatome aus der Reihe Sauerstoff, Stickstoff oder Schwefel enthalten und worin Het unsubstituiert oder unabhängig voneinander ein-, zwei- oder dreifach durch R15 substituiert ist und s die ganze Zahl Null, 1, 2 oder 3 bedeutet, steht,
R2 für
1) -(C₁-C₆)-Alkyl,
2) -(CH₂)ᵣ-(C₆-C₁₄)-Aryl, worin Aryl unsubstituiert oder unabhängig voneinander ein-, zwei- oder dreifach durch R15 substituiert ist und r die ganze Zahl Null, 1, 2 oder 3 bedeutet, oder
3) -(C₀-C₄)-Alkyl-(C₃-C₆)-Cycloalkyl, steht,
R3 für
1) -(C₂-C₆)-Alkylen-NH₂, wobei Alkylen unsubstiuiert ist oder ein-, zwei-, drei- oder vierfach durch Halogen substituiert ist,
2) -(C₁-C₄)-Alkylen-O-(C₁-C₄)-Alkylen-NH₂,
3) -(C₁-C₄)-Alkylen-SO₂-(C₁-C₄)-Alkylen-NH₂,
4) -(C₀-C₄)-Alkylen-Het, wobei Het wie oben definiert ist und durch-NH₂ und ein-, zwei- oder dreifach durch R15 substituiert ist,
5) -(C₀-C₄)-Alkylen-(C₃-C₈)-Cycloalkyl-NH₂ oder
6) -(C₀-C₆)-Alkylen-cyclisches Amin, steht
R6 für
1) Wasserstoffatom,
2) -(C₁-C₆)-Alkyl, wobei Alkyl unsubstituiert oder unabhängig voneinander ein-, zwei- oder dreifach durch R16 substituiert ist,
3) -O-(C₁-C₆)-Alkyl, wobei Alkyl unsubstituiert oder unabhängig voneinander ein-, zwei- oder dreifach durch R16 substituiert ist,
4) -(C₀-C₄)-Alkylen-Het, wobei Het wie oben definiert ist, wobei Alkylen und Het unsubstituiert oder unabhängig voneinander ein-, zwei- oder dreifach durch R16 substituiert sind,
5) -(C₀-C₄)-Alkylen-Aryl, wobei Alkylen und Aryl unsubstituiert oder unabhängig voneinander ein-, zwei- oder dreifach durch R16 substituiert sind, oder
6) -(C₀-C₄)-Alkylen-(C₃-C₈)-Cycloalkyl, wobei Alkylen und Cycloalkyl unsubstituiert oder unabhängig voneinander ein-, zwei- oder dreifach durch R16 substituiert sind, steht,
R7 für Wasserstoffatom, Halogen oder -(C₁-C₆)-Alkyl steht,
R8 für Wasserstoffatom, Halogen oder -(C₁-C₆)-Alkyl steht,
R9 für Wasserstoffatom, Halogen oder -(C₁-C₆)-Alkyl steht,
R15 für Wasserstoffatom, -(C₁-C₄)-Alkyl, -O-CF₃, -NH₂, -OH, -CF₃ oder Halogen steht, und
R16 für -O-CF₃, -NH₂, -OH, -CF₃ oder Halogen steht.

3. Verbindung der Formel I gemäß der Ansprüch 1 oder 2, wobei
X für -C(O)- oder -SO₂- steht,
U für Sauerstoffatom, Schwefelatom, NH, -C(O)-NH- oder -(C₀-C₄)-Alkylen-steht,
A für Sauerstoffatom oder -(C₀-C₄)-Alkylen- steht,
V für -(C₂-C₈)-Alkylen- oder -(C₃-C₆)-Alkenylen- steht,
D für -(C₁-C₂)-Alkylen- steht,
Y für
1) kovalente Bindung,
2) -(C₃-C₆)-Cycloalkyl, wobei Cycloalkyl unabhängig voneinander ein-, zwei- oder dreifach durch R15 substituiert ist,
3) -(C₆-C₁₄)-Aryl, wobei Aryl ausgewählt ist aus der Gruppe Phenyl, Naphthyl, Anthryl oder Fluorenyl und worin Aryl unsubstituiert oder unabhängig voneinander ein-, zwei- oder dreifach durch R15 substituiert ist, oder
4) Het, wobei Het ausgewählt ist aus der Gruppe Acridinyl, Azepinyl, Azetidinyl, Aziridinyl, Benzimidazalinyl, Benzimidazolyl, Benzo[1,3]dioxolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chinazolinyl, Chinolinyl, 4H-Chinolizinyl, Chinoxalinyl, Chinuclidinyl,
Chromanyl, Chromenyl, Cinnolinyl, Deca-hydrochinolinyl, Dibenzofuranyl, Dibenzothiophenyl, Dihydrofuran[2,3-b]-tetrahydrofuranyl, Dihydrofuranyl, Dioxolyl, Dioxanyl, 2H, 6H-1,5,2-Dithiazinyl, Furanyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolidinyl, 2-Isothiazolinyl, Isothiazolyl, Isoxazolyl, Isoxazolidinyl, 2-Isoxazolinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Oxothiolanyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, Tetrahydropyridinyl, 6H-1,2,5-Thiadazinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienoimidazolyl, Thienooxazolyl, Thienopyridin, Thienothiazolyl, Thiomorpholinyl, Thiophenyl, Triazinyl, 1,2,3-Triazolyl, 1,2,3-Triazolyl, 1,2,4-Triazolyl, 1,2,5-Triazolyl, 1,3,4-Triazolyl und Xanthenyl, und
worin Het unsubstituiert oder unabhängig voneinander ein-, zwei- oder dreifach durch ein -(C₁-C₃)-Alkyl, Halogen, -NH₂, -CF₃ oder -O-CF₃ substituiert ist, steht,
R1 für
1) Wasserstoffatom oder
2) -((C₁-C₄)-Alkyl, steht,
R3 für
1) -(C₂-C₆)-Alkylen-NH₂, wobei Alkylen unsubstituiert ist oder ein-, zwei-, drei- oder vierfach durch Halogen substituiert ist,
2) -(C₁-C₄)-Alkylen-SO₂-(C₁-C₄)-Alkylen-NH₂ oder
3) -(C₀-C₄)-Alkylen-Het, wobei Het wie oben definiert ist und durch-NH₂ und ein-, zwei- oder dreifach durch R15 substituiert ist, steht
R6 für
1) Wasserstoffatom,
2) -(C₁-C₆)-Alkyl, wobei Alkyl unsubstituiert oder unabhängig voneinander ein-, zwei- oder dreifach durch R16 substituiert ist,
3) -O-(C₁-C₆)-Alkyl, wobei Alkyl unsubstituiert oder unabhängig voneinander ein-, zwei- oder dreifach durch R16 substituiert ist,
4) -(C₀-C₄)-Alkylen-Het, wobei Het wie oben definiert ist, wobei Alkylen und Het unsubstituiert oder unabhängig voneinander ein-, zwei- oder dreifach durch R16 substituiert sind,
5) -(C₀-C₄)-Alkylen-Aryl, wobei Alkylen und Aryl unsubstituiert oder unabhängig voneinander ein-, zwei- oder dreifach durch R16 substituiert sind, oder
6) -(C₀-C₄)-Alkylen-(C₃-C₆)-Cycloalkyl, wobei Alkylen und Cycloalkyl unsubstituiert oder unabhängig voneinander ein-, zwei- oder dreifach durch R16 substituiert sind, steht,
R7 für Wasserstoffatom, F oder -(C₁-C₄)-Alkyl steht,
R8 für Wasserstoffatom, F oder -(C₁-C₄)-Alkyl steht,
R9 für Wasserstoffatom, F oder -(C₁-C₄)-Alkyl steht,
R15 für Wasserstoffatom, -(C₁-C₄)-Alkyl, -O-CF₃, -NH₂, -OH, -CF₃ oder Halogen steht, und
R16 für -O-CF₃, -OH, -CF₃ oder F steht.

4. Verbindung der Formel I gemäß der Ansprüche 1 oder 2, wobei
X für -C(O)- steht,
U für Sauerstoffatom steht,
A für Sauerstoffatom oder -(C₀-C₄)-Alkylen- steht,
V für
1) -(C₂-C₈)-Alkylen-, wobei Alkylen unsubstituiert oder unabhängig voneinander ein- oder zweifach durch -OH, F oder Cl substituiert ist,
2) -(C₁-C₂)-Alkylen-Cyclopropyl-(C₁-C₂)-Alkylen-, wobei Cyclopropyl ein- oder zweifach durch F substituiert ist, oder
3) -(C₃-C₆)-Alkenylen- steht,
D für -(C₁-C₂)-Alkylen- steht,
Y für
1) kovalente Bindung oder
2) Phenyl, worin Phenyl unsubstituiert oder unabhängig voneinander ein-, zwei- oder dreifach durch R15 substituiert ist, steht,
R1 für
1) Wasserstoffatom oder
2) -(C₁-C₄)-Alkyl, steht,
R3 für
1) -(C₂-C₆)-Alkylen-NH₂,
2) -(C₁-C₄)-Alkylen-SO₂-(C₁-C₄)-Alkylen-NH₂ oder
3) -(C₀-C₄)-Alkylen-Pyridyl, wobei Pyridyl durch -NH₂ und ein-, zwei- oder dreifach durch R15 substituiert ist, steht
R6 für
1) Wasserstoffatom,
2) -(C₁-C₆)-Alkyl,
3) -CF3,
4) -(C₀-C₄)-Alkylen-Phenyl oder
5) -(C₀-C₄)-Alkylen-(C₃-C₆)-Cycloalkyl steht,
R7, R8 und R9 jeweils für Wasserstoffatom stehen und
R15 für Wasserstoffatom, -(C₁-C₄)-Alkyl, -CF₃ oder Halogen steht.

5. Verbindung der Formel I gemäß der Ansprüche 1 bis 4, wobei
X für -C(O)- steht,
U für Sauerstoffatom steht,
A für Sauerstoffatom oder -(C₀-C₄)-Alkylen- steht,
V für -(C₂-C₈)-Alkylen- oder -(C₃-C₆)-Alkenylen- steht,
D für -(C₁-C₂)-Alkylen- steht,
Y für
1) kovalente Bindung oder
2) Phenyl, worin Phenyl unsubstituiert oder unabhängig voneinander ein-, zwei- oder dreifach durch R15 substituiert ist, steht,
R1 für
1) Wasserstoffatom oder
2) -(C₁-C₄)-Alkyl, steht,
R3 für
1) -(C₂-C₆)-Alkylen-NH₂,
2) -(C₁-C₄)-Alkylen-SO₂-(C₁-C₄)-Alkylen-NH₂ oder
3) -(C₀-C₄)-Alkylen-Pyridyl, wobei Pyridyl durch -NH₂ und ein-, zwei- oder dreifach durch R15 substituiert ist, steht
R6 für
1) Wasserstoffatom,
2) -(C₁-C₆)-Alkyl,
3) -CF₃,
4) -(C₀-C₄)-Alkylen-Phenyl oder
5) -(C₀-C₄)-Alkylen-(C₃-C₆)-Cycloalkyl steht,
R7, R8 und R9 jeweils für Wasserstoffatom stehen und
R15 für Wasserstoffatom, -(C₁-C₄)-Alkyl, -CF₃ oder Halogen steht.

6. Verbindung der Formel I gemäß einem oder mehreren der Ansprüche 1 bis 5, **dadurch gekennzeichnet, dass** es die Verbindung der Formel I
(S)-6-Amino-2-[3-((9S,12R)-9-isopropyl-11-oxo-2,7-dioxa-10-aza-bicyclo[12.2.2] octadeca-1(17),14(18),15-trien-12-yl)-ureido]-hexansäure, (S)-6-Amino-2-[3-((R)-11-oxo-2,7-dioxa-10-azabicyclo[12.2.2]octadeca-1(17),14(18),15-trien12-yl)-ureido]-hexansäure, (S)-6-Amino-2-[3-((R)-9,9-dimethyl-11-oxo-2,7-dioxa-10-aza-bicyclo[12.2.2]octadeca-1(17), 14(18), 15-trien-12-yl)-ureido]-hexansäure,
(S)-6-Amino-2-[3-((9S,12R)-11-oxo-9-phenyl-2,7-dioxa-10-aza-bicyclo[12.2.2]octadeca-1(17),14(18),15-trien-12-yl)-ureido]-hexansäure, (S)-6-Amino-2-[3-((9S,12R)-9-benzyl-11-oxo-2,7-dioxa-10-aza-bicyclo[12.2.2]octadeca-1(17),14(18),15-trien-12-yl)-ureido]-hexansäure, (S)-6-Amino-2-[3-((9S,12R)-9-cyclohexyl-11-oxo-2,7-dioxa-10-aza-bicyclo[12.2.2]octadeca-1(17),14(18),15-trien-12-yl)-ureido]-hexansäure, (S)-6-Amino-2-[3-((9S,12R)-9-ethyl-11-oxo-2,7-dioxa-10-aza-bicyclo[12.2.2] octadeca-1 (17),14(18),15-trien-12-yl)-ureido]-hexansäure, (S)-6-Amino-2-[3-((9S,12R)-9-methyl-11-oxo-2,7-dioxa-10-aza-bicyclo[12.2.2]octadeca-1(17),14(18),15-trien-12-yl)-ureido]-hexansäure, (S)-6-Amino-2-[3-((9S,12R)-9-tert-butyl-11-oxo-2,7-dioxa-10-aza-bicyclo[12.2.2]octadeca-1(17),14(18),15-trien-12-yl)-ureido]-hexansäure, (S)-6-Amino-2-[3-((13S,16R)-13-isopropyl-15-oxo-2,11-dioxa-14-aza-bicyclo[16.2.2] docosa-1(21), 18(22), 19-trien-16-yl)-ureido]-hexansäure, (S)-6-Amino-2-[3-((E)-(9S,12R)-9-isopropyl-11-oxo-2,7-dioxa-10-aza-bicyclo[12.2.2]octadeca-1(17),4,14(18),15-tetraen-12-yl)-ureido]-hexansäure, (S)-6-Amino-2-[3-((3S,6R)-3-isopropyl-5-oxo-1-oxa-4-aza-cyclotetradec-6-yl)-ureido]-hexansäure, (R)-3-(2-Amino-ethansulfonyl)-2-[3-((9S,12R)-9-isopropyl-11-oxo-2,7-dioxa-10-aza-bicyclo[12.2.2]octadeca-1(17),14(18),15-trien-12-yl)-ureido]-propionsäure, (S)-3-(6-Amino-pyridin-3-yl)-2-[3-((9S,12R)-9-isopropyl-11-oxo-2,7-dioxa-10-aza-bicyclo[12.2.2]octadeca-1 (17),14(18),15-trien-12-yl)-ureido]-propionsäure, (S)-6-Amino-2-{3-[(9S,12R)-9-((S)-sec-butyl)-11-oxo-2,7-dioxa-10-aza-bicyclo[12.2.2]octadeca-1(17), 14(18),15-trien-12-yl]-ureido}-hexansäure, (S)-3-(6-Amino-pyridin-3-yl)-2-[3-((3S,6R)-3-isopropyl-5-oxo-1-oxa-4-aza-cyclotetradec-6-yl)-ureido]-propionsäure, (S)-3-(6-Amino-pyridin-3-yl)-2-[3-((3S,6R)-3-methyl-5-oxo-1-oxa-4-aza-cyclotetradec-6-yl)-ureido]-propionsäure, (S)-3-(6-Amino-pyridin-3-yl)-2-[3-((3S,6R)-3-isopropyl-5-oxo-1-oxa-4-aza-cyclotetradec-6-yl)-ureido]-propionsäureethylester, (S)-3-(6-Amino-pyridin-3-yl)-2-[3-((3S,6R)-3-methyl-5-oxo-1-oxa-4-aza-cyclotetradec-6-yl)-ureido]-propionsäureethylester, (S)-3-(6-Amino-pyridin-3-yl)-2-[3-((9S, 12R)-9-cyclopropyl-11-oxo-2,7-dioxa-10-aza-bicyclo[12.2.2]octadeca-1(17),14(18),15-trien-12-yl)-ureido]-propionsäure, (S)-6-Amino-2-[3-((9S, 12R)-9-isopropyl-11-oxo-2,7-dioxa-10-aza-bicyclo[12.2.2]octadeca-1(17),14(18),15-trien-12-yl)-ureido]-6-methyl-heptansäure, (S)-3-(6-Amino-pyridin-3-yl)-2-[3-((9S,12R)-9-isopropyl-11-oxo-2,7-dioxa-10-aza-bicyclo[12.2.2]octadeca-1-(17),14(18),15-trien-12-yl)-ureido]-propionsäureethylester,
(S)-2-[3-((9S, 12R)-9-Isopropyl-11-oxo-2,7-dioxa-10-aza-bicyclo[12.2.2] octadeca-1(17), 14(18), 15-trien-12-yl)-ureido]-3-piperidin-3-yl-propionsäure,
(S)-3-(6-Amino-pyridin-3-yl)-2-[3-((9S,12R)-9-methyl-11-oxo-2,7-dioxa-10-aza-bicyclo[12.2.2]octadeca-1(17), 14(18), 15-trien-12-yl)-ureido]-propionsäure,
(S)-2-[3-((9S,12R)-9-Isopropyl-11-oxo-2,7-dioxa-10-aza-bicyclo[12.2.2] octadeca-1(17),14(18),15-trien-12-yl)-ureido]-3-piperidin-3-yl-propionsäure,
(S)-3-(6-Amino-pyridin-3-yl)-2-[3-((8S, 11R)-8-isopropyl-10-oxo-6-oxa-1,9,14-triaza-bicyclo[11.2.1]hexadeca-13(16),14-dien-11-yl)-ureido]-propionsäure,
(S)-3-(6-Amino-pyridin-3-yl)-2-[3-((3R,6S)-6-isopropyl-4-oxo-8-oxa-5-azabicyclo[11.2.2]heptadeca-1(16),13(17),14-trien-3-yl)ureido]-propionsäure,
(S)-3-(6-Amino-pyridin-3-yl)-2-[3-((9S, 12R)-16-fluoro-9-isopropyl-11-oxo-2,7-dioxa-10-aza-bicyclo[12.2.2]octadeca-1(17),14(18),15-trien-12-yl)-ureido]-propionsäure,
(S)-3-(6-Amino-pyridin-3-yl)-2-[3-((3S,6R)-3-isopropyl-5-oxo-1,8-dioxa-4-aza-cyclododec-6-yl)-ureido]-propionsäure,
(S)-3-(6-Amino-pyridin-3-yl)-2-[3-((3S,6R)-3-isopropyl-5-oxo-1,8-dioxa-4-aza-cyclododec-6-yl)-ureido]-propionsäuremethylester,
(S)-6-Amino-2-[3-((3S,6R)-3-isopropyl-5-oxo-1,8-dioxa-4-aza-cyclododec-6-yl)-ureido]-hexansäure,
(S)-6-Amino-2-[3-((9S,12S)-9-isopropyl-11-oxo-2,7-dioxa-10-aza-bicyclo-[12.2.2]octadec-12-yl)-ureido]-hexansäure,
(S)-6-Amino-2-((8S,11R)-8-isopropyl-3,10-dioxo-6-oxa-2,9-diaza-bicyclo[11.2.2]heptadeca-1(16),13(17),14-trien-11-ylamino)-hexansäure,
S)-3-(6-Amino-pyridin-3-yl)-2-[3-((8S,11R)-8-methyl-3,10-dioxo-6-oxa-2,9-diaza-bicyclo[11.2.2]heptadeca-1(16),13(17),14-trien-11-yl)-ureido]-propionic acid,
(S)-3-(6-Amino-pyridin-3-yl)-2-[3-((3S,6R)-3-isopropyl-5,9-dioxo-1-oxa-4,10-diaza-cyclotridec-6-yl)-ureido]-propionic acid,
(S)-3-(6-Amino-pyridin-3-yl)-2-[3-((8S,11R)-8-isopropyl-2,10-dioxo-6-oxa-3,9-diaza-bicyclo[11.2.2]heptadeca-1(16),13(17),14-trien-11-yl)-ureido]-propionsäure,
(S)-6-Amino-2-[3-((9S,12R)-4,5-dihydroxy-9-isopropyl-11-oxo-2,7-dioxa-10-aza-bicyclo[12.2.2]octadeca-1(17),14(18),15-trien-12-yl)-ureido]-hexansäure,
(S)-6-Amino-2-[3-((9S,12R)-5-hydroxy-9-isopropyl-11-oxo-2,7-dioxa-10-aza-bicyclo[12.2.2]octadeca-1(17),14(18),15-trien-12-yl)-ureido]-hexansäure, 3-(6-Amino-pyridin-3-yl)-2-[3-((9S,12R)-4,5-dihydroxy-9-isopropyl-11-oxo-2,7-dioxa-10-aza-bicyclo[12.2.2]octadeca-1(17),14(18),15-trien-12-yl)-ureido]-propionsäure,
(S)-6-Amino-2-[3-((9S,12R)-4-hydroxy-9-isopropyl-11-oxo-2,7-dioxa-10-aza-bicyclo[12.2.2]octadeca-1(17),14(18),15-trien-12-yl)-ureido]-hexansäure oder (S)-6-Amino-2-[((9S,12R)-9-isopropyl-11-oxo-2,7-dioxa-10-aza-bicyclo[12.2.2] octadeca-1(17),14(18),15-trien-12-ylsulfamoyl)-amino]-hexansäure ist.

7. Verfahren zur Herstellung der Verbindung der Formel I gemäß einem oder mehreren der Ansprüche 1 bis 6, **dadurch gekennzeichnet, dass** man
a) eine Verbindung der Formel (II) 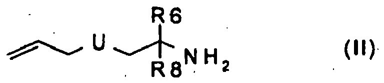 wobei U, R6 und R8 die in der Verbindung der Formel (I) genannten Bedeutungen haben mit einer Aminosäure der Formel (III) umsetzt, 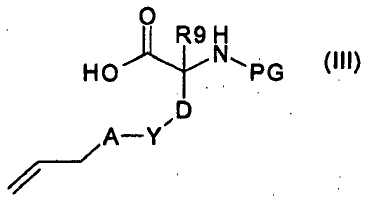 worin R9, A, Y und D die in der Verbindung der Formel (I) genannten Bedeutungen haben, wobei eine Verbindung der Formel (IV) erhalten wird, 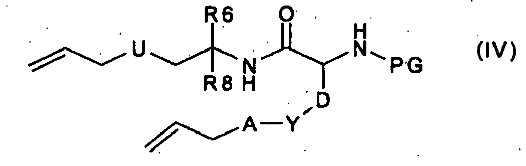 welche unter den Bedingungen der Ringschlußmetathese und anschließender Hydrierung der entstandenen Doppelbindung zu einer Verbindung der Formel (V) umgesetzt wird, 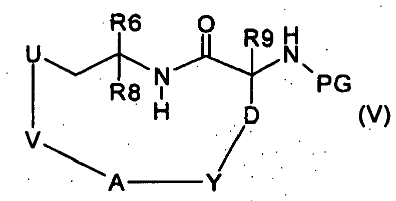 wobei V für -(C₂-C₉)-Alkylen- oder -(C₃-C₉)-Alkenylen- steht, anschließend die Schutzgruppe PG abspaltet und die Verbindung der Formel (VI) erhält, 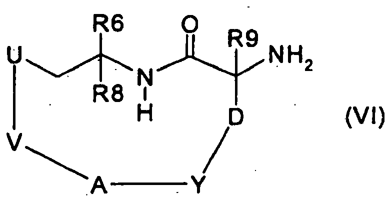 und mit einer Verbindung der Formel (VII) 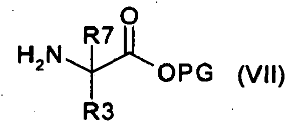 worin R3 und R7 die in Formel (I) genannten Bedeutungen haben, PG einen geeigneten Esterschutzgruppenrest darstellt, sowie der Stickstoff in R3 gegebenfalls durch eine geeignete Aminoschutzgruppe geschützt ist, mit Phosgen oder eines Phosgenäquivalents zu einer Verbindung der Formel (VIII) 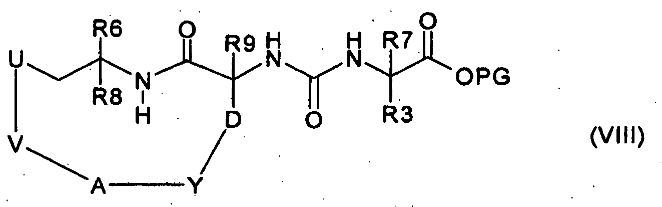 umsetzt, anschließend werden die Schutzgruppe PG und die gegebenenfalls vorhandene Schutzgruppe am Stickstoff in R3 abgespalten, wobei die Verbindung der Formel (I) erhalten wird, oder
b) eine Verbindung der Formel (IX) 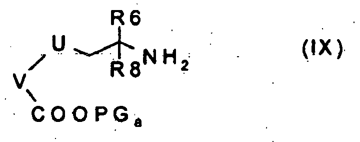 worin U, V, R6 und R8 die in der Verbindung der Formel (I) genannten Bedeutungen haben und PGₐ eine geeignete Carboxylschutzgruppe darstellt, mit einer Aminosäure der Formel (X) umsetzt, 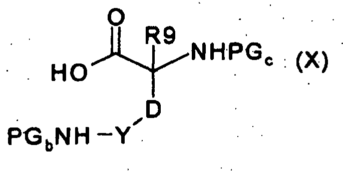 worin R9, Y und D die in der Verbindung der Formel I genannten Bedeutungen haben und PG_{b} und PG_{c} geeignete Aminoschutzgruppen sind, wobei eine Verbindung der Formel (XI) erhalten wird, 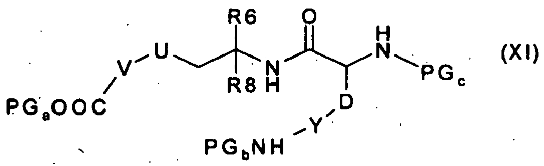 welche nach Abspaltung der Schutzgruppen PGₐ und PG_{b} in die Verbindung der Formel (XII) überführt wird, 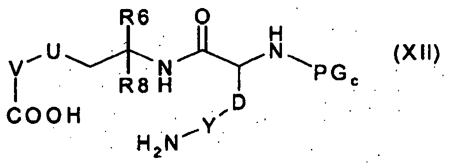 welche mit Hilfe einer Amidkupplung zu einer Verbindung der Formel (V) umgesetzt wird,
worin A die in der Verbindung der Formel I genannten Bedeutungen hat, anschließend wird die Scutzgruppe abgespalten und die Verbindung der Formel (VI) erhalten,
und mit einer Verbindung der Formel (VII)
worin R3 und R7 die in Formel I genannten Bedeutungen haben, PG einen geeigneten Esterschutzgruppenrest darstellt, sowie der Stickstoff in R3 gegebenfalls durch eine geeignete Aminoschutzgruppe geschützt ist, mit Phosgen oder eines Phosgenäquivalents zu einer Verbindung der Formel (VIII) umgesetzt, anschließend werden die Schutzgruppe PG und die gegebenenfalls vorhandene Schutzgruppe am Stickstoff in R3 abgespalten, wobei die Verbindung der Formel (I) erhalten wird, oder
c) eine Verbindung der Formel (XIII) 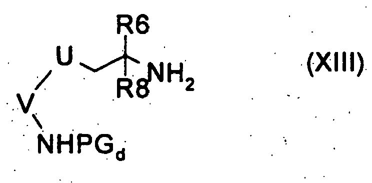 worin U, V, R6 und R8 die in der Verbindung der Formel I genannten Bedeutungen haben und PG_{d} eine geeignete Aminoschutzgruppe darstellt, mit einer Aminosäure der Formel (XIV) umsetzt, 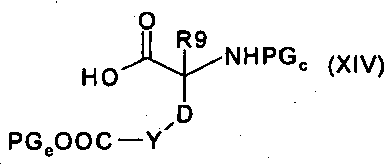 worin R9, Y und D die in der Verbindung der Formel I genannten Bedeutungen haben und PG_{c} eine geeignete Aminoschutzgruppe und PGₑ eine geeignete Carboxylschutzgruppe ist, wobei eine Verbindung der Formel (XV) erhalten wird, 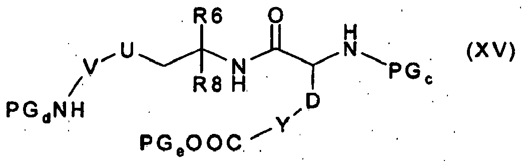 welche nach Abspaltung der Schutzgruppen PG_{d} und PGₑ in die Verbindung der Formel (XVI) überführt wird, 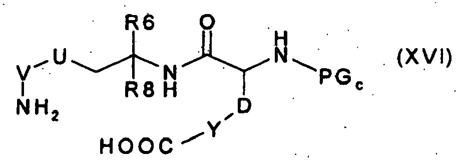 welche zu einer Verbindung der Formel (V) umgesetzt wird,
worin A die in der Verbindung der Formel I genannten Bedeutungen hat, anschließend wird die Schutzgruppe abgespalten und die Verbindung der Formel (VI) wird erhalten,
und mit einer Verbindung der Formel (VII),
worin R3 und R7 die in Formel I genannten Bedeutungen haben, PG einen geeigneten Esterschutzgruppenrest darstellt, sowie der Stickstoff in R3 gegebenfalls durch eine geeignete Aminoschutzgruppe geschützt ist, mit Phosgen oder eines Phosgenäquivalents zu einer Verbindung der Formel (VIII) umgesetzt, anschließend werden die Schutzgruppe PG und die gegebenenfalls vorhandene Schutzgruppe am Stickstoff in R3 abgespalten, wobei die Verbindung der Formel (I) erhalten wird, oder
d) eine Verbindung der Formel XVII, 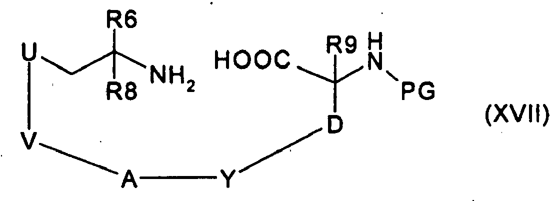 worin U, V, A, Y, D, R₆, R₈ und R₉ die in der Verbindung der Formel I genannten Bedeutungen haben, mit einer Verbindung der Formel (V) umsetzt, anschließend werden die Schutzgruppen abgespalten und eine Verbindung der Formel (VI) wird erhalten,
und mit einer Verbindung der Formel (VII),
worin R3 und R7 die in Formel I genannten Bedeutungen haben, PG einen geeigneten Esterschutzgruppenrest darstellt, sowie der Stickstoff in R3 gegebenfalls durch eine geeignete Aminoschutzgruppe geschützt ist, mit Phosgen oder eines Phosgenäquivalents zu einer Verbindung der Formel (VIII) umgesetzt, und anschließend werden die Schutzgruppen PG und gegebenenfalls die Schutzgruppe am Stickstoff in R3 abgespalten, wobei die Verbindung der Formel (I) erhalten wird, oder
e) eine Verbindung der Formel (VIIIa) 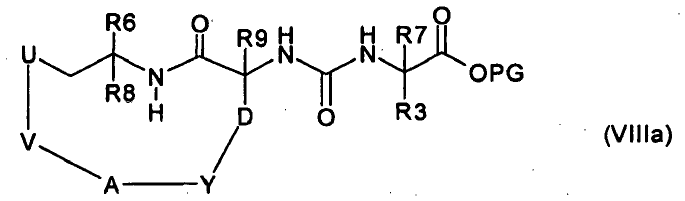 worin V für -(C₃-C₉)-Alkenylen- steht in die Verbindung der Formel (VIIIb) umwandelt, 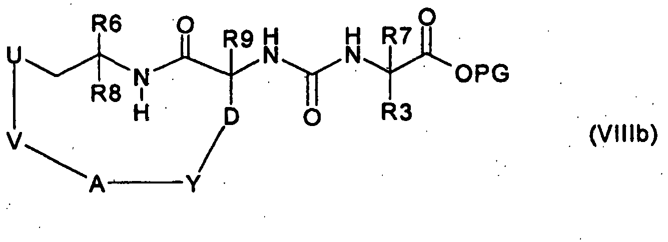 worin V für -(C₃-C₉)-Alkylen- steht, wobei Alkylen unabhängig voneinander ein-, zwei- oder dreifach durch -OH, NH₂ oder Halogen substituiert ist, oder für -(C₁-C₂)-Alkylen-(C₃-C₆)-Cycloalkyl-(C₁-C₂)-Alkylen- steht, wobei Cycloalkyl unabhängig voneinander ein-, zwei- oder dreifach durch R15 substituiert ist, anschließend wird die Verbindung der Formel (VIIIb) analog zu Verfahren a) in die Verbindung der Formel (I) umgesetzt, oder
f) eine Verbindung der Formel (XVIII) 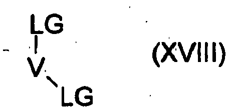 worin V wie in der Verbindung der Formel (I) definiert ist, nacheinander mit den Verbindungen der Formel (XIX) und (XX) 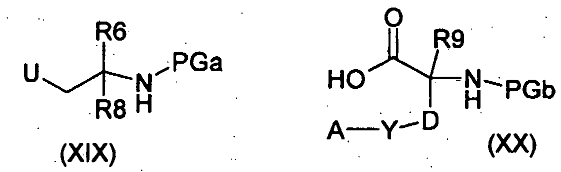 unter Einsatz von Basen in polaren, aprotischen Lösungsmitteln umsetzt und die erhaltenen Verbindungen der Formel (XXI) 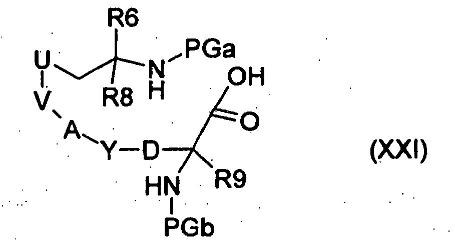 durch Entfernen der Schutzgruppe PGa und anschließender Bildung einer Peptidbindung in eine Verbindung der Formel (V) umwandelt, und diese wie in Verfahren a) zu Verbindungen der Formel (I) umsetzt, worin R6, R8, R9 sowie A, D, U, V und Y die in Formel (I) genannten Bedeutungen haben und PG geeignete Schutzgruppen darstellt sowie LG für eine Abgangsgruppe wie Chlor, Brom, lod oder Sulfonsäureester steht, oder
g) eine Verbindung der Formel VI mit einer Verbindung der Formel (XXII), 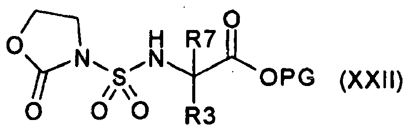 worin R3 und R7 die in der Verbindung der Formel I genannten Bedeutungen haben und PG einen geeigneten Schutzgruppenrest darstellt, zu einer Verbindung der Formel (XXIII) umsetzt, 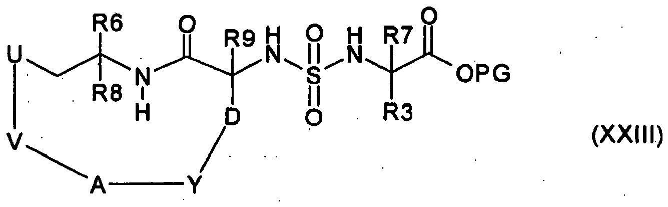 und dann in eine Verbindung der Formel (I) überführt, oder
h) eine nach den Verfahren a), b), c), d), e), f) oder g) hergestellte Verbindung der Formel I, oder eine geeignete Vorstufe der Formel (I), die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
i) die nach den Verfahren a), b), c), d), e), f) oder g) hergestellte Verbindung der Formel (I) entweder in freier Form isoliert oder im Falle des Vorliegens von sauren oder basischen Gruppen in physiologisch verträgliche Salze umwandelt.

8. Arzneimittel, **gekennzeichnet durch** einen wirksamen Gehalt an mindestens einer Verbindung der Formel I gemäß einem oder mehreren der Ansprüche 1 bis 6 zusammen mit einem pharmazeutisch geeigneten und physiologisch verträglichen Trägerstoff, Zusatzstoff und/oder anderen Wirk- und Hilfsstoffen.

9. Verbindung der Formel I gemäß einem oder mehreren der Ansprüche 1 bis 6 für die Prophylaxe, Sekundärprevention und Therapie einer oder mehrerer Erkrankungen, die mit Thrombosen, Embolien, Hyperkoagulabilität oder fibrotischen Veränderungen einhergehen.

10. Verbindung gemäß Anspruch 9, **dadurch gekennzeichnet, dass** es sich um eine oder mehrere Erkrankungen aus der Reihe Myokardinfarkt, Angina pectoris und andere Formen des akuten Koronarsyndroms, den Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung und Angioplastie und ähnlichen Eingriffen wie Stentimplantationen und Bypass-Operationen handelt, oder die Reduktion der Thrombosegefahr nach chirurgischen Eingriffen wie bei Knie- und Hüftgelenksoperationen, oder um die disseminierte intravaskuläre Koagulation, Sepsis und anderen intravaskulären Ereignissen, die mit einer Entzündung einhergehen, Atherosklerose, Diabetes und dem metabolischen Syndrom und deren Folgen, Tumorwachstum und Tumormetastasierung, entzündliche und degenerative Gelenkserkrankungen wie der rheumatoiden Arthritis und der Arthrose, Störungen des hämostatischen Systems wie Fibrinablagerungen, fibrotische Veränderungen der Lunge wie die chronische obstruktive Lungenerkrankung, das adult respiratory distress syndrom oder Fibrinablagerungen des Auges nach Augenoperationen oder Verhinderung oder Behandlung von Narbenbildung handelt.

## Claims

1. A compound of the formula I 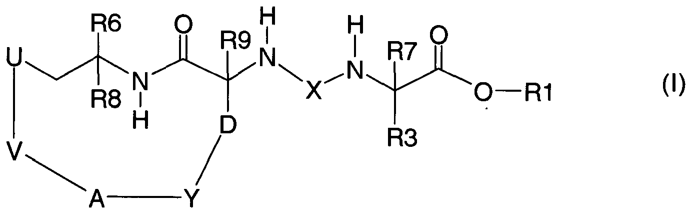 and/or a stereoisomeric form of the compound of the formula I and/or mixtures of these forms in any ratio, and/or a physiologically tolerated salt of the compound of the formula I, where
X is -C(O)- or -SO₂-,
U is oxygen atom, sulfur atom, NH, -C(O)-NH- or -(C₀-C₄)-alkylene-,
A is oxygen atom, sulfur atom, NH, -C(O)-NH- -NH-C(O)-, -NR2- or -(C₀-C₄)-alkylene-,
V is
1) -(C₂-C₉)-alkylene-, where alkylene is unsubstituted or substituted independently of one another once, twice or three times by -OH, NH₂ or halogen,
2) -(C₁-C₂)-alkylene-(C₃-C₆)-cycloalkyl-(C₁-C₂)-alkylene-, where cycloalkyl is substituted independently of one another once, twice or three times by R15, or
3) -(C₃-C₉)-alkenylene-,
D is -(C₁-C₂)-alkylene-,
Y is
1) covalent bond,
2) -(C₃-C₁₂)-cycloalkyl, where cycloalkyl is substituted independently of one another once, twice or three times by R15,
3) -(C₆-C₁₄)-aryl, where aryl is unsubstituted or substituted independently of one another once, twice or three times by R15, or
4) Het, where Het means a 4- to 15-membered heterocyclic ring system having 4 to 15 ring atoms which are present in one, two or three ring systems which are connected together, and which comprise, depending on the ring size, one, two, three or four identical or different heteroatoms from the series oxygen, nitrogen or sulfur, and
in which Het is unsubstituted or substituted independently of one another once, twice or three times by a -(C₁-C₃)-alkyl, halogen, -NH₂,-CF₃ or -O-CF₃,
R1 is
1) hydrogen atom,
2) -(C₁-C₆)-alkyl,
3) -(C₁-C₆)-alkyl-OH,
4) -(C₀-C₄)-alkyl-(C₃-C₆)-cycloalkyl,
5) -(C₁-C₁₀)-alkyl-O-C(O)-O-R2,
6) -(CH₂)ᵣ-(C₆-C₁₄)-aryl, in which aryl is unsubstituted or substituted independently of one another once, twice or three times by R15, and r is the integer zero, 1, 2 or 3, or
7) -(CH₂)ₛ-Het, where Het means a 4- to 15-membered heterocyclic ring system having 4 to 15 ring atoms which are present in one, two or three ring systems which are connected together, and which comprise, depending on the ring size, one, two, three or four identical or different heteroatoms from the series oxygen, nitrogen or sulfur, and in which Het is unsubstituted or substituted independently of one another once, twice or three times by R15, and s is the integer zero, 1, 2 or 3,
R2 is
1) -(C₁-C₆)-alkyl,
2) -(CH₂)ᵣ-(C₆-C₁₄)-aryl, in which aryl is unsubstituted or substituted independently of one another once, twice or three times by R15, and r is the integer zero, 1, 2 or 3, or
3) -(C₀-C₄)-alkyl-(C₃-C₆)-cycloalkyl,
R3 is
1) -(C₂-C₆)-alkylene-NH₂ , where alkylene is unsubstituted or substituted once, twice, three or four times by halogen,
2) -(C₁-C₄)-alkylene-O-(C₁-C₄)-alkylene-NH₂,
3) -(C₁-C₄)-alkylene-SO₂-(C₁-C₄)-alkylene-NH₂,
4) -(C₀-C₄)-alkylene-Het, where Het is as defined above and is substituted by -NH₂ and once, twice or three times by R15,
5) -(C₀-C₄)-alkylene-(C₃-C₈)-cycloalkyl-NH₂ or
6) -(C₀-C₆)-alkylene-cyclic amine,
R6 is
1) hydrogen atom,
2) -(C₁-C₆)-alkyl, where alkyl is unsubstituted or substituted independently of one another once, twice or three times by R16,
3) -O-(C₁-C₆)-alkyl, where alkyl is unsubstituted or substituted independently of one another once, twice or three times by R16,
4) -(C₀-C₄)-alkylene-Het, where Het is as defined above, where alkylene and Het are unsubstituted or substituted independently of one another once, twice or three times by R16,
5) -(C₀-C₄)-alkylene-aryl, where alkylene and aryl are unsubstituted or substituted independently of one another once, twice or three times by R16, or
6) -(C₀-C₄)-alkylene-(C₃-C₈)-cycloalkyl, where alkylene and cycloalkyl are unsubstituted or substituted independently of one another once, twice or three times by R16,
R7 is hydrogen atom, halogen or -(C₁-C₆)-alkyl,
R8 is hydrogen atom, halogen or -(C₁-C₆)-alkyl,
R9 is hydrogen atom, halogen or -(C₁-C₆)-alkyl,
R15 is hydrogen atom, -(C₁-C₄)-alkyl, -O-CF₃, -NH₂, -OH, -CF₃ or halogen, and
R16 is -O-CF₃, -NH₂, -OH, -CF₃ or halogen.

2. A compound of the formula I as claimed in claim 1, where
X is -C(O)- or -SO₂-,
U is oxygen atom, sulfur atom, NH, -C(O)-NH- or -(C₀-C₄)-alkylene-,
A is oxygen atom, sulfur atom, NH, -C(O)-NH- or -(C₀-C₄)-alkylene-,
V is
1) -(C₂-C₉)-alkylene- or
2) -(C₃-C₉)-alkenylene-,
D is -(C₁-C₂)-alkylene-,
Y is
1) covalent bond,
2) -(C₃-C₁₂)-cycloalkyl, where cycloalkyl is substituted independently of one another once, twice or three times by R15,
3) -(C₆-C₁₄)-aryl, where aryl is unsubstituted or substituted independently of one another once, twice or three times by R15, or
4) Het, where Het means a 4- to 15-membered heterocyclic ring system having 4 to 15 ring atoms, which are present in one, two or three ring systems which are connected together, and which comprise, depending on the ring size, one, two, three or four identical or different heteroatoms from the series oxygen, nitrogen or sulfur, and
in which Het is unsubstituted or substituted independently of one another once, twice or three times by a -(C₁-C₆)-alkyl, halogen, -NH₂, -CF₃ or -O-CF₃,
R1 is
1) hydrogen atom,
2) -(C₁-C₆)-alkyl,
3) -(C₁-C₆)-alkyl-OH,
4) -(C₀-C₄)-alkyl-(C₃-C₆)-cycloalkyl,
5) -(C₁-C₁₀)-alkyl-O-C(O)-O-R2,
6) -(CH₂)ᵣ-(C₆-C₁₄)-aryl, in which aryl is unsubstituted or substituted independently of one another once, twice or three times by R15, and r is the integer zero, 1, 2 or 3, or
7) -(CH₂)ₛ-Het, where Het means a 4- to 15-membered heterocyclic ring system having 4 to 15 ring atoms which are present in one, two or three ring systems which are connected together, and which comprise, depending on the ring size, one, two, three or four identical or different heteroatoms from the series oxygen, nitrogen or sulfur, and in which Het is unsubstituted or substituted independently of one another once, twice or three times by R15, and s is the integer zero, 1, 2 or 3,
R2 is
1) -(C₁-C₆)-alkyl,
2) -(CH₂)ᵣ-(C₆-C₁₄)-aryl, in which aryl is unsubstituted or substituted independently of one another once, twice or three times by R15, and r is the integer zero, 1, 2 or 3, or
3) -(C₀-C₄)-alkyl-(C₃-C₆)-cycloalkyl,
R3 is
1) -(C₂-C₆)-alkylene-NH₂ , where alkylene is unsubstituted or substituted once, twice, three or four times by halogen,
2) -(C₁-C₄)-alkylene-O-(C₁-C₄)-alkylene-NH₂,
3) -(C₁-C₄)-alkylene-SO₂-(C₁-C₄)-alkylene-NH₂,
4) -(C₀-C₄)-alkylene-Het, where Het is as defined above and is substituted by -NH₂ and once, twice or three times by R15,
5) -(C₀-C₄)-alkylene-(C₃-C₈)-cycloalkyl-NH₂ or
6) -(C₀-C₆)-alkylene-cyclic amine,
R6 is
1) hydrogen atom,
2) -(C₁-C₆)-alkyl, where alkyl is unsubstituted or substituted independently of one another once, twice or three times by R16,
3) -O-(C₁-C₆)-alkyl, where alkyl is unsubstituted or substituted independently of one another once, twice or three times by R16,
4) -(C₀-C₄)-alkylene-Het, where Het is as defined above, where alkylene and Het are unsubstituted or substituted independently of one another once, twice or three times by R16,
5) -(C₀-C₄)-alkylene-aryl, where alkylene and aryl are unsubstituted or substituted independently of one another once, twice or three times by R16, or
6) -(C₀-C₄)-alkylene-(C₃-C₈)-cycloalkyl, where alkylene and cycloalkyl are unsubstituted or substituted independently of one another once, twice or three times by R16,
R7 is hydrogen atom, halogen or -(C₁-C₆)-alkyl,
R8 is hydrogen atom, halogen or -(C₁-C₆)-alkyl,
R9 is hydrogen atom, halogen or -(C₁-C₆)-alkyl,
R15 is hydrogen atom, -(C₁-C₄)-alkyl, -O-CF₃, -NH₂, -OH, -CF₃ or halogen, and
R16 is -O-CF₃, -NH₂, -OH, -CF₃ or halogen.

3. A compound of the formula I as claimed in claim 1 or 2, where
X is -C(O)- or -SO₂-,
U is oxygen atom, sulfur atom, NH, -C(O)-NH- or -(C₀-C₄)-alkylene-,
A is is oxygen atom or -(C₀-C₄)-alkylene-,
V is -(C₂-C₈)-alkylene- or -(C₃-C₆)-alkenylene-,
D is -(C₁-C₂)-alkylene-,
Y is
1) covalent bond,
2) -(C₃-C₆)-cycloalkyl, where cycloalkyl is substituted independently of one another once, twice or three times by R15,
3) -(C₆-C₁₄)-aryl, where aryl is selected from the group of phenyl, naphthyl, anthryl or fluorenyl, and in which aryl is unsubstituted or substituted independently of one another once, twice or three times by R15, or
4) Het, where Het is selected from the group of acridinyl, azepinyl, azetidinyl, aziridinyl, benzimidazalinyl, benzimidazolyl, benzo[1,3]dioxolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, quinazolinyl, quinolinyl, 4H-quinolizinyl, quinoxalinyl, quinuclidinyl, chromanyl, chromenyl, cinnolinyl, deca-hydroquinolinyl, dibenzofuranyl, dibenzothiophenyl, dihydrofuran[2,3-b]-tetrahydrofuranyl, dihydrofuranyl, dioxolyl, dioxanyl, 2H, 6H-1,5,2-dithiazinyl, furanyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl (benzimidazolyl), isothiazolidinyl, 2-isothiazolinyl, isothiazolyl, isoxazolyl, isoxazolidinyl, 2-isoxazolinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, oxothiolanyl, pyrimidinyl, phenanthridinyl, phenanthrolinyl, phenazinyl, phenothiazinyl, phenoxathiinyl, phenoxazinyl, phthalazinyl, piperazinyl, piperidinyl, pteridinyl, purynyl, pyranyl, pyrazinyl, pyroazolidinyl, pyrazolinyl, pyrazolyl, pyridazinyl, pyridooxazolyl, pyridoimidazolyl, pyridothiazolyl, pyridothiophenyl, pyridinyl, pyridyl, pyrimidinyl, pyrrolidinyl, pyrrolinyl, 2H-pyrrolyl, pyrrolyl, tetrahydrofuranyl, tetrahydroisoquinolinyl, tetrahydroquinolinyl, tetrahydropyridinyl, 6H-1,2,5-thiadazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienoimidazolyl, thienooxazolyl, thienopyridine, thienothiazolyl, thiomorpholinyl, thiophenyl, triazinyl, 1,2,3-triazolyl, 1,2,3-triazolyl, 1,2,4-triazolyl, 1,2,5-triazolyl, 1,3,4-triazolyl and xanthenyl, and in which Het is unsubstituted or substituted independently of one another once, twice or three times by a -(C₁-C₃)-alkyl, halogen, -NH₂,-CF₃ or -O-CF₃,
R1 is
1) hydrogen atom or
2) -(C₁-C₄)-alkyl,
R3 is
1) -(C₂-C₆)-alkylene-NH₂, where alkylene is unsubstituted or substituted once, twice, three or four times by halogen,
2) -(C₁-C₄)-alkylene-SO₂-(C₁-C₄)-alkylene-NH₂ or
3) -(C₀-C₄)-alkylene-Het, where Het is as defined above and is substituted by -NH₂ and once, twice or three times by R15,
R6 is
1) hydrogen atom,
2) -(C₁-C₆)-alkyl, where alkyl is unsubstituted or substituted independently of one another once, twice or three times by R16,
3) -O-(C₁-C₆)-alkyl, where alkyl is unsubstituted or substituted independently of one another once, twice or three times by R16,
4) -(C₀-C₄)-alkylene-Het, where Het is as defined above, where alkylene and Het are unsubstituted or substituted independently of one another once, twice or three times by R16,
5) -(C₀-C₄)-alkylene-aryl, where alkylene and aryl are unsubstituted or substituted independently of one another once, twice or three times by R16, or
6) -(C₀-C₄)-alkylene-(C₃-C₆)-cycloalkyl, where alkylene and cycloalkyl are unsubstituted or substituted independently of one another once, twice or three times by R16,
R7 is hydrogen atom, F or -(C₁-C₄)-alkyl,
R8 is hydrogen atom, F or -(C₁-C₄)-alkyl,
R9 is hydrogen atom, F or -(C₁-C₄)-alkyl,
R15 is hydrogen atom, -(C₁-C₄)-alkyl, -O-CF₃, -NH₂, -OH, -CF₃ or halogen, and
R16 is -O-CF₃, -OH, -CF₃ or F.

4. A compound of the formula I as claimed in claim 1 or 2, where
X is -C(O)-,
U is oxygen atom,
A is oxygen atom or -(C₀-C₄)-alkylene-,
V is
1) -(C₂-C₈)-alkylene-, where alkylene is unsubstituted or substituted independently of one another once or twice by -OH, F or Cl,
2) -(C₁-C₂)-alkylene-cyclopropyl-(C₁-C₂)-alkylene-, where cyclopropyl is substituted once or twice by F, or
3) -(C₃-C₆)-alkenylene-,
D is -(C₁-C₂)-alkylene-,
Y is
1) covalent bond or
2) phenyl, in which phenyl is unsubstituted or substituted independently of one another once, twice or three times by R15,
R1 is
1) hydrogen atom or
2) -(C₁-C₄)-alkyl,
R3 is
1) -(C₂-C₆)-alkylene-NH₂,
2) -(C₁-C₄)-alkylene-SO₂-(C₁-C₄)-alkylene-NH₂ or
3) -(C₀-C₄)-alkylene-pyridyl, where pyridyl is substituted by -NH₂ and once, twice or three times by R15,
R6 is
1) hydrogen atom,
2) -(C₁-C₆)-alkyl,
3) -CF₃,
4) -(C₀-C₄)-alkylene-phenyl or
5) -(C₀-C₄)-alkylene-(C₃-C₆)-cycloalkyl,
R7, R8 and R9 are each hydrogen atom, and
R15 is hydrogen atom, -(C₁-C₄)-alkyl, -CF₃ or halogen.

5. A compound of the formula I as claimed in claims 1 to 4, where
X is -C(O)-,
U is oxygen atom,
A is oxygen atom or -(C₀-C₄)-alkylene-,
V is -(C₂-C₈)-alkylene- or -(C₃-C₆)-alkenylene-,
D is -(C₁-C₂)-alkylene-,
Y is
1) covalent bond or
2) phenyl, in which phenyl is unsubstituted or substituted independently of one another once, twice or three times by R15,
R1 is
1) hydrogen atom or
2) -(C₁-C₄)-alkyl,
R3 is
1) -(C₂-C₆)-alkylene-NH₂,
2) -(C₁-C₄)-alkylene-SO₂-(C₁-C₄)-alkylene-NH₂ or
3) -(C₀-C₄)-alkylene-pyridyl, where pyridyl is substituted by -NH₂ and once, twice or three times by R15,
R6 is
1) hydrogen atom,
2) -(C₁-C₆)-alkyl,
3) -CF₃,
4) -(C₀-C₄)-alkylene-phenyl or
5) -(C₀-C₄)-alkylene-(C₃-C₆)-cycloalkyl,
R7, R8 and R9 are each hydrogen atom, and
R15 is hydrogen atom, -(C₁-C₄)-alkyl, -CF₃ or halogen.

6. A compound of the formula I as claimed in one or more of claims 1 to 5, which is the compound of the formula I
(S)-6-amino-2-[3-((9S,12R)-9-isopropyl-11-oxo-2,7-dioxa-10-aza-bicyclo[12.2.2] octadeca-1(17),14(18),15-trien-12-yl)ureido]hexanoic acid, (S)-6-amino-2-[3-((R)-11-oxo-2,7-dioxa-10-azabicyclo[12.2.2]octadeca-1(17),14(18),15-trien12-yl)ureido]hexanoic acid, (S)-6-amino-2-[3-((R)-9,9-dimethyl-11-oxo-2,7-dioxa-10-aza-bicyclo[12.2.2]octadeca-1(17),14(18),15-trien-12-yl)ureido]hexanoic acid, (S)-6-amino-2-[3-((9S,12R)-11-oxo-9-phenyl-2,7-dioxa-10-aza-bicyclo[12.2.2]octadeca-1(17),14(18),15-trien-12-yl)ureido]hexanoic acid, (S)-6-amino-2-[3-((9S,12R)-9-benzyl-11-oxo-2,7-dioxa-10-aza-bicyclo[12.2.2]octadeca-1(17),14(18),15-trien-12-yl)ureido]hexanoic acid, (S)-6-amino-2-[3-((9S,12R)-9-cyclohexyl-11-oxo-2,7-dioxa-10-aza-bicyclo[12.2.2]octadeca-1(17),14(18),15-trien-12-yl)ureido]hexanoic acid, (S)-6-amino-2-[3-((9S,12R)-9-ethyl-11-oxo-2,7-dioxa-10-azabicyclo[12.2.2] octadeca-1(17),14(18),15-trien-12-yl)ureido]hexanoic acid, (S)-6-amino-2-[3-((9S,12R)-9-methyl-11-oxo-2,7-dioxa-10-aza-bicyclo[12.2.2]octadeca-1(17),14(18),15-trien-12-yl)ureido]hexanoic acid, (S)-6-amino-2-[3-((9S,12R)-9-tert-butyl-11-oxo-2,7-dioxa-10-aza-bicyclo[12.2.2]octadeca-1(17),14(18),15-trien-12-yl)ureido]hexanoic acid, (S)-6-amino-2-[3-((13S,16R)-13-isopropyl-15-oxo-2,11-dioxa-14-aza-bicyclo[16.2.2]docosa-1(21),18(22),19-trien-16-yl)ureido]hexanoic acid, (S)-6-amino-2-[3-((E)-(9S,12R)-9-isopropyl-11-oxo-2,7-dioxa-10-aza-bicyclo[12.2.2]octadeca-1(17),4,14(18),15-tetraen-12-yl)ureido]hexanoic acid, (S)-6-amino-2-[3-((3S,6R)-3-isopropyl-5-oxo-1-oxa-4-azacyclotetradec-6-yl)ureido]hexanoic acid,
(R)-3-(2-aminoethanesulfonyl)-2-[3-((9S,12R)-9-isopropyl-11-oxo-2,7-dioxa-10-azabicyclo[12.2.2]octadeca-1(17),14(18),15-trien-12-yl)ureido]propionic acid,
(S)-3-(6-aminopyridin-3-yl)-2-[3-((9S,12R)-9-isopropyl-11-oxo-2,7-dioxa-10-azabicyclo[12.2.2]octadeca-1(17),14(18),15-trien-12-yl)ureido]propionic acid, (S)-6-amino-2-{3-[(9S,12R)-9-((S)-sec-butyl)-11-oxo-2,7-dioxa-10-aza-bicyclo[12.2.2]octadeca-1(17),14(18),15-trien-12-yl]ureido}hexanoic acid, (S)-3-(6-aminopyridin-3-yl)-2-[3-((3S,6R)-3-isopropyl-5-oxo-1-oxa-4-azacyclo-tetradec-6-yl)ureido]propionic acid,
(S)-3-(6-aminopyridin-3-yl)-2-[3-((3S,6R)-3-methyl-5-oxo-1-oxa-4-azacyclotetradec-6-yl)ureido]propionic acid,
ethyl (S)-3-(6-aminopyridin-3-yl)-2-[3-((3S,6R)-3-isopropyl-5-oxo-1-oxa-4-azacyclotetradec-6-yl)ureido]propionate,
ethyl (S)-3-(6-aminopyridin-3-yl)-2-[3-((3S,6R)-3-methyl-5-oxo-1-oxa-4-azacyclotetradec-6-yl)ureido]propionate,
(S)-3-(6-aminopyridin-3-yl)-2-[3-((9S,12R)-9-cyclopropyl-11-oxo-2,7-dioxa-10-azabicyclo[12.2.2]octadeca-1(17),14(18),15-trien-12-yl)ureido]propionic acid,
(S)-6-amino-2-[3-((9S,12R)-9-isopropyl-11-oxo-2,7-dioxa-10-azabicyclo[12.2.2]octadeca-1(17),14(18),15-trien-12-yl)ureido]-6-methylheptanoic acid,
ethyl (S)-3-(6-aminopyridin-3-yl)-2-[3-((9S,12R)-9-isopropyl-11-oxo-2,7-dioxa-10-azabicyclo[12.2.2]octadeca-1-(17),14(18),15-trien-12-yl)ureido]propionate,
(S)-2-[3-((9S,12R)-9-isopropyl-11-oxo-2,7-dioxa-10-azabicyclo[12.2.2]octadeca-1(17),14(18),15-trien-12-yl)ureido]-3-piperidin-3-ylpropionic acid,
(S)-3-[6-aminopyridin-3-yl)-2-[3-((9S,12R)-9-methyl-11-oxo-2,7-dioxa-10-azabicyclo[12.2.2]octadeca-1(17),14(18),15-trien-12-yl)ureido]propionic acid,
(S)-2-[3-((9S,12R)-9-isopropyl-11-oxo-2,7-dioxa-10-azabicyclo[12.2.2]octadeca-1(17),14(18),15-trien-12-yl)ureido]-3-piperidin-3-ylpropionic acid,
(S)-3-(6-aminopyridin-3-yl)-2-[3-((8S,11R)-8-isopropyl-10-oxo-6-oxa-1,9,14-triazabicyclo[11.2.1]hexadeca-13(16),14-dien-11-yl)ureido]propionic acid,
(S)-3-(6-aminopyridin-3-yl)-2-[3-((3R,6S)-6-isopropyl-4-oxo-8-oxa-5-azabicyclo[11.2.2]heptadeca-1(16),13(17),14-trien-3-yl)ureido]propionic acid,
(S)-3-(6-aminopyridin-3-yl)-2-[3-((9S,12R)-16-fluoro-9-isopropyl-11-oxo-2,7-dioxa-10-azabicyclo[12.2.2]octadeca-1(17),14(18),15-trien-12-yl)ureido]propionic acid,
(S)-3-(6-aminopyridin-3-yl)-2-[3-((3S,6R)-3-isopropyl-5-oxo-1,8-dioxa-4-azacyclododec-6-yl)ureido]propionic acid,
methyl (S)-3-(6-aminopyridin-3-yl)-2-[3-((3S,6R)-3-isopropyl-5-oxo-1,8-dioxa-4-azacyclododec-6-yl)ureido]propionate,
(S)-6-amino-2-[3-((3S,6R)-3-isopropyl-5-oxo-1,8-dioxa-4-azacyclododec-6-yl)ureido]hexanoic acid,
(S)-6-amino-2-[3-((9S,12S)-9-isopropyl-11-oxo-2,7-dioxa-10-azabicyclo-[12.2.2]octadec-12-yl)ureido]hexanoic acid,
(S)-6-amino-2-((8S,11R)-8-isopropyl-3,10-dioxo-6-oxa-2,9-diaza-bicyclo[11.2.2]heptadeca-1(16),13(17),14-trien-11-ylamino)hexanoic acid,
(S)-3-(6-aminopyridin-3-yl)-2-[3-((8S,11R)-8-methyl-3,10-dioxo-6-oxa-2,9-diazabicyclo[11.2.2]heptadeca-1(16),13(17),14-trien-11-yl)ureido]propionic acid,
(S)-3-(6-aminopyridin-3-yl)-2-[3-((3S,6R)-3-isopropyl-5,9-dioxo-1-oxa-4,10-diazacyclotridec-6-yl)ureido]propionic acid,
(S)-3-(6-aminopyridin-3-yl)-2-[3-((8S,11R)-8-isopropyl-2,10-dioxo-6-oxa-3,9-diazabicyclo[11.2.2]heptadeca-1(16),13(17),14-trien-11-yl)ureido]propionic acid,
(S)-6-amino-2-[3-((9S,12R)-4,5-dihydroxy-9-isopropyl-11-oxo-2,7-dioxa-10-azabicyclo[12.2.2]octadeca-1(17),14(18),15-trien-12-yl)ureido]hexanoic acid,
(S)-6-amino-2-[3-((9S,12R)-5-hydroxy-9-isopropyl-11-oxo-2,7-dioxa-10-azabicyclo[12.2.2]octadeca-1(17),14(18),15-trien-12-yl)ureido]hexanoic acid,
3-((6-aminopyridin-3-yl)-2-[3-((9S,12R)-4,5-dihydroxy-9-isopropyl-11-oxo-2,7-dioxa-10-azabicyclo[12.2.2]octadeca-1(17),14(18),15-trien-12-yl)ureido]propionic acid,(S)-6-amino-2-[3-((9S,12R)-4-hydroxy-9-isopropyl-11-oxo-2,7-dioxa-10-azabicyclo[12.2.2]octadeca-1(17),14(18),15-trien-12-yl)ureido]hexanoic acid or
(S)-6-amino-2-[((9S,12R)-9-isopropyl-11-oxo-2,7-dioxa-10-azabicyclo[12.2.2]octadeca-1(17),14(18),15-trien-12-ylsulfamoyl)amino]hexanoic acid.

7. A process for preparing the compound of the formula I as claimed in one or more of claims 1 to 6, which comprises
a) reacting a compound of the formula (II) 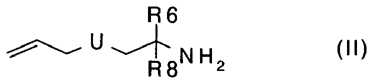 where U, R6 and R8 have the meanings mentioned in the compound of the formula (I), with an amino acid of the formula (III) 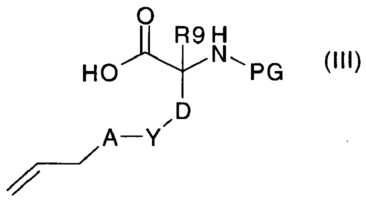 in which R9, A, Y and D have the meanings mentioned in the compound of the formula (I), resulting in a compound of the formula (IV) 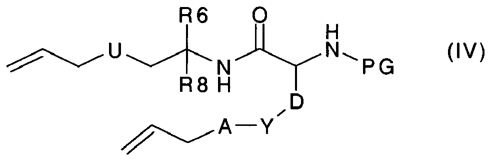 which is converted under the conditions of ring-closure metathesis and subsequent hydrogenation of the resulting double bond into a compound of the formula (V) 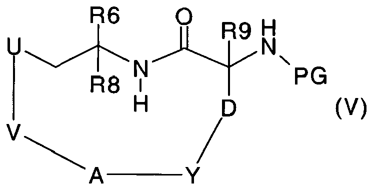 where V is -(C₂-C₆)-alkylene- or -(C₃-C₉)-alkenylene-, subsequently eliminating the protective group PG, and obtaining the compound of the formula (VI), 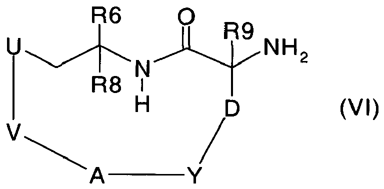 and reacting with a compound of the formula (VII) 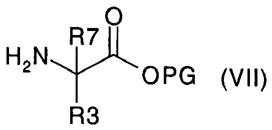 in which R3 and R7 have the meanings mentioned in formula (I), PG is a suitable ester protective group radical, and the nitrogen in R3 is protected where appropriate by a suitable amino protective group, with phosgene or a phosgene equivalent to give a compound of the formula (VIII) 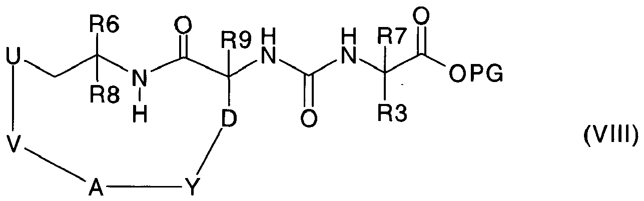 subsequently the protective group PG and the protective group which is present where appropriate on the nitrogen in R3 are eliminated, resulting in the compound of the formula (I), or
b) reacting a compound of the formula (IX) 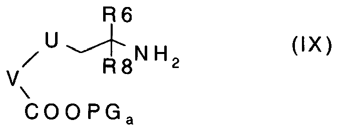 in which U, V, R6 and R8 have the meanings mentioned in the compound of the formula (I), and PGₐ is a suitable carboxyl protective group, with an amino acid of the formula (X) 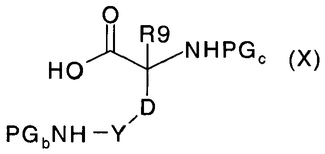 in which R9, Y and D have the meanings mentioned in the compound of the formula I, and PG_{b} and PG_{c} are suitable amino protective groups, resulting in a compound of the formula (XI) 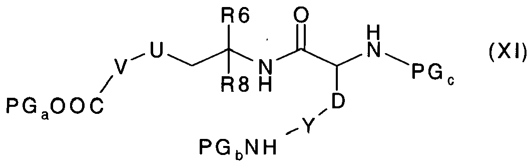 which, after elimination of the protective groups PGₐ and PG_{b}, is converted into the compound of the formula (XII) 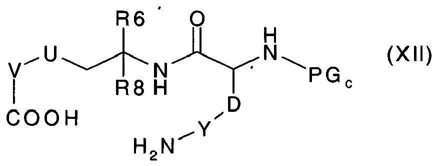 which is converted by means of an amide coupling into a compound of the formula (V),
in which A has the meanings mentioned in the compound of the formula I, subsequently the protective group is eliminated and the compound of the formula (VI) is obtained,
and reacted with a compound of the formula (VII) in which R3 and R7 have the meanings mentioned in formula I, PG is a suitable ester protective group radical, and the nitrogen in R3 is protected where appropriate by a suitable amino protective group, with phosgene or a phosgene equivalent to give a compound of the formula (VIII), subsequently the protective group PG and the protective group which is present where appropriate on the nitrogen in R3 are eliminated, resulting in the compound of the formula (I), or
c) reacting a compound of the formula (XIII) 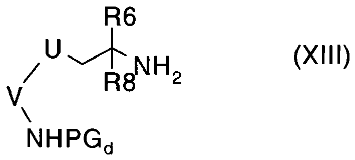 in which U, V, R6 and R8 have the meanings mentioned in the compound of the formula I, and PG_{d} is a suitable amino protective group, with an amino acid of the formula (XIV) 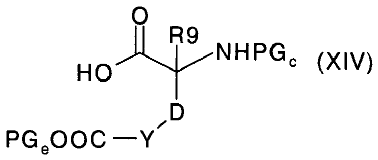 in which R9, Y and D have the meanings mentioned in the compound of the formula I, and PG_{c} is a suitable amino protective group and PGₑ is a suitable carboxyl protective group, resulting in a compound of the formula (XV) 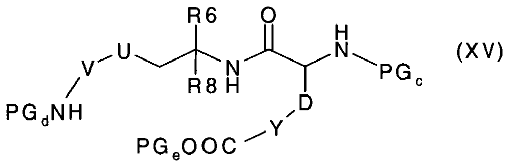 which, after elimination of the protective groups PG_{d} and PGₑ, is converted into the compound of the formula (XVI) 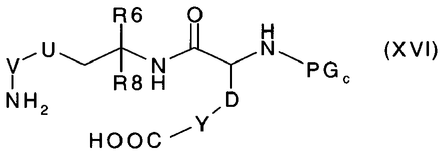 which is reacted to give a compound of the formula (V),
in which A has the meanings mentioned in the compound of the formula I, subsequently the protective group is eliminated and the compound of the formula (VI) is obtained,
and reacted with a compound of the formula (VII),
in which R3 and R7 have the meanings mentioned in formula I, PG is a suitable ester protective group radical, and the nitrogen in R3 is protected where appropriate by a suitable amino protective group, with phosgene or a phosgene equivalent to give a compound of the formula (VIII), subsequently the protective group PG and the protective group which is present where appropriate on the nitrogen in R3 are eliminated, resulting in the compound of the formula (I), or
d) reacting a compound of the formula XVII 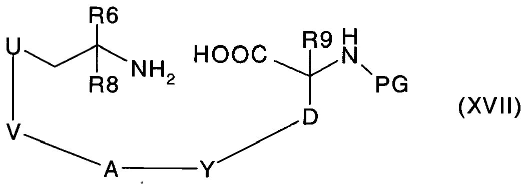 in which U, V, A, Y, D, R₆, R₈ and R₉ have the meanings mentioned in the compound of the formula I, with a compound of the formula (V), subsequently the protective groups are eliminated, and a compound of the formula (VI) is obtained,
and reacted with a compound of the formula (VII),
in which R3 and R7 have the meanings mentioned in formula I, PG is a suitable ester protective group radical, and the nitrogen in R3 is
protected where appropriate by a suitable amino protective group, with phosgene or a phosgene equivalent to give a compound of the formula (VIII), and subsequently the protective groups PG and, where appropriate, the protective group on the nitrogen in R3 are eliminated, resulting in the compound of the formula (I), or
e) converting a compound of the formula (VIIIa) 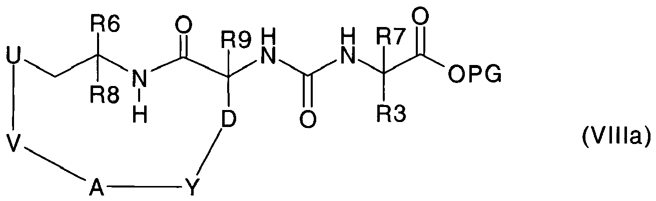 in which V is -(C₃-C₉)-alkenylene-, into the compound of the formula (VIIIb) 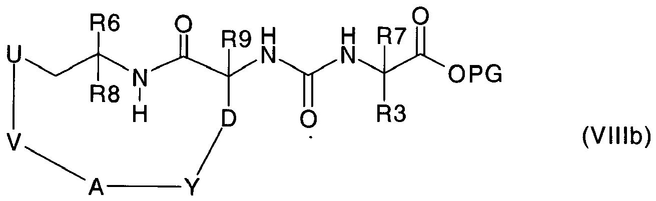 in which V is -(C₃-C₉)-alkylene-, where alkylene is substituted independently of one another once, twice or three times by -OH, NH₂ or halogen, or is -(C₁-C₂)-alkylene-(C₃-C₆)-cycloalkyl-(C₁-C₂)-alkylene-, where cycloalkyl is substituted independently of one another once, twice or three times by R15, subsequently the compound of the formula (VIIIb) is converted in analogy to process a) into the compound of the formula (I), or
f) reacting a compound of the formula (XVIII) 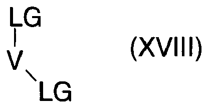 in which V is as defined in the compound of the formula (I), successively with the compounds of the formula (XIX) and (XX) 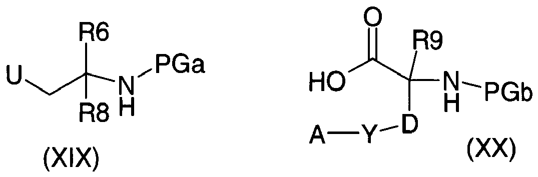 employing bases in polar, aprotic solvents, and converting the resulting compounds of the formula (XXI) 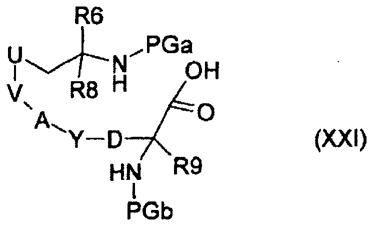 by removing the protective group PGa and subsequent formation of a peptide linkage into a compound of the formula (V), and reacting the latter as in process a) to give compounds of the formula (I) in which R6, R8, R9, and A, D, U, V and Y have the meanings mentioned in formula (I), and PG is suitable protective groups, and LG is a leaving group such as chlorine, bromine, iodine or sulfonic ester, or
g) reacting a compound of the formula VI with a compound of the formula (XXII) 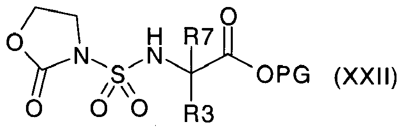 in which R3 and R7 have the meanings mentioned in the compound of the formula I, and PG is a suitable protective group radical, to give a compound of the formula (XXIII) 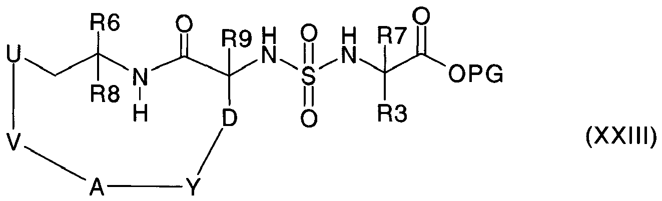 and then convering into a compound of the formula (I), or
h) fractionating a compound of the formula I prepared by processes a), b), c), d), e), f) or g), or a suitable precursor of the formula (I) which occurs in enantiomeric forms owing to its chemical structure, by salt formation with enantiopure acids or bases, chromatography on chiral stationary phases or derivatization by means of chiral enantiopure compounds such as amino acids, separation of the diastereomers obtained in this way, and elimination of the chiral auxiliary groups into the pure enantiomers, or
i) either isolating in free form the compound of the formula (I) prepared by processes a), b), c), d), e), f) or g), or converting into physiologically tolerated salts in the case where acidic or basic groups are present.

8. A medicament comprising an effective content of at least one compound of the formula I as claimed in one or more of claims 1 to 6 together with a pharmaceutically suitable and physiologically tolerated carrier, additive and/or other active ingredients and excipients.

9. The compound of the formula I as claimed in one or more of claims 1 to 6 for the prophylaxis, secondary prevention and therapy of one or more disorders which are associated with thromboses, embolisms, hypercoagulability or fibrotic changes.

10. The compound as claimed in claim 9, which is concerned with one or more disorders from the series myocardial infarction, angina pectoris and other forms of acute coronary syndrome, stroke, peripherally vascular disorders, deep vein thrombosis, pulmonary embolism, embolic or thrombotic events caused by cardiac arrhythmias, cardiovascular events such as restenosis following revascularization and angioplasty and similar procedures such as stent implantations and bypass operations, or reducing the risk of thrombosis following surgical procedures such as operations on the knee and hip joints, or in the context of disseminated intravascular coagulation, sepsis and other intravascular events associated with inflammation, atherosclerosis, diabetes and the metabolic syndrome and the sequelae thereof, tumor growth and tumor metastasis, inflammatory and degenerative articular disorders such as rheumatoid arthritis and arthrosis, impairments of the hemostatic system such as fibrin deposits, fibrotic changes of the lung such as chronic obstructive pulmonary disease, adult respiratory distress syndrome or fibrin deposits in the eye following eye operations or prevention or treatment of scarring.

## Revendications

1. Composé de formule I 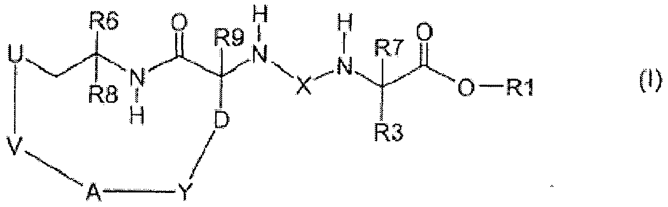 et/ou une forme stéréoisomère du composé de formule I et/ou mélanges de ces formes en n'importe quelle proportion, et/ou un sel toléré sur le plan physiologique du composé de formule I, formule dans laquelle
X représente -C(O)- ou -SO₂-,
U représente un atome d'oxygène, un atome de soufre, NH, -C(O)-NH- ou - alkylène (en C₀-C₄)-,
A représente un atome d'oxygène, un atome de soufre, NH, -C(O)-NH- - NH-C(O)-, -NR2- ou -alkylène (en C₀-C₄)-,
V représente
1) -alkylène (en C₂-C₉)-, où le groupe alkylène est non substitué ou substitué, indépendamment des autres, une fois, deux fois ou trois fois par -OH, NH₂ ou un halogène,
2) -alkylène (en C₁-C₂)-cycloalkyl(en C₃-C₆)-alkylène (en C₁-C₂)-, où le groupe cycloalkyle est substitué, indépendamment des autres, une fois, deux fois ou trois fois par R15, ou
3) -alcénylène (en C₃-C₉)-,
D représente -alkylène (en C₁-C₂)-,
Y représente
1) une liaison covalente,
2) -cycloalkyle (en C₃-C₁₂), où le groupe cycloalkyle est substitué, indépendamment des autres, une fois, deux fois ou trois fois par R15,
3) -aryle (en C₆-C₁₄), où le groupe aryle est non substitué ou substitué, indépendamment des autres, une fois, deux fois ou trois fois par R15, ou
4) Het, où Het désigne un système cyclique hétérocyclique à 4 à 15 chaînons comportant 4 à 15 atomes dans le cycle qui sont présents dans un, deux ou trois systèmes cycliques qui sont reliés les uns aux autres, et qui comprennent, suivant la taille du cycle, un, deux, trois ou quatre hétéroatomes identiques ou différents de la série oxygène, azote ou soufre, et
dans lequel Het est non substitué ou substitué, indépendamment des autres, une fois, deux fois ou trois fois par un groupe -alkyle (en C₁-C₃), un halogène, -NH₂, -CF₃ ou -O-CF₃,
R1 représente
1) un atome d'hydrogène,
2) -alkyle (en C₁-C₆),
3) -alkyl(en C₁-C₆)-OH,
4) -alkyl(en C₀-C₄)-cycloalkyle (en C₃-C₆),
5) -alkyl(en C₁-C₁₀)-O-C(O)-O-R2,
6) -(CH₂)ᵣ-aryle (en C₆-C₁₄), où le groupe aryle est non substitué ou substitué, indépendamment des autres, une fois, deux fois ou trois fois par R15, et r est le nombre entier zéro, 1, 2 ou 3, ou
7) -(CH₂)ₛ-Het, où Het désigne un système cyclique hétérocyclique à 4 à 15 chaînons comportant 4 à 15 atomes dans le cycle qui sont présents dans un, deux ou trois systèmes cycliques qui sont reliés les uns aux autres et qui comprennent, suivant la taille du cycle, un, deux, trois ou quatre hétéroatomes identiques ou différents de la série oxygène, azote ou soufre, et dans lequel Het est non substitué ou substitué, indépendamment des autres, une fois, deux fois ou trois fois par R15, et s est le nombre entier zéro, 1, 2 ou 3,
R2 représente
1) -alkyle (en C₁-C₆),
2) -(CH₂)ᵣ-aryle (en C₆-C₁₄), où le groupe aryle est non substitué ou substitué, indépendamment des autres, une fois, deux fois ou trois fois par R15, et r est le nombre entier zéro, 1, 2 ou 3, ou
3) -alkyl(en C₀-C₄)-cycloalkyle (en C₃-C₆),
R3 représente
1) -alkylène (en C₂-C₆)-NH₂, où le groupe alkylène est non substitué ou substitué une fois, deux fois, trois fois ou quatre fois par un halogène,
2) -alkylène (en C₁-C₄)-O-alkylène (en C₁-C₄)-NH₂,
3) -alkylène (en C₁-C₄)-SO₂-alkylène (en C₁-C₄)-NH₂,
4) -alkylène (en C₀-C₄)-Het, où Het est tel que défini ci-dessus et est substitué par -NH₂ et une fois, deux fois ou trois fois par R15,
5) -alkylène (en C₀-C₄)-cycloalkyl(en C₃-C₈)-NH₂ ou
6) -alkylène (en C₀-C₆)-amine cyclique,
R6 représente
1) un atome d'hydrogène,
2) -alkyle (en C₁-C₆), où le groupe alkyle est non substitué ou substitué, indépendamment des autres, une fois, deux fois ou trois fois par R16,
3) -O-alkyle (en C₁-C₆), où le groupe alkyle est non substitué ou substitué, indépendamment des autres, une fois, deux fois ou trois fois par R16,
4) -alkylène (en C₀-C₄)-Het, où Het est tel que défini ci-dessus, où les groupes alkylène et Het sont non substitués ou substitués, indépendamment l'un de l'autre, une fois, deux fois ou trois fois par R16,
5) -alkylène (en C₀-C₄)-aryle, où les groupes alkylène et aryle sont non substitués ou substitués, indépendamment l'un de l'autre, une fois, deux fois ou trois fois par R16, ou
6) -alkylène (en C₀-C₄)-cycloalkyle (en C₃-C₈), où les groupes alkylène et cycloalkyle sont non substitués ou substitués, indépendamment l'un de l'autre, une fois, deux fois ou trois fois par R16,
R7 représente un atome d'hydrogène, un halogène ou un groupe -alkyle (en C₁-C₆),
R8 représente un atome d'hydrogène, un halogène ou un groupe -alkyle (en C₁-C₆),
R9 représente un atome d'hydrogène, un halogène ou un groupe -alkyle (en C₁-C₆),
R15 représente un atome d'hydrogène, ou un groupe -alkyle (en C₁-C₄), -O-CF₃, -NH₂, -OH, -CF₃ ou un halogène, et
R16 représente -O-CF₃, -NH₂, -OH, -CF₃ ou un halogène.

2. Composé de formule I selon la revendication 1, dans lequel
X représente -C(O)- ou -SO₂-,
U représente un atome d'oxygène, un atome de soufre, NH, -C(O)-NH- ou - alkylène (en C₀-C₄)-,
A représente un atome d'oxygène, un atome de soufre, NH, -C(O)-NH- ou - alkylène (en C₀-C₄)-,
V représente -alkylène (en C₂-C₉)- ou -alcénylène (en C₃-C₉)-,
D représente -alkylène (en C₁-C₂)-,
Y représente
1) une liaison covalente,
2) -cycloalkyle (en C₃-C₁₂), où le groupe cycloalkyle est substitué, indépendamment des autres, une fois, deux fois ou trois fois par R15,
3) -aryle (en C₆-C₁₄), où le groupe aryle est non substitué ou substitué, indépendamment des autres, une fois, deux fois ou trois fois par R15, ou
4) Het, où Het désigne un système cyclique hétérocyclique à 4 à 15 chaînons comportant 4 à 15 atomes dans le cycle, qui sont présents dans un, deux ou trois systèmes cycliques qui sont reliés les uns aux autres, et qui comprennent, suivant la taille du cycle, un, deux, trois ou quatre hétéroatomes identiques ou différents de la série oxygène, azote ou soufre, et
dans lequel Het est non substitué ou substitué, indépendamment des autres, une fois, deux fois ou trois fois par un groupe -alkyle (en C₁-C₃), un halogène, -NH₂, -CF₃ ou -O-CF₃,
R1 représente
1) un atome d'hydrogène,
2) -alkyle (en C₁-C₆),
3) -alkyl(en C₁-C₆)-OH,
4) -alkyl(en C₀-C₄)-cycloalkyle (en C₃-C₆),
5) -alkyl(en C₁-C₁₀)-O-C(O)-O-R2,
6) -(CH₂)ᵣ-aryle (en C₆-C₁₄), où le groupe aryle est non substitué ou substitué, indépendamment des autres, une fois, deux fois ou trois fois par R15, et r est le nombre entier zéro, 1, 2 ou 3, ou
7) -(CH₂)ₛ-Het, où Het désigne un système cyclique hétérocyclique à 4 à 15 chaînons comportant 4 à 15 atomes dans le cycle qui sont présents dans un, deux ou trois systèmes cycliques qui sont reliés les uns aux autres, et qui comprennent, suivant la taille du cycle, un, deux, trois ou quatre hétéroatomes identiques ou différents de la série oxygène, azote ou soufre, et dans lequel Het est non substitué ou substitué, indépendamment des autres, une fois, deux fois ou trois fois par R15, et s est le nombre entier zéro, 1, 2 ou 3,
R2 représente
1) -alkyle (en C₁-C₆),
2) -(CH₂)ᵣ-aryle (en C₆-C₁₄), où le groupe aryle est non substitué ou substitué, indépendamment des autres, une fois, deux fois ou trois fois par R15, et r est le nombre entier zéro, 1, 2 ou 3, ou
3) -alkyl(en C₀-C₄)-cycloalkyle (en C₃-C₆),
R3 représente
1) -alkylène (en C₂-C₆)-NH₂, où le groupe alkylène est non substitué ou substitué une fois, deux fois, trois fois ou quatre fois par un halogène,
2) -alkylène (en C₁-C₄)-O-alkylène (en C₁-C₄)-NH₂,
3) -alkylène (en C₁-C₄)-SO₂-alkylène (en C₁-C₄)-NH₂,
4) -alkylène (en C₀-C₄)-Het, où Het est tel que défini ci-dessus et est substitué par -NH₂ et une fois, deux fois ou trois fois par R15,
5) -alkylène (en C₀-C₄)-cycloalkyl(en C₃-C₈)-NH₂ ou
6) -alkylène (en C₀-C₆)-amine cyclique,
R6 représente
1) un atome d'hydrogène,
2) -alkyle (en C₁-C₆), où le groupe alkyle est non substitué ou substitué, indépendamment des autres, une fois, deux fois ou trois fois par R16,
3) -O-alkyle (en C₁-C₆), où le groupe alkyle est non substitué ou substitué, indépendamment des autres, une fois, deux fois ou trois fois par R16,
4) -alkylène (en C₀-C₄)-Het, où Het est tel que défini ci-dessus, où les groupes alkylène et Het sont non substitués ou substitués, indépendamment l'un de l'autre, une fois, deux fois ou trois fois par R16,
5) -alkylène (en C₀-C₄)-aryle, où les groupes alkylène et aryle sont non substitués ou substitués, indépendamment l'un de l'autre, une fois, deux fois ou trois fois par R16, ou
6) -alkylène (en C₀-C₄)-cycloalkyle (en C₃-C₈), où les groupes alkylène et cycloalkyle sont non substitués ou substitués, indépendamment l'un de l'autre, une fois, deux fois ou trois fois par R16,
R7 représente un atome d'hydrogène, un halogène ou un groupe -alkyle (en C₁-C₆),
R8 représente un atome d'hydrogène, un halogène ou un groupe -alkyle (en C₁-C₆),
R9 représente un atome d'hydrogène, un halogène ou un groupe -alkyle (en C₁-C₆),
R15 représente un atome d'hydrogène, ou un groupe -alkyle (en C₁-C₄),-O-CF₃, -NH₂, -OH, -CF₃ ou un halogène, et
R16 représente -O-CF₃, -NH₂, -OH, -CF₃ ou un halogène.

3. Composé de formule I selon la revendication 1 ou 2, dans lequel
X représente -C(O)- ou -SO₂-,
U représente un atome d'oxygène, un atome de soufre, NH, -C(O)-NH- ou - alkylène (en C₀-C₄)-,
A représente un atome d'oxygène ou un groupe -alkylène (en C₀-C₄)-,
V représente -alkylène (en C₂-C₈)- ou -alcénylène (en C₃-C₆)-,
D représente -alkylène (en C₁-C₂)-,
Y représente
1) une liaison covalente,
2) -cycloalkyle (en C₃-C₆), où le groupe cycloalkyle est substitué, indépendamment des autres, une fois, deux fois ou trois fois par R15,
3) -aryle (en C₆-C₁₄), où le groupe aryle est choisi parmi les groupes phényle, naphtyle, anthryle ou fluorényle, et où le groupe aryle est non substitué ou substitué, indépendamment des autres, une fois, deux fois ou trois fois par R15, ou
4) Het, où Het est choisi parmi les groupes acridinyle, azépinyle, azétidinyle, aziridinyle, benzimidazalinyle, benzimidazolyle, benzo[1,3]dioxolyle, benzofuranyle, benzothiofuranyle, benzothiophényle, benzoxazolyle, benzothiazolyle, benzotriazolyle, benzotétrazolyle, benzisoxazolyle, benzisothiazolyle, carbazolyle, 4αH-carbazolyle, carbolinyle, quinazolinyle, quinolinyle, 4H-quinolizinyle, quinoxalinyle, quinuclidinyle, chromanyle, chroményle, cinnolinyle, décahydroquinolinyle, dibenzofuranyle,
dibenzothiophényle, dihydrofurane[2,3-b]-tétrahydrofuranyle, dihydrofuranyle, dioxolyle, dioxanyle, 2H,6H-1,5,2-dithiazinyle, furanyle, furazanyle, imidazolidinyle, imidazolinyle, imidazolyle, 1 H-indazolyle, indolinyle, indolizinyle, indolyle, 3H-indolyle, isobenzofuranyle, isochromanyle, isoindazolyle, isoindolinyle, isoindolyle, isoquinolinyle (benzimidazolyle), isothiazolidinyle, 2-isothiazolinyle, isothiazolyle, isoxazolyle, isoxazolidinyle, 2-isoxazolinyle, morpholinyle, naphtyridinyle, octahydroisoquinolinyle, oxadiazolyle, 1,2,3-oxadiazolyle, 1,2,4-oxadiazolyle, 1,2,5-oxadiazolyle, 1,3,4-oxadiazolyle, oxazolidinyle, oxazolyle, oxazolidinyle, oxothiolanyle, pyrimidinyle, phénanthridinyle, phénanthrolinyle, phénazinyle, phénothiazinyle, phénoxathiinyle, phénoxazinyle, phtalazinyle, pipérazinyle, pipéridinyle, ptéridinyle, purynyle, pyranyle, pyrazinyle, pyroazolidinyle, pyrazolinyle, pyrazolyle, pyridazinyle, pyridooxazolyle, pyridoimidazolyle, pyridothiazolyle, pyridothiophényle, pyridinyle, pyridyle, pyrimidinyle, pyrrolidinyle, pyrrolinyle, 2H-pyrrolyle, pyrrolyle, tétrahydrofuranyle, tétrahydroisoquinolinyle, tétrahydroquinolinyle, tétrahydropyridinyle, 6H-1,2,5-thiadazinyle, 1,2,3-thiadiazolyle, 1,2,4-thiadiazolyle, 1,2,5-thiadiazolyle, 1,3,4-thiadiazolyle, thianthrényle, thiazolyle, thiényle, thiénoimidazolyle, thiénooxazolyle, thiénopyridine, thiénothiazolyle, thiomorpholinyle, thiophényle, triazinyle, 1,2,3-triazolyle, 1,2,3-triazolyle, 1,2,4-triazolyle, 1,2,5-triazolyle, 1,3,4-triazolyle et xanthényle, et
dans lequel Het est non substitué ou substitué, indépendamment des autres, une fois, deux fois ou trois fois par un groupe -alkyle (en C₁-C₃), un halogène, -NH₂, -CF₃ ou -O-CF₃,
R1 représente
1) un atome d'hydrogène ou
2) -alkyle (en C₁-C₄),
R3 représente
1) -alkylène (en C₂-C₆)-NH₂, où le groupe alkylène est non substitué ou substitué une fois, deux fois, trois fois ou quatre fois par un halogène,
2) -alkylène (en C₁-C₄)-SO₂-alkylène (en C₁-C₄)-NH₂ ou
3) -alkylène (en C₀-C₄)-Het, où Het est tel que défini ci-dessus et est substitué par -NH₂ et une fois, deux fois ou trois fois par R15,
R6 représente
1) un atome d'hydrogène,
2) -alkyle (en C₁-C₆), où le groupe alkyle est non substitué ou substitué, indépendamment des autres, une fois, deux fois ou trois fois par R16,
3) -O-alkyle (en C₁-C₆), où le groupe alkyle est non substitué ou substitué, indépendamment des autres, une fois, deux fois ou trois fois par R16,
4) -alkylène (en C₀-C₄)-Het, où Het est tel que défini ci-dessus, où les groupes alkylène et Het sont non substitués ou substitués, indépendamment l'un de l'autre, une fois, deux fois ou trois fois par R16,
5) -alkylène (en C₀-C₄)-aryle, où les groupes alkylène et aryle sont non substitués ou substitués, indépendamment l'un de l'autre, une fois, deux fois ou trois fois par R16, ou
6) -alkylène (en C₀-C₄)-cycloalkyle (en C₃-C₆), où les groupes alkylène et cycloalkyle sont non substitués ou substitués, indépendamment l'un de l'autre, une fois, deux fois ou trois fois par R16,
R7 représente un atome d'hydrogène, F ou un groupe -alkyle (en C₁-C₄),
R8 représente un atome d'hydrogène, F ou un groupe -alkyle (en C₁-C₄),
R9 représente un atome d'hydrogène, F ou un groupe -alkyle (en C₁-C₄),
R15 représente un atome d'hydrogène, un groupe -alkyle (en C₁-C₄), -O-CF₃, -NH₂, -OH, -CF₃ ou un halogène, et
R16 représente -O-CF₃, -OH, -CF₃ ou F.

4. Composé de formule I selon la revendication 1 ou 2, dans lequel
X représente -C(O)-,
U représente un atome d'oxygène,
A représente un atome d'oxygène ou un groupe -alkylène (en C₀-C₄)-,
V représente
1) -alkylène (en C₂-C₈)-, où le groupe alkylène est non substitué ou substitué, indépendamment des autres, une fois ou deux fois par -OH, F ou CI,
2) -alkylène (en C₁-C₂)-cyclopropyl-alkylène (en C₁-C₂)-, où le groupe cyclopropyle est substitué une fois ou deux fois par F, ou
3) -alcénylène (en C₃-C₆)-,
D représente -alkylène (en C₁-C₂)-,
Y représente
1) une liaison covalente ou
2) phényle, où le groupe phényle est non substitué ou substitué, indépendamment des autres, une fois, deux fois ou trois fois par R15,
R1 représente
1) un atome d'hydrogène ou
2) -alkyle (en C₁-C₄),
R3 représente
1) -alkylène (en C₂-C₆)-NH₂,
2) -alkylène (en C₁-C₄)-SO₂-alkylène (en C₁-C₄)-NH₂ ou
3) -alkylène (en C₀-C₄)-pyridyle, où le groupe pyridyle est substitué par -NH₂ et une fois, deux fois ou trois fois par R15,
R6 représente
1) un atome d'hydrogène,
2) -alkyle (en C₁-C₆),
3) -CF₃,
4) -alkylène (en C₀-C₄)-phényle ou
5) -alkylène (en C₀-C₄)-cycloalkyle (en C₃-C₆),
R7, R8 et R9 représentent chacun un atome d'hydrogène, et
R15 représente un atome d'hydrogène, ou un groupe -alkyle (en C₁-C₄),-CF₃ ou un halogène.

5. Composé de formule I selon les revendications 1 à 4, dans lequel
X représente -C(O)-,
U représente un atome d'oxygène,
A représente un atome d'oxygène ou un groupe -alkylène (en C₀-C₄)-,
V représente -alkylène (en C₂-C₈)- ou -alcénylène (en C₃-C₆)-,
D représente -alkylène (en C₁-C₂)-,
Y représente
1) une liaison covalente ou
2) phényle, où le groupe phényle est non substitué ou substitué, indépendamment des autres, une fois, deux fois ou trois fois par R15,
R1 représente
1) un atome d'hydrogène ou
2) -alkyle (en C₁-C₄),
R3 représente
1) -alkylène (en C₂-C₆)-NH₂,
2) -alkylène (en C₁-C₄)-SO₂-alkylène (en C₁-C₄)-NH₂ ou
3) -alkylène (en C₀-C₄)-pyridyle, où le groupe pyridyle est substitué par -NH₂ et une fois, deux fois ou trois fois par R15,
R6 représente
1) un atome d'hydrogène,
2) -alkyle (en C₁-C₆),
3) -CF₃,
4) -alkylène (en C₀-C₄)-phényle ou
5) -alkylène (en C₀-C₄)-cycloalkyle (en C₃-C₆),
R7, R8 et R9 représentent chacun un atome d'hydrogène, et
R15 représente un atome d'hydrogène, un groupe -alkyle (en C₁-C₄), -CF₃ ou un halogène.

6. Composé de formule I selon une ou plusieurs des revendications 1 à 5, qui est le composé de formule I
acide (S)-6-amino-2-[3-((9S,12R)-9-isopropyl-11-oxo-2,7-dioxa-10-azabicyclo[12.2.2]octadéca-1(17),14(18),15-trién-12-yl)uréido]hexanoïque,
acide (S)-6-amino-2-[3-((R)-11-oxo-2,7-dioxa-10-azabicyclo-[12.2.2]octadéca-1(17),14(18),15-trién-12-yl)uréido]hexanoïque,
acide (S)-6-amino-2-[3-((R)-9,9-diméthyl-11-oxo-2,7-dioxa-10-azabicyclo[12.2.2]octadéca-1 (17),14(18),15-trién-12-yl)uréido]hexanoïque,
acide (S)-6-amino-2-[3-((9S,12R)-11-oxo-9-phényl-2,7-dioxa-10-azabicyclo[12.2.2]octadéca-1 (17),14(18),15-trién-12-yl)uréido]hexanoïque,
acide (S)-6-amino-2-[3-((9S,12R)-9-benzyl-11-oxo-2,7-dioxa-10-azabicyclo[12.2.2]octadéca-1 (17),14(18),15-trién-12-yl)uréido]hexanoïque,
acide (S)-6-amino-2-[3-((9S,12R)-9-cyclohexyl-11-oxo-2,7-dioxa-10-azabicyclo[12.2.2]octadéca-1 (17),14(18),15-trién-12-yl)uréido]hexanoïque,
acide (S)-6-amino-2-[3-((9S,12R)-9-éthyl-11-oxo-2,7-dioxa-10-azabicyclo[12.2.2]octadéca-1(17),14(18),15-trién-12-yl)uréido]hexanoïque,
acide (S)-6-amino-2-[3-((9S,12R)-9-méthyl-11-oxo-2,7-dioxa-10-azabicyclo[12.2.2]octadéca-1 (17),14(18),15-trién-12-yl)uréido]hexanoïque,
acide (S)-6-amino-2-[3-((9S,12R)-9-tert-butyl-11-oxo-2,7-dioxa-10-azabicyclo[12.2.2]octadéca-1 (17),14(18),15-trién-12-yl)uréido]hexanoïque,
acide (S)-6-amino-2-[3-((13S,16R)-13-isopropyl-15-oxo-2,11-dioxa-14-azabicyclo[16.2.2]docosa-1(21),18(22),19-trién-16-yl)uréido]hexanoïque,
acide (S)-6-amino-2-[3-((E)-(9S,12R)-9-isopropyl-11-oxo-2,7-dioxa-10-azabicyclo[12.2.2]octadéca-1 (17),4,14(18),15-tétraén-12-yl)uréido]hexanoïque,
acide (S)-6-amino-2-[3-((3S,6R)-3-isopropyl-5-oxo-1-oxa-4-azacyclotétradéc-6-yl)uréido]hexanoïque,
acide (R)-3-(2-aminoéthanesulfonyl)-2-[3-((9S,12R)-9-isopropyl-11-oxo-2,7-dioxa-10-azabicyclo[12.2.2]octadéca-1(17),14(18),15-trién-12-yl)uréido]propionique,
acide (S)-3-(6-aminopyridin-3-yl)-2-[3-((9S,12R)-9-isopropyl-11-oxo-2,7-dioxa-10-azabicyclo[12.2.2]octadéca-1(17),14(18),15-trién-12-yl)uréido]propionique,
acide (S)-6-amino-2-{3-[(9S,12R)-9-((S)-sec-butyl)-11-oxo-2,7-dioxa-10-azabicyclo[12.2.2]octadéca-1(17),14(18),15-trién-12-yl]uréido}hexanoïque,
acide (S)-3-(6-aminopyridin-3-yl)-2-[3-((3S,6R)-3-isopropyl-5-oxo-1-oxa-4-azacyclotétradéc-6-yl)uréido]propionique,
acide (S)-3-(6-aminopyridin-3-yl)-2-[3-((3S,6R)-3-méthyl-5-oxo-1-oxa-4-azacyclotétradéc-6-yl)uréido]propionique,
(S)-3-(6-aminopyridin-3-yl)-2-[3-((3S,6R)-3-isopropyl-5-oxo-1-oxa-4-azacyclotétradéc-6-yl)uréido]propionate d'éthyle,
(S)-3-(6-aminopyridin-3-yl)-2-[3-((3S,6R)-3-méthyl-5-oxo-1-oxa-4-azacyclotétradéc-6-yl)uréido]propionate d'éthyle,
acide (S)-3-(6-aminopyridin-3-yl)-2-[3-((9S,12R)-9-cyclopropyl-11-oxo-2,7-dioxa-10-azabicyclo[12.2.2]octadéca-1(17),14(18),15-trién-12-yl)uréido]propionique,
acide (S)-6-amino-2-[3-((95,12R)-9-isopropyl-11-oxo-2,7-dioxa-10-azabicyclo[12.2.2]octadéca-1(17),14(18),15-trién-12-yl)uréido]-6-méthylheptanoïque,
(S)-3-(6-aminopyridin-3-yl)-2-[3-((9S,12R)-9-isopropyl-11-oxo-2,7-dioxa-10-azabicyclo[12.2.2]octadéca-1-(17),14(18),15-trién-12-yl)uréido]propionate d'éthyle,
acide (S)-2-[3-((9S,12R)-9-isopropyl-11-oxo-2,7-dioxa-10-azabicyclo[12.2.2]octadéca-1(17),14(18),15-trién-12-yl)uréido]-3-pipéridin-3-ylpropionique,
acide (S)-3-(6-aminopyridin-3-yl)-2-[3-((9S,12R)-9-méthyl-11-oxo-2,7-dioxa-10-azabicyclo[12.2.2]octadéca-1(17),14(18),15-trién-12-yl)uréido]propionique,
acide (S)-2-[3-((9S,12R)-9-isopropyl-11-oxo-2,7-dioxa-10-azabicyclo[12.2.2]octadéca-1 (17),14(18),15-trién-12-yl)uréido]-3-pipéridin-3-ylpropionique,
acide (S)-3-(6-aminopyridin-3-yl)-2-[3-((8S,11R)-8-isopropyl-10-oxo-6-oxa-1,9,14-triazabicyclo[11.2.1]hexadéca-13(16),14-dién-11-yl)uréido]propionique,
acide (S)-3-(6-aminopyridin-3-yl)-2-[3-((3R,6S)-6-isopropyl-4-oxo-8-oxa-5-azabicyclo[11.2.2]heptadéca-1(16),13(17),14-trién-3-yl)uréido]propionique,
acide (S)-3-(6-aminopyridin-3-yl)-2-[3-((9S,12R)-16-fluoro-9-isopropyl-11-oxo-2,7-dioxa-10-azabicyclo[12.2.2]octadéca-1(17),14(18),15-trién-12-yl)uréido]propionique,
acide (S)-3-(6-aminopyridin-3-yl)-2-[3-((3S,6R)-3-isopropyl-5-oxo-1,8-dioxa-4-azacyclododéc-6-yl)uréido]propionique,
(S)-3-(6-aminopyridin-3-yl)-2-[3-((3S,6R)-3-isopropyl-5-oxo-1,8-dioxa-4-azacyclododéc-6-yl)uréido]propionate de méthyle,
acide (S)-6-amino-2-[3-((3S,6R)-3-isopropyl-5-oxo-1,8-dioxa-4-azacyclododéc-6-yl)uréido]hexanoïque,
acide (S)-6-amino-2-[3-((9S,12S)-9-isopropyl-11-oxo-2,7-dioxa-10-azabicyclo-[12.2.2]octadéc-12-yl)uréido]hexanoïque,
acide (S)-6-amino-2-((8S,11R)-8-isopropyl-3,10-dioxo-6-oxa-2,9-diazabicyclo[11.2.2]heptadéca-1(16),13(17),14-trién-11-ylamino)hexanoïque,
acide (S)-3-(6-aminopyridin-3-yl)-2-[3-((8S,11R)-8-méthyl-3,10-dioxo-6-oxa-2,9-diazabicyclo[11.2.2]heptadéca-1(16),13(17),14-trién-11-yl)uréido]propionique,
acide (S)-3-(6-aminopyridin-3-yl)-2-[3-((3S,6R)-3-isopropyl-5,9-dioxo-1-oxa-4,10-diazacyclotridéc-6-yl)uréido]propionique,
acide (S)-3-(6-aminopyridin-3-yl)-2-[3-((8S,11R)-8-isopropyl-2,10-dioxo-6-oxa-3,9-diazabicyclo[11.2.2]heptadéca-1(16),13(17),14-trién-11-yl)uréido]propionique,
acide (S)-6-amino-2-[3-((9S,12R)-4,5-dihydroxy-9-isopropyl-11-oxo-2,7-dioxa-10-azabicyclo[12.2.2]octadéca-1(17),14(18),15-trién-12-yl)uréido]hexanoïque,
acide (S)-6-amino-2-[3-((9S,12R)-5-hydroxy-9-isopropyl-11-oxo-2,7-dioxa-10-azabicyclo[12.2.2]octadéca-1(17),14(18),15-trién-12-yl)uréido]hexanoïque,
acide 3-(6-aminopyridin-3-yl)-2-[3-((9S,12R)-4,5-dihydroxy-9-isopropyl-11-oxo-2,7-dioxa-10-azabicyclo[12.2.2]octadéca-1(17),14(18),15-trién-12-yl)uréido]propionique,
acide (S)-6-amino-2-[3-((9S,12R)-4-hydroxy-9-isopropyl-11-oxo-2,7-dioxa-10-azabicyclo[12.2.2]octadéca-1(17),14(18),15-trién-12-yl)uréido]hexanoïque ou
acide (S)-6-amino-2-[((9S,12R)-9-isopropyl-11-oxo-2,7-dioxa-10-azabicyclo[12.2.2]octadéca-1(17),14(18),15-trién-12-ylsulfamoyl)amino]hexanoïque.

7. Procédé de préparation du composé de formule I selon une ou plusieurs des revendications 1 à 6, qui comprend
a) la réaction d'un composé de formule (II) 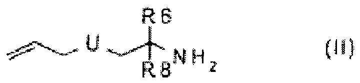 dans laquelle U, R6 et R8 ont les significations indiquées pour le composé de formule (I), avec un acide aminé de formule (III) 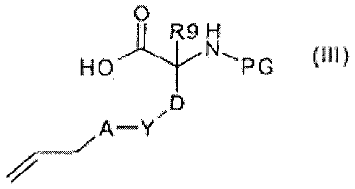 dans laquelle R9, A, Y et D ont les significations indiquées pour le composé de formule (I), pour donner un composé de formule (IV) 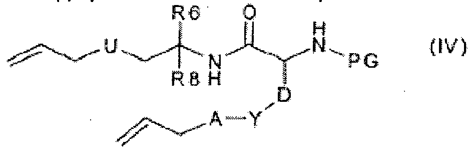 qui est converti, dans les conditions de métathèse avec fermeture de cycle suivie d'une hydrogénation de la double liaison ainsi obtenue, en un composé de formule (V) 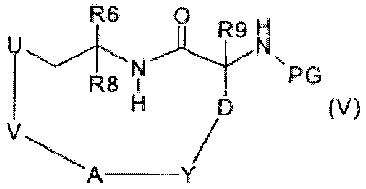 dans laquelle V représente un groupe -alkylène (en C₂-C₉)- ou -alcénylène (en C₃-C₉)-, puis l'élimination du groupe protecteur PG, et l'obtention du composé de formule (VI), 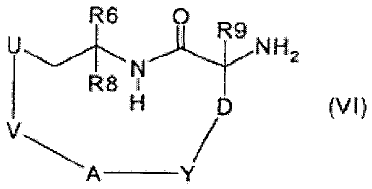 et la réaction avec un composé de formule (VII) 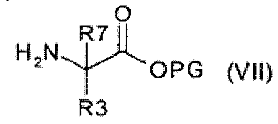 dans laquelle R3 et R7 ont les significations indiquées pour la formule (I), PG est un radical d'un groupe protecteur de fonction ester convenable, et l'atome d'azote dans R3 est protégé si besoin est par un groupe protecteur de fonction amino convenable, avec du phosgène ou un équivalent du phosgène, pour donner un composé de formule (VIII) 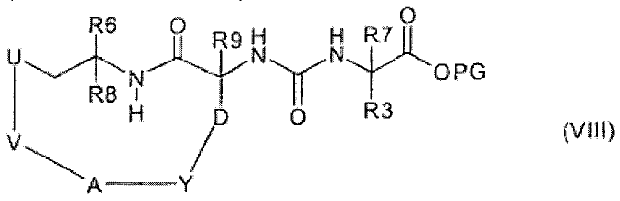 à la suite de quoi le groupe protecteur PG et le groupe protecteur qui est présent si besoin est sur l'atome d'azote dans R3 sont éliminés, pour aboutir au composé de formule (I), ou
b) la réaction d'un composé de formule (IX) 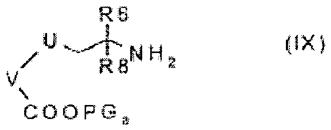 dans laquelle U, V, R6 et R8 ont les significations indiquées pour le composé de formule (I), et PGₐ est un groupe protecteur de fonction carboxyle convenable, avec un acide aminé de formule (X) 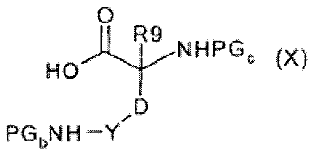 dans laquelle R9, Y et D ont les significations indiquées pour le composé de formule I, et PG_{b} et PG_{c} sont des groupes protecteurs de fonction amino convenables, pour donner un composé de formule (XI) 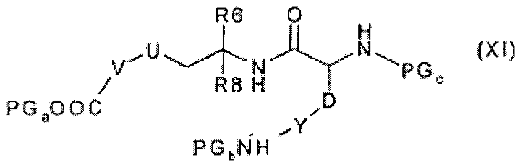 qui, après élimination des groupes protecteurs PGₐ et PG_{b}, est converti en composé de formule (XII) 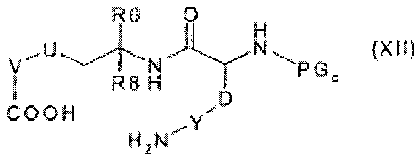 qui est converti au moyen d'un couplage amide en composé de formule (V),
dans laquelle A a les significations indiquées pour le composé de formule I, puis le groupe protecteur est éliminé et le composé de formule (VI) est obtenu,
et mis à réagir avec un composé de formule (VII)
dans laquelle R3 et R7 ont les significations indiquées pour la formule I, PG est un radical d'un groupe protecteur de fonction ester convenable, et l'atome d'azote dans R3 est protégé si besoin est par un groupe protecteur de fonction amino convenable, avec du phosgène ou un équivalent du phosgène, pour donner un composé de formule (VIII), à la suite de quoi le groupe protecteur PG et le groupe protecteur qui est présent si besoin est sur l'atome d'azote dans R3 sont éliminés, pour aboutir au composé de formule (I), ou
c) la réaction d'un composé de formule (XIII) 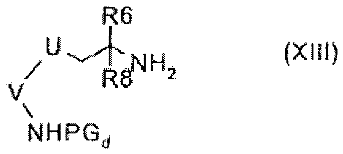 dans laquelle U, V, R6 et R8 ont les significations indiquées pour le composé de formule I, et PG_{d} est un groupe protecteur de fonction amino convenable, avec un acide aminé de formule (XIV) 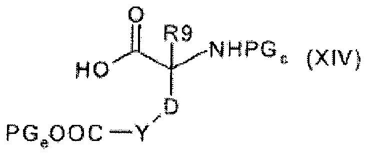 dans laquelle R9, Y et D ont les significations indiquées pour le composé de formule I, et PG_{c} est un groupe protecteur de fonction amino convenable et PGₑ est un groupe protecteur de fonction carboxyle convenable, pour donner un composé de formule (XV) 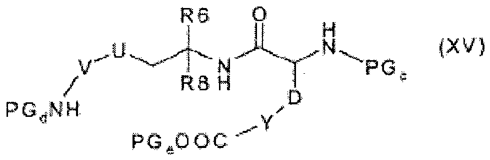 qui, après élimination des groupes protecteurs PG_{d} et PGₑ, est converti en composé de formule (XVI) 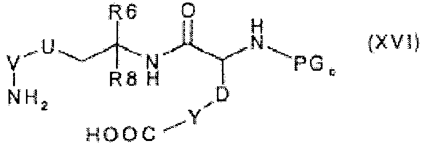 qui est mis à réagir pour donner un composé de formule (V),
dans laquelle A a les significations indiquées pour le composé de formule 1, puis le groupe protecteur est éliminé et le composé de formule (VI) est obtenu,
et mis à réagir avec un composé de formule (VII),
dans laquelle R3 et R7 ont les significations indiquées pour la formule I, PG est un radical d'un groupe protecteur de fonction ester convenable, et l'atome d'azote dans R3 est protégé si besoin est par un groupe protecteur de fonction amino convenable, avec du phosgène ou un équivalent du phosgène, pour donner un composé de formule (VIII), à la suite de quoi le groupe protecteur PG et le groupe protecteur qui est présent si besoin est sur l'atome d'azote dans R3 sont éliminés, pour aboutir au composé de formule (I), ou
d) la réaction d'un composé de formule XVII 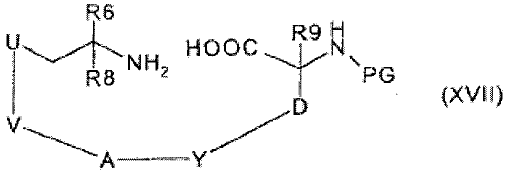 dans laquelle U, V, A, Y, D, R₆, R₈ et R₉ ont les significations indiquées pour le composé de formule I, avec un composé de formule (V), suivie de l'élimination des groupes protecteurs, pour obtenir un composé de formule (VI),
qui est mis à réagir avec un composé de formule (VII),
dans laquelle R3 et R7 ont les significations indiquées pour la formule I, PG est un radical d'un groupe protecteur de fonction ester convenable, et l'atome d'azote dans R3 est protégé si besoin est par un groupe protecteur de fonction amino convenable, avec du phosgène ou un équivalent du phosgène, pour donner un composé de formule (VIII), à la suite de quoi les groupes protecteurs PG et, le cas échéant, le groupe protecteur sur l'atome d'azote dans R3 sont éliminés, pour aboutir au composé de formule (I), ou
e) la conversion d'un composé de formule (VIIIa) 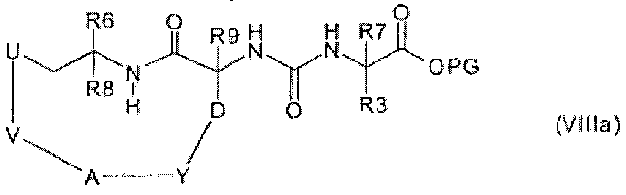 dans laquelle V représente un groupe -alcénylène (en C₃-C₉)-, en composé de formule (VIIIb) 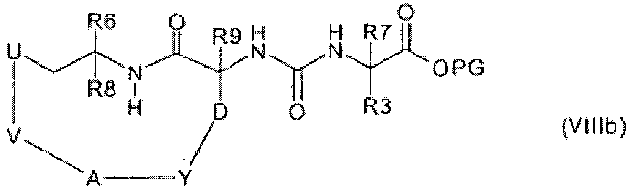 dans laquelle V représente un groupe -alkylène (en C₃-C₉)-, où le groupe alkylène est substitué, indépendamment des autres, une fois, deux fois ou trois fois par -OH, NH₂ ou un halogène, ou représente un groupe -alkylène (en C₁-C₂)-cycloalkyl(en C₃-C₆)-alkylène (en C₁-C₂)-, où le groupe cycloalkyle est substitué, indépendamment des autres, une fois, deux fois ou trois fois par R15, à la suite de quoi le composé de formule (VIIIb) est converti par analogie avec le procédé a) en composé de formule (I), ou
f) la réaction d'un composé de formule (XVIII) 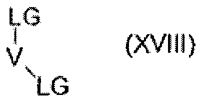 dans laquelle V est tel que défini pour le composé de formule (I), successivement avec les composés de formules (XIX) et (XX) 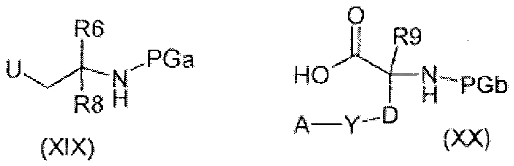 à l'aide de bases dans des solvants aprotiques polaires, et la conversion des composés résultants de formule (XXI) 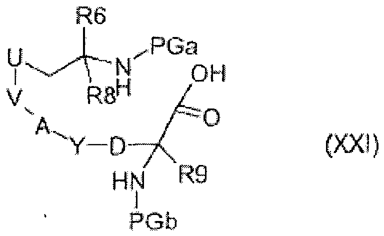 par élimination du groupe protecteur PGa suivie de la formation d'une liaison peptidique, en composé de formule (V), et la réaction de ce dernier comme dans le procédé a), pour donner les composés de formule (I), dans laquelle R6, R8, R9 et A, D, U, V et Y ont les significations indiquées pour la formule (I), et PG représente des groupes protecteurs convenables, et LG est un groupe partant tel que le chlore, le brome, l'iode ou un ester sulfonique, ou
g) la réaction d'un composé de formule VI avec un composé de formule (XXII) 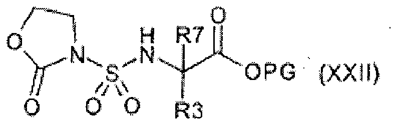 dans laquelle R3 et R7 ont les significations indiquées pour le composé de formule I, et PG est un radical d'un groupe protecteur convenable, pour donner un composé de formule (XXIII) 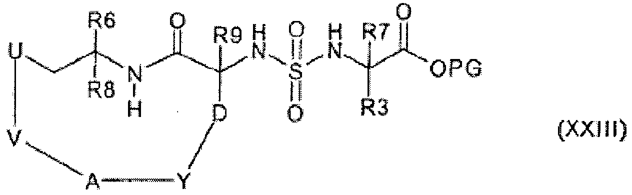 suivie de sa conversion en composé de formule (I), ou
h) le fractionnement d'un composé de formule I préparé par les procédés a), b), c), d), e), f) ou g), ou d'un précurseur convenable de formule (I) qui apparaît sous des formes énantiomères du fait de sa structure chimique, par formation d'un sel avec des acides ou des bases énantiomères purs, chromatographie sur phases stationnaires chirales ou formation d'un dérivé au moyen de composés chiraux énantiomères purs tels que des acides aminés, séparation des diastéréoisomères obtenus de cette façon, et élimination des groupes auxiliaires chiraux pour former les énantiomères purs, ou
i) soit l'isolement sous forme libre du composé de formule (I) préparé par les procédés a), b), c), d), e), f) ou g), soit la conversion en sels tolérés sur le plan physiologique dans le cas où des groupes acides ou basiques sont présents.

8. Médicament comprenant une teneur efficace en au moins un composé de formule I selon une ou plusieurs des revendications 1 à 6, conjointement avec un véhicule, un additif et/ou d'autres ingrédients actifs et excipients pharmaceutiquement convenables et tolérés sur le plan physiologique.

9. Composé de formule I selon une ou plusieurs des revendications 1 à 6 pour la prophylaxie, la prévention secondaire et le traitement d'un ou plusieurs troubles qui sont associés à des thromboses, des embolies, une hypercoagulabilité ou des changements dus à une fibrose.

10. Composé selon la revendication 9, qui concerne un ou plusieurs troubles de la série constituée par l'infarctus du myocarde, l'angine de poitrine et d'autres formes de syndrome coronarien aigu, l'A.V.C., les troubles vasculaires périphériques, la thrombose des veines profondes, l'embolie pulmonaire, les accidents emboliques ou thrombotiques provoqués par les arythmies cardiaques, les accidents cardiovasculaires tels que la resténose après revascularisation et l'angioplastie et des opérations semblables telles que l'implantation de stents et les opérations de pontage, ou la réduction du risque de thrombose après des opérations chirurgicales telles que des opérations sur les articulations du genou et de la hanche, ou dans le contexte de la coagulation intravasculaire disséminée, de la septicémie et d'autres accidents intravasculaires associés à une inflammation, de l'athérosclérose, du diabète et du syndrome métabolique et de ses séquelles, de la croissance des tumeurs et de la métastase des tumeurs, des troubles articulaires inflammatoires et dégénératifs tels que la polyarthrite rhumatoïde et l'arthrose, des insuffisances du système hémostatique telles que les dépôts de fibrine, les changements dus à une fibrose du poumon tels que la broncho-pneumopathie chronique obstructive, le syndrome de détresse respiratoire de l'adulte ou les dépôts de fibrine dans l'oeil après chirurgie oculaire, ou de la prévention ou du traitement de la cicatrisation.
